# EUROPEAN PATENT SPECIFICATION

(11) **EP 1 613 960 B1**
(45) Date of publication and mention of the grant of the patent: **03.06.2009**
(21) Application number: 04759450.2
(22) Date of filing: 09.04.2004
(51) Int. Cl.: G01N 33/68

(54) **METHODS OF ASSESSING CROHN'S DISEASE PATIENT PHENOTYPE BY I2, OMPC AND ASCA SEROLOGIC RESPONSE**
VERFAHREN ZUR BEURTEILUNG DES PHÄNOTYPS EINES PATIENTEN MIT MORBUS CROHN ÜBER EINE SEROLOGISCHE I2-, OMPC- UND ASCA-REAKTION
PROCEDES POUR FAIRE UNE ESTIMATION DU PHENOTYPE D'UN PATIENT SOUFFRANT DE LA MALADIE DE CROHN PAR LA REACTION SEROLOGIQUE I2, OMPC ET ASCA

(30) Priority: 11.04.2003 US 413501; 26.11.2003 US 723164
(43) Date of publication of application: 11.01.2006
(73) Proprietor: Cedars-Sinai Medical Center, Los Angeles, CA 90048 (US)
(72) Inventor: TARGAN, Stephan, R., Santa Monica, CA 90402 (US); VASILIAUSKAS, Eric, A., Manhattan Beach, CA 90266 (US); MOW, William, S., Culver City, CA 90230 (US); YANG, Huiying, Cerritos, CA 90703 (US); FLESHNER, Phillip, R., Beverly Hills, CA 90212 (US); ROTTER, Jerome, I., Los Angeles, CA 90064 (US)
(74) Representative: UEXKÜLL & STOLBERG
(86) International application number: PCT/US2004/011227
(87) International publication number: WO 2004/091372

(56) References cited:
- WO-A-01/89361
- SUTTON C L ET AL: "Identification of a novel bacterial sequence associated with Crohn's disease." GASTROENTEROLOGY. JUL 2000, vol. 119, no. 1, July 2000 (2000-07), pages 23-31, XP002383654 ISSN: 0016-5085
- ABREU MARIA T ET AL: "Mutations in NOD2 are associated with fibrostenosing disease in patients with Crohn's disease." GASTROENTEROLOGY. SEP 2002, vol. 123, no. 3, September 2002 (2002-09), pages 679-688, XP002979597 ISSN: 0016-5085
- MOW WILLIAM S ET AL: "Association of antibody responses to microbial antigens and complications of small bowel Crohn's disease." GASTROENTEROLOGY. FEB 2004, vol. 126, no. 2, February 2004 (2004-02), pages 414-424, XP002383656 ISSN: 0016-5085
- LANDERS ET AL: 'Selected loss of tolerance evidenced by Crohn's disease-associated immune responses to auto- and microbial antigens' GASTROENTEROLOGY vol. 123, September 2002, pages 689 - 699, XP002907234

## Description

### BACKGROUND OF THE INVENTION

This invention relates generally to the fields of diagnostics and autoimmune disease and, more specifically, to serologic and genetic methods for diagnosing clinical subtypes of Crohn's disease.

Inflammatory bowel disease (IBD) is the collective term used to describe two gastrointestinal disorders of unknown etiology: Crohn's disease (CD) and ulcerative colitis (UC). The course,and prognosis of IBD, which occurs world-wide and is reported to afflict as many as two million people, varies widely. Onset of IBD is predominantly in young adulthood with diarrhea, abdominal pain, and fever the three most common presenting symptoms. The diarrhea may range from mild to severe, and anemia and weight loss are additional common signs of IBD. Of all patients with IBD, ten percent to fifteen percent will require surgery over a ten year period. In addition, patients with IBD are at increased risk for the development of intestinal cancer. Reports of an increasing occurrence of psychological problems, including anxiety and depression, are perhaps not surprising symptoms of what is often a debilitating disease that strikes people in the prime of life.

Unfortunately, the available therapies for inflammatory bowel disease are few, and both diagnosis and treatment have been hampered by a lack of knowledge regarding the etiology of the disease. However, it is thought that a combination of genetic factors, exogenous triggers and endogenous microflora can contribute to the immune-mediated damage to the intestinal mucosa seen in inflammatory bowel disease. In Crohn's disease, bacteria have been implicated in initiation and progression of the disease: the intestinal inflammation in Crohn's disease is notable for its frequent responsiveness to antibiotics and susceptibility to bacterial fecal flow. Common intestinal colonists and novel pathogens have been implicated in Crohn's by direct detection or by disease associated anti-microbial immune responses. Furthermore, in many genetically susceptible animal models of chronic colitis, lumenal micro-organisms are a necessary cofactor for disease; animals housed in a germ-free environment do not develop colitis.

It is increasingly apparent that Crohn's disease is a classification representing a number of heterogeneous disease subtypes that affect the gastrointestinal tract and produce similar symptoms. Both environmental and genetic factors likely contribute to the etiology of such disease subtypes. Patients with Crohn's disease can be classified, for example, into subtypes based on the presence of fibrostenotic disease, internal-perforating disease, perianal fistulizing disease or ulcerative colitis-like disease according to previously described criteria. The extensive and often protracted clinical testing required to determine Crohn's disease subtypes may delay optimal treatment and involves invasive procedures such as endoscopy.

Identification of serologic and genetic markers which are closely associated with a clinical subtype of Crohn's disease would provide the basis for novel diagnostic tests and eliminate or reduce the need for the battery of laboratory, radiological, and endoscopic evaluations typically required to determine disease subtype. The availability of methods for diagnosing clinical subtypes of Crohn's disease would represent a major clinical advance that would aid in the therapeutic management of Crohn's disease and would further lay the groundwork for the design of treatment modalities which are specific to a particular disease subtype.

Landers et al., Gastroenterology 2002, 123: 689-699, discusses the assessment of responses of a heterogeneous population of patients with different clinical subtypes of Colitis ulcerosa to ASCA, I2, pANCA and OmpC.

WO 2001/89361 refers to the use of combinations of anti-Omp-C, ASCA, IgA anti-I2 and pANCA antibodies to diagnose Colitis ulcerosa.

Sutton et al., Gastroenterology 2000, 119: 23-31, teaches an ELISA for detection of IgA anti-I2 antibodies in patients with Colitis ulcerosa.

Abreu et al., Gastroenterology 2002, 3: 679-688, states that variations in the NOD2 gene contribute to the occurrence of the fibrostenotic clinical subtype of CD.

Nakamura et al., MLO Med Lab Obs. 2001, 33:8-15, also regards serologic markers in inflammatory bowel disease, e.g., expression of pANCA in a subgroup characterized as colitis-like.

Mow et al., Gastroenterology 2004, 2: 414-424, was published after the priority date of the present application and refers to the relationship between the marker I2, ASCA, OmpC, NOD2 and ANCA and certain clinical subtypes of CD.

Such methods can reduce costs associated with treatment of unresponsive disease subtypes and eliminate the disappointment of those needlessly undergoing ineffective therapy. In particular, a reliable genetic test for the fibrostenotic subtype of Crohn's disease would be highly prized as a non-invasive method for the early diagnosis of this disease subtype and would also be useful for predicting susceptibility to the fibrostenotic subtype of Crohn's disease in asymptomatic individuals, making prophylactic therapy possible. The present invention satisfies this need and provides related advantages as well.

### SUMMARY OF THE INVENTION

The invention provides an in vitro method of diagnosing a clinical subtype of Crohn's disease in a subject having Crohn's disease by determining the presence or absence of IgA anti-I2 antibodies in a sample from the subject, wherein the presence of said IgA anti-I2 antibodies indicates that the subject has said subtype of Crohn's disease wherein said clinical subtype of Crohn's disease is a fibrostenotic subtype of Crohn's disease, a subtype of Crohn's disease characterized by the need for small bowel surgery, or a subtype of Crohn's disease characterized by the absence of features of ulcerative colitis. In one embodiment, a method of the invention is practiced by further determining the presence or absence of a NOD2 variant, anti-Saccharomyces cerevisiae antibodies (ASCA), or anti-OmpC antibodies, wherein said NOD2 variant is selected from R702W, G908R, and 1007fs, and wherein the presence of said IgA anti-I2 antibodies and the presence of one of said one or more fibrostenotic markers indicates a greater chance that the subject will have an aggressive form of the fibrostenotic subtype of Crohn's disease requiring small bowel surgery.

### BRIEF DESCRIPTION OF THE DRAWINGS

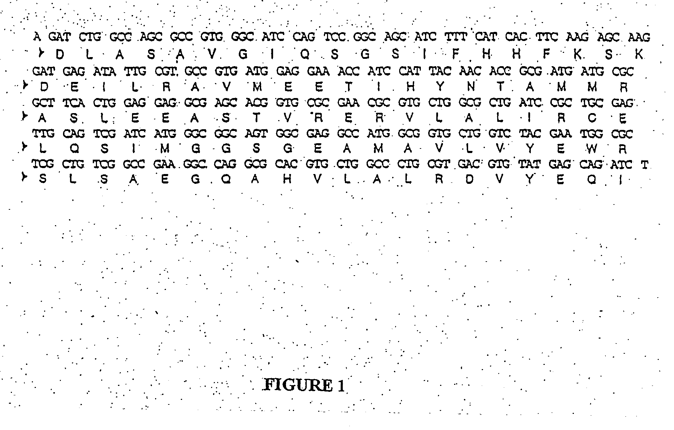

Figure 1 shows an I2 nucleotide sequence (SEQ ID NO: 1) and predicted amino acid sequence (SEQ ID NO: 2)

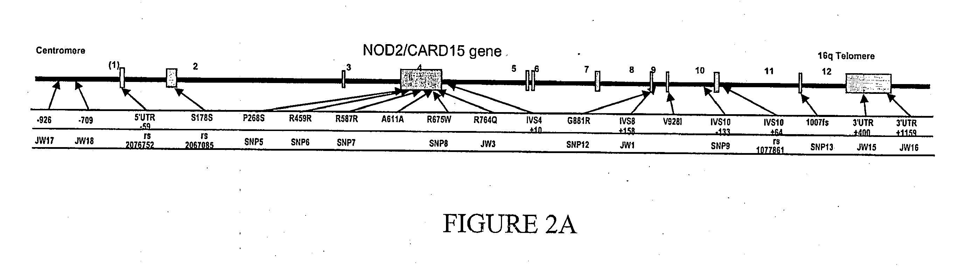

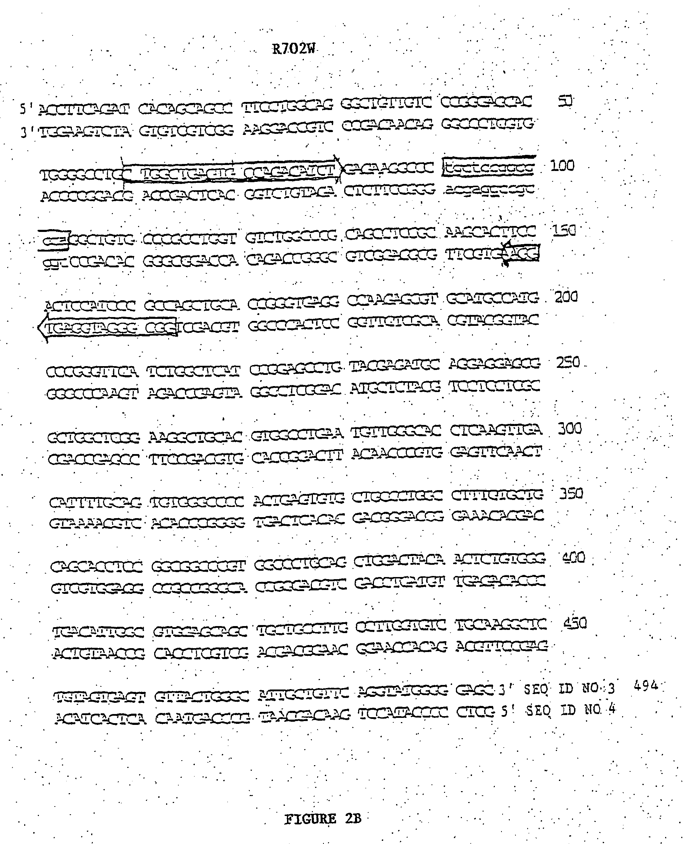

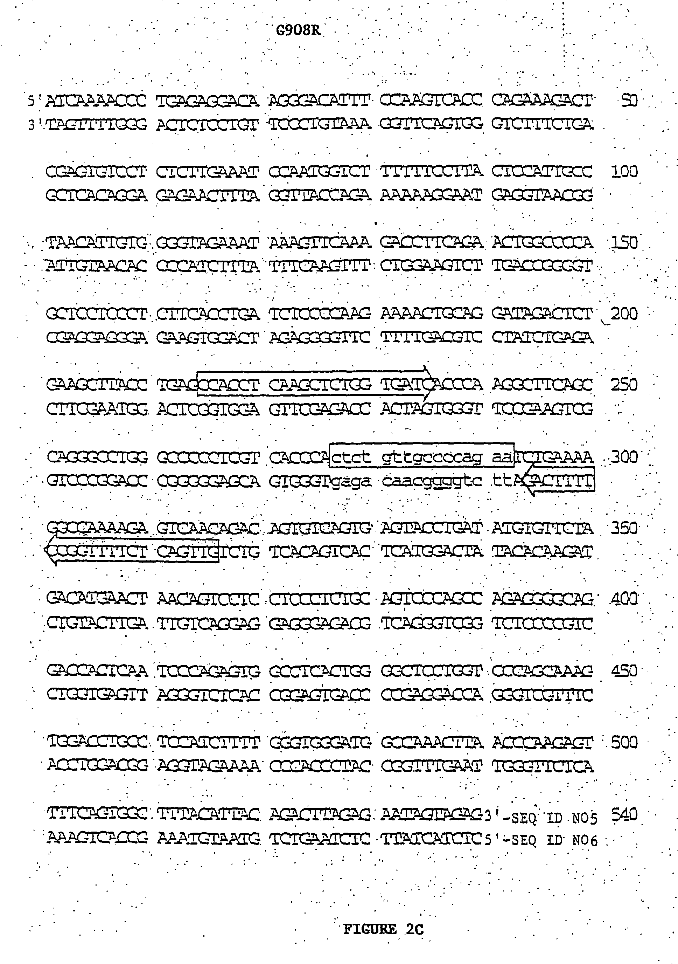

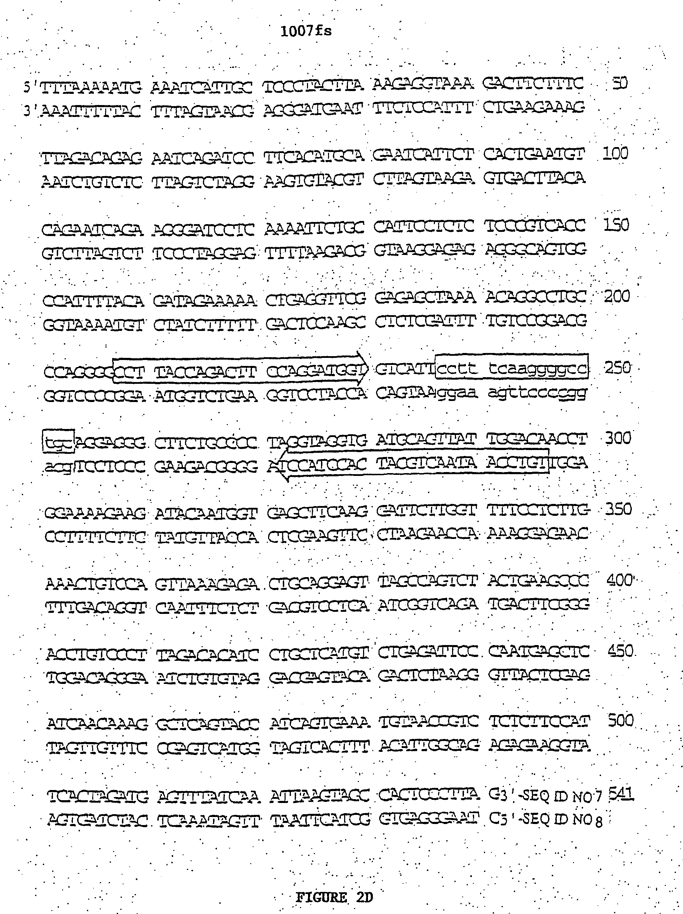

Figure 2A shows an illustration of the NOD2 gene locus. The location of selected NOD2 variants is indicated. Figure 2B shows the nucleotide sequence of the NOD2 gene surrounding the R702W NOD2 variant. The top strand is labeled as SEQ ID NO:3 and the bottom strand is labeled as SEQ ID NO:4. Nucleotide sequences which can be used as primers for PCR amplification are indicated. Figure 2C shows the nucleotide sequence of the NOD2 gene surrounding the G908R NOD2 variant. The top strand is labeled as SEQ ID NO:5 and the bottom strand is labeled as SEQ ID NO:6. Nucleotide sequences which can be used as primers for PCR amplification are indicated. Figure 2D shows the nucleotide sequence of the NOD2 gene surrounding the 1007fs NOD2 variant. The top strand is labeled as SEQ ID NO:7 and the bottom strand is labeled as SEQ ID NO:8. Nucleotide sequences which can be used as primers for PCR amplification are indicated. In Figures 2B, C and D, the position of a nucleotide sequence which can be used as a probe in an allelic discrimination assay is boxed and the position of the polymorphic site is underlined.

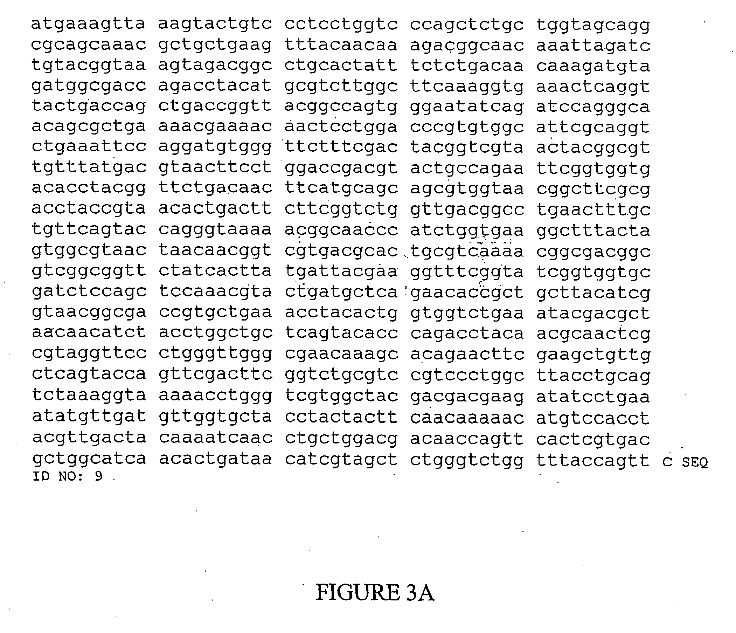

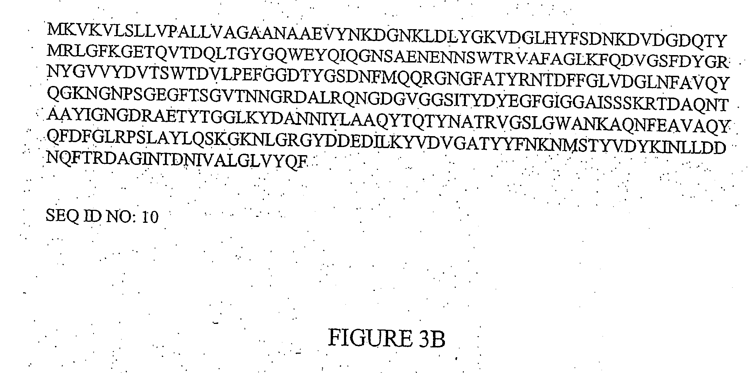

Figure 3A shows the nucleotide sequence (SEQ ID NO:9) of an E. coli outer membrane protein c (OmpC) precursor and Figure 3B shows the corresponding amino acid sequence (SEQ ID NO:10).

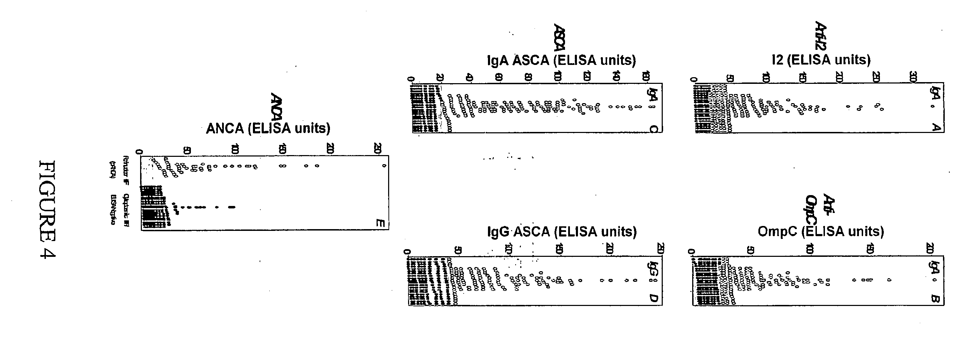

Figure 4 shows scatter graphs of the level of patient serum reactivity towards microbial and autoantigens in a Crohn's disease cohort of 303 patients. (A) IgA anti-I2, (B) IgA anti-OmpC, (C) IgA ASCA, (D) IgG ASCA and (E) ANCA. In each panel, the shaded zone at the bottom indicates negative serum reactivity. Circles show I2-, OmpC-, ASCA-, and pANCA-positive reactivity. In panel E, the open circles in the left-side portion represent a perinuclear staining pattern while the black circles shown in the right-side portion represent ANCA-positive samples with a cytoplasmic indirect immunofluorescent (IIF) staining pattern.

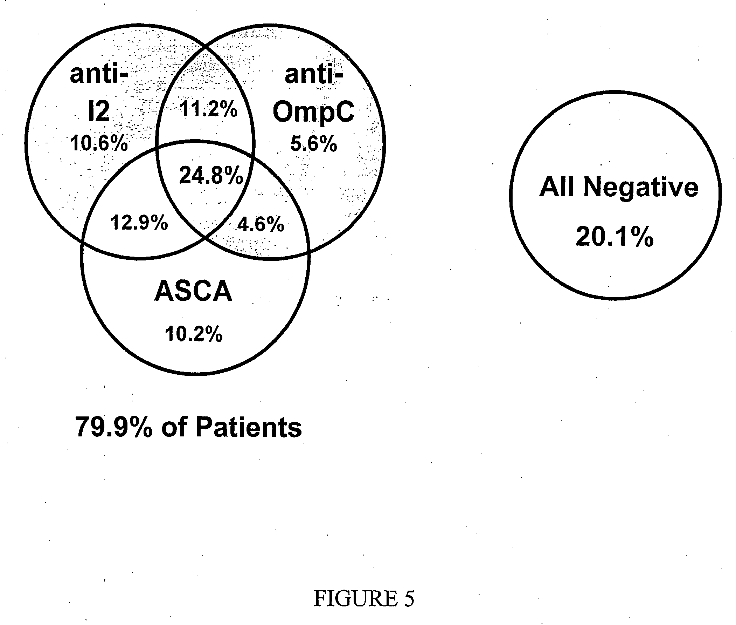

Figure 5 is a Venn diagram showing the relationship between microbial marker antibodies in the Crohn's disease cohort of 303 patients. Shown are the percentage of patients positive for a single marker, the three combinations of two markers, and all three markers.

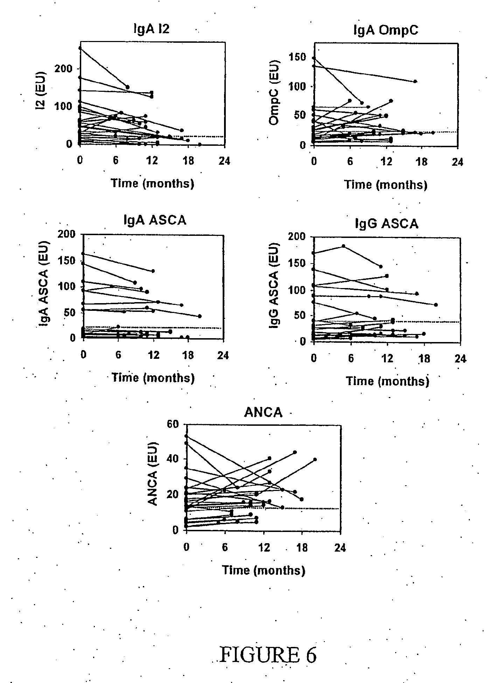

Figure 6 shows that no significant changes in serologic response to microbial and autoantigens occurred over time. Serologic responses were determined following small bowel surgery (time 0) in 26 patients with at least one sequential follow up analysis six months or more after the surgery. The dotted line represents the demarcation between positive and negative values.

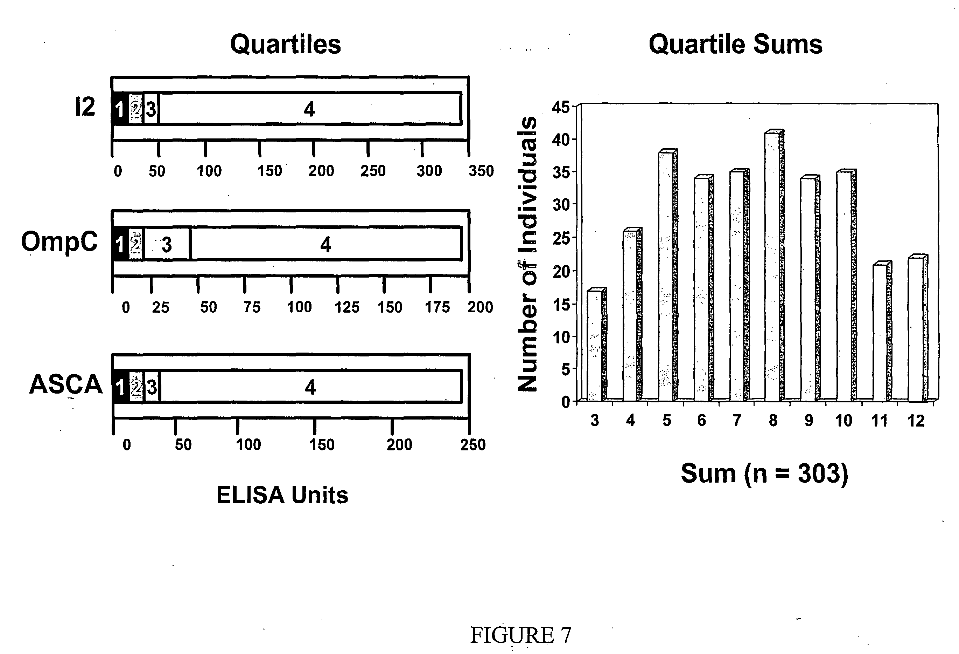

Figure 7 shows quartile analysis of the 303 Crohn's disease patient cohort for three microbial antigens: I2, OmpC and ASCA. The population was subdivided into four quartiles by I2 (top left), OmpC (middle left), and ASCA (bottom left) binding levels. Values for binding levels are in ELISA units. Quartile sums were calculated by the addition of each individual's quartile values for the three microbial antigens to give a quartile sum ranging from 3 to 12. Patients with the lowest level reactivity towards all three antigens had a quartile sum score of 3 while patients with the highest level antibody reactivity towards all three had a quartile sum score of 12. The distribution of quartile sums for the 303 patient cohort is shown in the right panel.

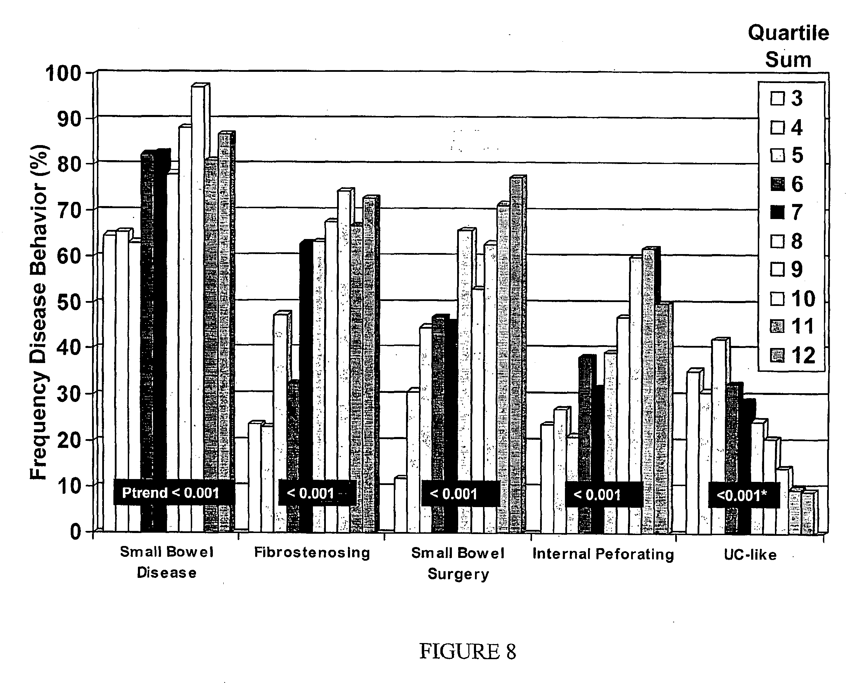

Figure 8 shows that the frequency of complicated small bowel disease increases with antibody reactivity, as represented by the quartile sum score against all three antigens (* denotes negative ptrend). Those patients with the highest level antibody reactivity towards all three microbial antigens have the highest association with complicated small bowel disease phenotypes.

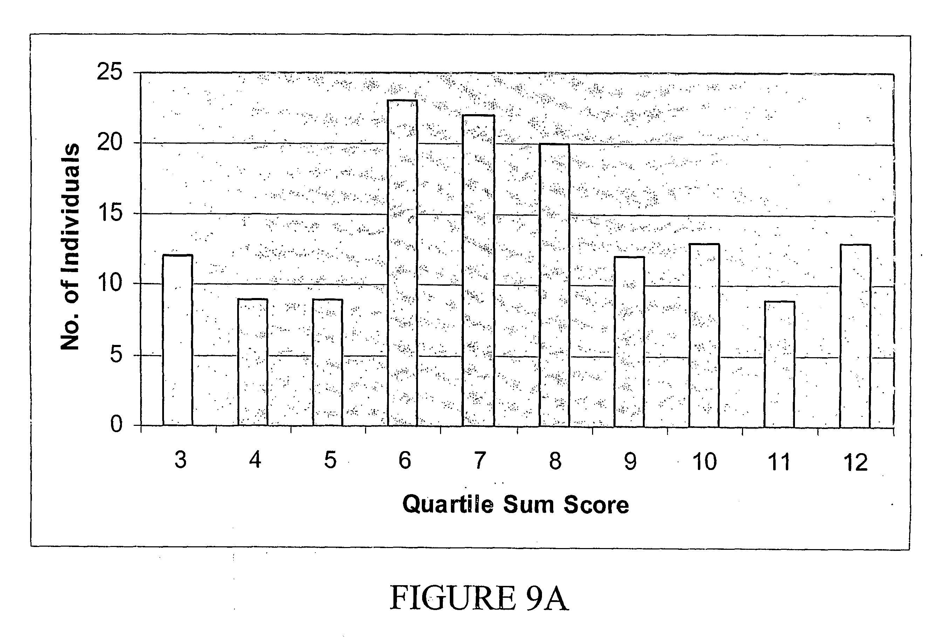

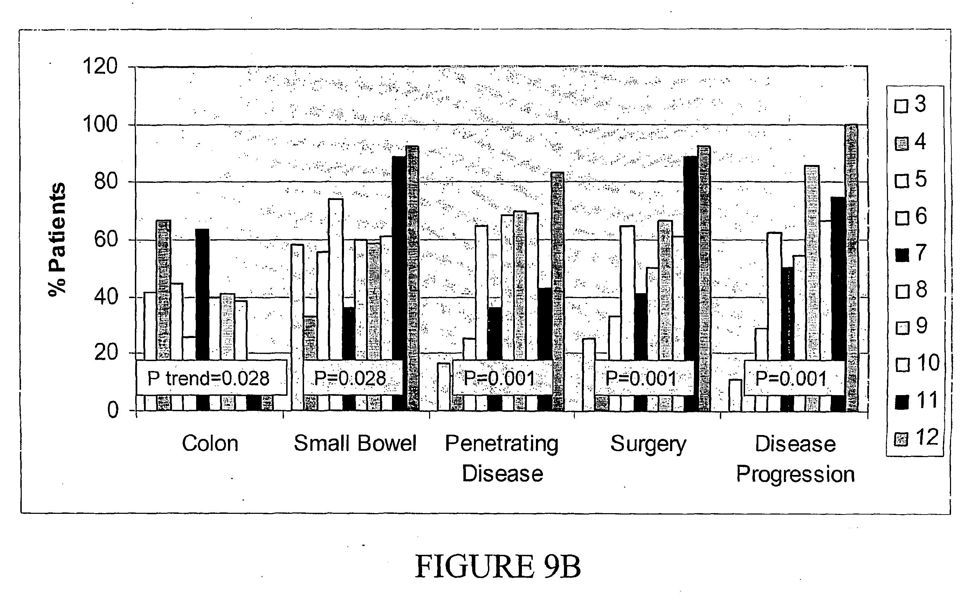

Figure 9 shows quartile analysis for a cohort of 142 Scottish patients. 9A. Individuals with lower quartile sums have a lower total level of response to microbial antigens while those with higher scores have a greater level of response to microbial antigens. 9B. Changes in phenotypic characteristics of disease are associated with increasing quartile sums in the Scottish patient cohort. Changes in anatomical distribution, the need for surgery, penetrating disease and the frequency of disease progression were statistically significant. Disease duration also varied between the groups. Other parameters such as age at diagnosis, sex, family history, need for azathioprine or infliximab or NOD2/CARD15 status did not vary across the quartile sums.

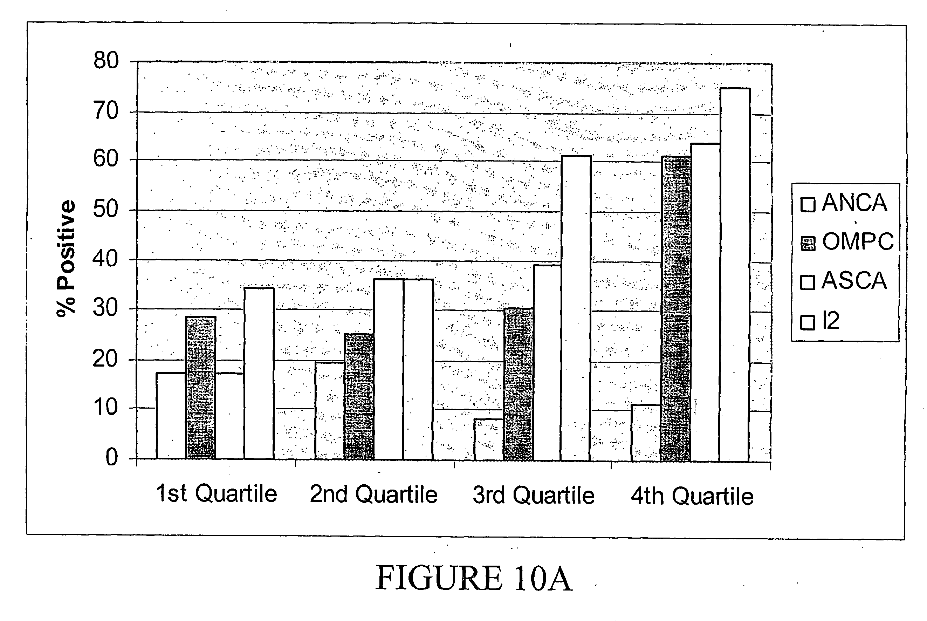

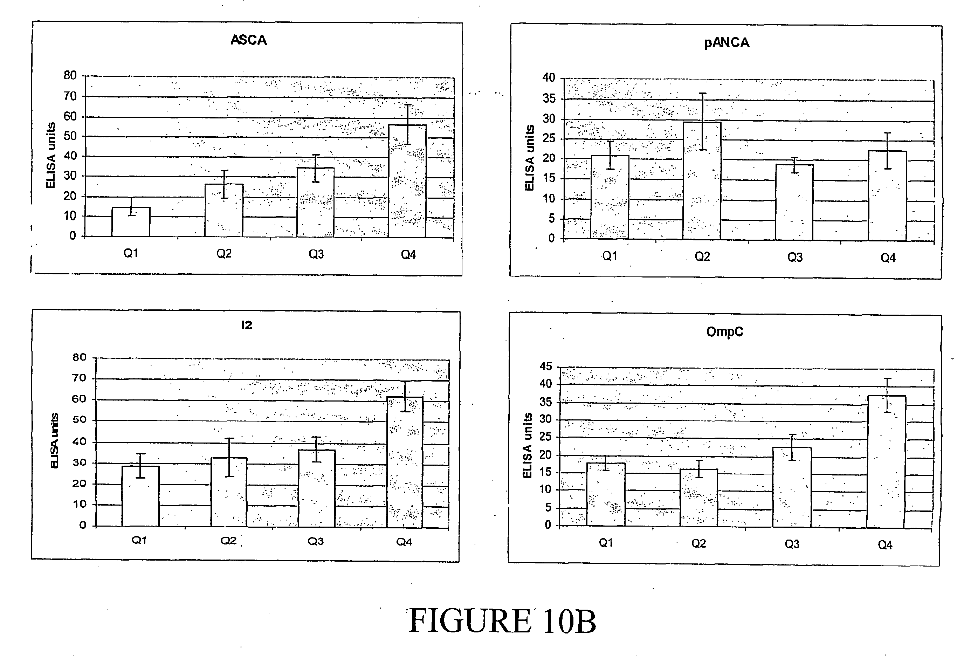

Figure 10 shows associations between antibody response and disease duration as well as age quartile. Figure 10A represents changes in the frequency of antibody detection with increasing disease duration quartile. Statistically significant results are seen for ASCA (p=0.001), I2 (p=0.001) and OmpC (p=0.005) but not ANCA (p=0.496). Figure 10B shows changes in the magnitude of antibody response with increasing age quartile. Significant results are seen for ASCA (p=0.001), I2 (p=0.005) and OmpC (p=0.003) but not for pANCA. The values are expressed as mean +/- standard error of the mean.

### DETAILED DESCRIPTION OF THE INVENTION

The present invention is directed to the exciting discovery of serologic and genetic markers that are closely associated with the fibrostenotic subtype of Crohn's disease. These markers can be used to diagnose the fibrostenotic subtype of Crohn's disease in a subject having Crohn's disease.

As disclosed herein, ELISAs for IgA anti-I2 antibodies and anti-Saccharomyces cerevisiae antibodies (ASCA) were performed on 258 Crohn's disease patients (Examples II and III, respectively). In addition, genotyping was performed on these patients for three Crohn's disease associated variants of the NOD2 gene, R702W, G908R, and 1007 fs, using the Taqman® MGB system as described in Example IV.

The results disclosed herein demonstrate that IgA antibodies to I2 were present in 56.5% of the Crohn's disease patients in the study (see Example I). Patients expressing these IgA anti-I2 antibodies were significantly more likely to have a fibrostenotic subtype of Crohn's disease than those not expressing IgA anti-I2 antibodies (71.4% vs. 43.3%, p<0.001) and significantly more likely to require small bowel surgery (66.7% vs. 37.1%, p< 0.001). In addition; IgA anti-I2 antibody expression was negatively associated with ulcerative colitis-like Crohn's disease (20.6% vs. 41.24%, p<0.001). Quartile analyses revealed that higher levels of IgA anti-I2 antibodies were more strongly associated with the fibrostenotic subtype of Crohn's disease (p for the trend < 0.001) and small bowel involvement (p= 0.023), and inversely associated with ulcerative colitis-like Crohn's disease (p= 0.005) compared to lower levels of IgA anti-I2 antibodies. In addition, as disclosed in Example I, conditional analysis performed on NOD2 variants and ASCA indicated that IgA anti-I2 antibodies were independently associated with the fibrostenotic subtype of Crohn' disease (p= 0.001 and p=0.005, respectively). Similarly, IgA anti-I2 was independently associated with small bowel surgery when conditioned on NOD2 variation (p= 0.001) or ASCA (p= 0.002). These results indicate that the presence of IgA anti-I2 antibodies can be used to diagnose a clinical subtype of Crohn's disease, such as the fibrostenotic subtype, in a subject having Crohn's disease.

As further disclosed in Example I, patients with all three markers, IgA anti-I2 antibodies, one of the three NOD2 variants, and ASCA showed a greater risk of the fibrostenotic subtype of Crohn's disease (82%, odds ratio=9.7, p<0.000001), compared with patients with two markers (74%, odds ratio = 6.0), one marker (48%, odds ratio= 1.9), or none of these markers (33%, odds ratio = reference group). These results indicate that the presence of IgA anti-I2 antibodies in combination with the presence of other markers can be used to diagnose a fibrostenotic subtype Crohn's disease in a patient having Crohn's disease.

The results disclosed herein in Example VII with a cohort of 303 Crohn's disease patients corroborate that anti-I2 reactivity is significantly associated with fibrostenosis and small bowel surgery in patients with Crohn's disease, and additionally show that anti-I2 reactivity is significantly associated with the occurrence of small bowel disease in these patients (Table 4). Furthermore, anti-OmpC reactivity was associated with subtypes of Crohn's disease characterized by fibrostenosis, internal perforating disease, or the need for small bowel surgery (see Table 4). Reactivity against both of these antigens was negatively associated with ulcerative colitis-like disease in Crohn's patients.

As additionally disclosed herein, the relationship between serum reactivity towards one, two, or three microbial antigens (I2, oligomannan and OmpC) and clinical phenotype was analyzed irrespective of pANCA and NOD2 status. Table 6 shows that, in the cohort of U.S. CD patients, individuals with all three associated markers were more likely to have fibrostenotic disease, internal perforating disease and to require small bowel surgery, as compared with CD patients having serum reactivity with fewer of these markers (p for all ≤ 0.001). These results indicate that Crohn's disease patients who have antibody responses towards a greater number of the microbial antigens I2, oligomannan and OmpC are at increased risk for fibrostenosis, internal perforating disease, and the need for small bowel surgery as compared with patients with a serologic response towards a smaller number of these antigens. The association between antibody response towards a greater number of the microbial antigens and increased risk of internal perforating disease and small bowel surgery was confirmed in a second cohort of 142 patients, as disclosed in Example VIII.

The importance of quantitative antibody response against I2, oligomannan, or OmpC to frequency of various Crohn's disease clinical subtypes is further disclosed herein. Table 7A shows the results of quartile analysis for anti-I2, ASCA and anti-OmpC for each disease characteristic. As disclosed herein in Example VII, there was an increasing percentage of Crohn's disease patients with small bowel disease, fibrostenotic disease, internal perforating disease, small bowel surgery, and a decreasing likelihood of UC-like disease, as the magnitude of an antibody response toward a microbial antigen increased. The importance of a quantitative antibody response against I2, oligomannan or OmpC was further studied in a second cohort comprised of 142 Scottish patients. As disclosed herein in Example VIII, the magnitude of an antibody response was associated with several clinical phenotypes, specifically, small bowel disease, internal perforating disease and the need for small bowel surgery, and was inversely associated with UC-like disease (see, also, Table 14). In sum, these results demonstrate that a greater antibody response towards I2, OmpC, or oligomannan is associated with increasing frequency of complicated small bowel Crohn's disease.

Further disclosed herein in Example VII is an analysis of the total level of antibody response towards all three microbial antigens. Quartile sum analysis (sum of quartile scores for anti-I2, ASCA and anti-OmpC) was performed in order to evaluate a possible association between the level of combined immune response towards I2, oligomannan and OmpC, and disease characteristics for an individual Crohn's patient. As shown in Figure 7, individual serologic responses were broken down by quartiles and assigned scores of 1 to 4 based on their designated quartile. Individual quartile scores for each microbial antigen were added to obtain a quartile sum score, ranging from 3 to 12, which represented the cumulative quantitative immune response towards the three microbial antigens. As revealed in Figure 8, Crohn's disease patients with greater quartile sum scores had an increasing likelihood of small bowel disease, fibrostenotic disease, and internal perforating disease, an increasing need for small bowel surgery, and a decreasing frequency of UC-like disease. These results demonstrate that the presence of multiple high-level antibody responses towards the microbial antigens I2, oligomannan and OmpC is associated with a higher frequency of complicated small bowel disease.

Based on these findings, the present invention provides an in vitro method of diagnosing a clinical subtype of Crohn's disease in a subject having Crohn's disease by determining the presence or absence of IgA anti-I2 antibodies in a sample from the subject, where the presence of the IgA anti-I2 antibodies indicates that the subject has a clinical subtype of Crohn's disease, wherein the subtype is a fibrostenotic subtype, a subtype characterized by the need for small bowel surgery, or a subtype characterized by the absence of features of ulcerative colitis, as described herein. The methods of the invention can be advantageous in that they are noninvasive and can be conveniently practiced, for example, with a blood sample from the subject. The methods of the invention can be used to quickly, easily and reliably diagnose or predict susceptibility to the clinical subtype of Crohn's disease. The methods of the invention can also be advantageous in that they can be useful for predicting how a subject will respond to a certain therapy.

In one embodiment, a method of the invention is practiced by determining the presence or absence of IgA anti-I2 antibodies in a sample from a subject having Crohn's disease and further determining the presence or absence in the subject of a NOD2 variant, anti-Saccharomyces cerevisiae antibodies (ASCA), or IgA anti-OmpC antibodies, wherein said NOD2 variant is selected from R702W, G908R, or 1007fs. In a further embodiment, determining the presence or absence of IgA anti-I2 antibodies in the subject is practiced by contacting a sample from the subject with an I2 antigen, or immunoreactive fragment thereof, under conditions suitable to form a complex of I2 antigen, or immunoreactive fragment thereof, and antibody against the I2 antigen; contacting the complex with a labeled secondary antibody; and detecting the presence or absence of the complex, where the presence of the complex indicates the presence of the anti-I2 antibodies in the subject.

The invention also provides a method of diagnosing a fibrostenotic subtype of Crohn,'s disease by determining the presence or absence of IgA anti-12 antibodies in a sample from a subject having Crohn's disease, where the presence of IgA ant-I2 antibodies indicates that the subject has the fibrostenotic subtype of Crohn's disease. In one embodiment, a method of the invention is practiced by further determining the presence or absence in the sample from the subject of one or more of the following fibrostenotic markers: a NOD2 variant, anti-Saccharomyces cerevisiae antibodies (ASCA), or IgA anti-OmpC antibodies, wherein the NOD2 variant can be R702W, G908R, or 1007fs NOD2 variant. In a further embodiment, a method of the invention is practiced by determining the presence or absence of anti-I2 antibodies, a NOD2 variant and ASCA. In yet a further embodiment, determining the presence or absence of IgA anti-I2 antibodies in the subject is practiced by contacting a sample from the subject with an I2 antigen, or immunoreactive fragment thereof, under conditions suitable to form a complex of I2 antigen, or immunoreactive fragment thereof, and antibody against the I2 antigen; contacting the complex with a labeled secondary antibody; and detecting the presence or absence of the complex, where the presence of the complex indicates the presence of the IgA anti-I2 antibodies in the subject.

In one embodiment, a method of the invention is practiced by determining the presence or absence in the sample from the subject of IgA anti-I2 antibodies and further determining the presence or absence of said NOD2 variant, where the presence of IgA anti-I2 antibodies and the presence of the NOD2 variant in the subject indicates that the subject has the fibrostenotic subtype of Crohn's disease. In a related embodiment, the combined presence of the IgA anti-I2 antibodies and the NOD2 variant in the subject is associated with the fibrostenotic subtype of Crohn's disease with an odds ratio of at least 6. In another embodiment, the invention is practiced by determining the presence or absence of IgA anti-I2 antibodies and further determining the presence or absence of ASCA in the subject, where the presence of the IgA anti-I2 antibodies and the presence of ASCA in the subject indicates that the subject has the fibrostenotic subtype of Crohn's disease. In a related embodiment, the combined presence of the anti-I2 antibodies and ASCA in the subject is associated with the fibrostenotic subtype of Crohn's disease with an odds ratio of at least 6. In a further embodiment, the invention is practiced by determining the presence or absence of IgA anti-I2 antibodies and further determining the presence or absence of the NOD2 variant and ASCA in the subject, where the combined presence of IgA anti-I2 antibodies; the NOD2 variant, and ASCA in the subject indicates that the subject has the fibrostenotic subtype of Crohn's disease. In a related embodiment, the combined presence of the anti-I2 antibodies, the NOD2 variant, and ASCA in the subject is associated with the fibrostenotic subtype of Crohn's disease with an odds ratio of at least 9.

The present invention also provides an in vitro method of determining a risk of having or developing a clinical subtype of Crohn's disease characterized by fibrostenosis, internal perforating disease or the need for small bowel surgery in a subject having Crohn's disease by determining the presence or absence of three markers in the subject, the three markers being IgA anti-I2, ASCA and IgA anti-OmpC antibodies, where the presence of the three markers indicates a first risk of having or developing the clinical subtype of Crohn's disease, the presence of exactly two of the three markers indicates a second risk of having or developing the clinical subtype of Crohn's disease, the presence of exactly one of the three markers indicates a third risk of having or developing the clinical subtype of Crohn's disease, and the absence of the three markers indicates a fourth risk of having or developing the clinical subtype of Crohn's disease, and where the first risk is greater than the second risk, the second risk is greater than the third risk, and the third risk is greater than the fourth risk.

Further provided herein is an in vitro method of determining a risk of having or developing a clinical subtype of Crohn's disease characterized by the need for small bowel surgery in a subject having Crohn's disease by determining the presence or absence of three markers in the subject, the three markers being IgA anti-I2 antibodies, ASCA and IgA anti-OmpC antibodies, where the presence of the three markers indicates a first risk of having or developing the clinical subtype of Crohn's disease, the presence of exactly two of the three markers indicates a second risk of having or developing the clinical subtype of Crohn's disease, the presence of exactly one of the three markers indicates a third risk of having or developing the clinical subtype of Crohn's disease, and the absence of the three markers indicates a fourth risk of having or developing the clinical subtype of Crohn's disease, and where the first risk is greater than the second risk, the second risk is greater than the third risk, and the third risk is greater than the fourth risk.

The present invention additionally provides an in vitro method of determining a risk of having or developing a clinical subtype of Crohn's disease characterized by fibrostenosis or the need for small bowel surgery in a subject having Crohn's disease by determining the presence and magnitude of IgA anti-I2 antibody response in the subject, where a greater magnitude of IgA anti-I2 antibody response indicates a greater risk of having or developing the clinical subtype characterized by fibrostenosis or the need for small bowel surgery.

Further provided herein is an in vitro method of determining a risk of having or developing a clinical subtype of Crohn's disease characterized by fibrostenosis, internal perforating disease or the need for small bowel surgery in a subject having Crohn's disease by determining the presence and magnitude of IgA anti-OmpC antibody response in the subject, where a greater magnitude of IgA anti-OmpC antibody response indicates a greater risk of having or developing the clinical subtype characterized by fibrostenosis, internal perforating disease or the need for small bowel surgery.

The invention additionally provides an in vitro method of determining a risk of having or developing a clinical subtype of Crohn's disease characterized by fibrostenosis, internal perforating disease or the nerd for small bowel surgery in a subject having Crohn's disease by determining the presence and magnitude of three markers in the subject, the three markers being IgA anti-I2 antibodies, anti-*Saccharomyces cerevisiae* antibodies (ASCA), and IgA anti-OmpC antibodies, where a greater magnitude of the three markers combined indicates a greater risk of having or developing the clinical subtype characterized by fibrostenosis, internal perforating disease or the need for small bowel surgery.

The methods of the invention relate to the diagnosis and treatment of Crohn's disease (regional enteritis), which is a disease of chronic inflammation that can involve any part of the gastrointestinal tract. Commonly the distal portion of the small intestine (ileum) and cecum are affected. In other cases, the disease is confined to the small intestine, colon or anorectal region. Crohn's disease occasionally involves the duodenum and stomach, and more rarely the esophagus and oral cavity.

The variable clinical manifestations of Crohn's disease are, in part, a result of the varying anatomic localization of the disease. The most frequent symptoms of Crohn's disease are abdominal pain, diarrhea and recurrent fever. Crohn's disease is commonly associated with intestinal obstruction or fistula, which is an abnormal passage, for example, between diseased loops of bowel. Crohn's disease also may include complications such as inflammation of the eye, joints and skin; liver disease; kidney stones or amyloidosis. In addition, Crohn's disease is associated with an increased risk of intestinal cancer.

Several features are characteristic of the pathology of Crohn's disease. The inflammation associated with Crohn's disease, known as transmural inflammation, involves all layers of the bowel wall. Thickening and edema, for example, typically also appear throughout the bowel wall, with fibrosis also present in long-standing disease. The inflammation characteristic of Crohn's disease also is discontinuous in that segments of inflamed tissue, known as "skip lesions," are separated by apparently normal intestine. Furthermore, linear ulcerations, edema, and inflammation of the intervening tissue lead to a "cobblestone" appearance of the intestinal mucosa, which is distinctive of Crohn's disease.

A hallmark of Crohn's disease is the presence of discrete aggregations of inflammatory cells, known as granulomas, which are generally found in the submucosa. Some Crohn's disease cases display the typical discrete granulomas, while others show a diffuse granulomatous reaction or nonspecific transmural inflammation. As a result, the presence of discrete granulomas is indicative of Crohn's disease, although the absence of granulomas also is consistent with the disease. Thus, transmural or discontinuous inflammation, rather than the presence of granulomas, is a preferred diagnostic indicator of Crohn's disease (Rubin and Farber, Pathology (Second Edition) Philadelphia: J.B. Lippincott Company (1994)).

In contrast to ulcerative colitis, which is characterized by a continuous inflammation of the colon that usually is more severe distally than proximally, Crohn's disease is a patchy disease with frequent sparing of the rectum. Furthermore, the inflammation in Crohn's disease is distinct from the superficial inflammation seen in ulcerative colitis, which is usually limited to the mucosal layer and characterized by an acute inflammatory infiltrate with neutrophils and crypt abscesses. Instead, Crohn's disease affects the entire thickness of the bowel wall with granulomas often, although not always, present. Furthermore, involvement of the terminal ileum, a cobblestone-like appearance, discrete ulcers or fistulas suggest Crohn's disease.

The methods of the invention are practiced in a sample from a subject having Crohn's disease. As used herein, the term "subject" means any animal, such as a human or other mammal, capable of having Crohn's disease. A subject having Crohn's disease can have one or more symptoms of Crohn's disease or can be asymptomatic, having been previously diagnosed as having Crohn's disease by one or more well established criteria. The methods of the invention can be useful, for example, for diagnosing a subtype of Crohn's disease in a subject with one or more symptoms of Crohn's disease. In one embodiment, the methods of the invention are used to determine the presence or absence of the fibrostenotic subtype of Crohn's disease in a subject known to have Crohn's disease. One skilled in the art understands that the methods of the invention also can be practiced in an individual not yet diagnosed as having Crohn's disease, for example, an individual at risk for having Crohn's disease. Such an individual can be, for example, genetically related to a subject with Crohn's disease or can belong to a population that is known to be at increased risk for having Crohn's disease such as the Ashkenazi Jewish population.

Several of the methods of the invention are practiced by determining the presence or absence of IgA anti-I2 antibodies in a subject having Crohn's disease. As used herein, the term "IgA anti-I2 antibodies" means IgA antibodies that selectively bind to an I2 antigen, as well as fragments of antibodies that retain a selective binding activity for an I2 antigen of at least about 1x105 M-1. Antibodies that selectively bind an I2 antigen bind with substantially higher affinity to that antigen than to an unrelated antigen. One skilled in the art understands that other isotypes of anti-I2 antibodies, such as IgG, IgM, IgE, and IgD anti-I2 antibodies, also can be useful.

An I2 antigen is a polypeptide having substantially the same amino acid sequence as the microbial I2 polypeptide (SEQ ID NO: 2) shown in Figure 1. The naturally occurring microbial I2 antigen SEQ ID NO: 2 is a polypeptide of 100 amino acids sharing some similarity to bacterial transcriptional regulators, with the greatest similarity in the amino-terminal 30 amino acids. The naturally occurring I2 SEQ ID NO:2 shares weak homology with the predicted protein 4 from C. pasteurianum; Rv3557c from Mycobacterium tuberculosis; and a transcriptional regulator from Aquifex aeolicus.

The I2 antigen (SEQ ID NO:2) was originally identified by overexpression of the encoding nucleic acid sequence in colonic microbes harbored in inflamed lesions in Crohn's disease patients (Sutton et al., Gastroenterology 119:23-31 (2000)). ELISA analysis showed frequent IgA serum seroreactivity to a recombinant I2 antigen in patients with Crohn's disease but infrequent seroreactivity in patients with ulcerative colitis, other inflammatory enteric disease, or normal individuals (Sutton et al., *supra*, 2000). The I2 antigen is also known to induce a proliferative and IL-10 response by CD4(+) T cells in unimmunized mice (Dalwadi et al., Immunity 15:149-158 (2001)). The I2 response is dependent on MHC classII-mediated recognition and does not require antigen processing. Furthermore, activation is observed for the TCR-Vbeta5 subpopulation of cells, indicating that the I2 antigen is a T cell superantigen (Dalwadi et al., *supra*, 2001). A microbial homologue of I2, PA2885, has been identified in the Pseudomonas aeruginosa genome (Wei et al., Infect. Immun. 70:6567-6575 (2002)). Furthermore, genomic cloning identified a locus containing the full-length I2 gene (pfiT) in *P. aeruginosa* (Wei et al., *supra*, 2002).

An I2 antigen can be the naturally occurring I2 antigen SEQ ID NO: 2 or a related polypeptide having substantial amino acid sequence similarity to this sequence. Such related polypeptides generally exhibit greater sequence similarity to the I2 antigen SEQ ID NO: 2 than to related sequences such as the predicted protein 4 from C. pasteurianum and include isotype variants or homologs of the amino acid sequence shown in Figure 1. As used herein, the term I2 antigen generally describes polypeptides having an amino acid sequence with greater than about 60% identity, greater than about 70% identity, greater than about 80% identity, and can be a polypeptide having greater than abut 90%, 95%, 97%, or 99% amino acid sequence identity with SEQ ID NO: 2, said amino acid identity determined with CLUSTALW using the BLOSUM 62 matrix with default parameters. The C.pasteurianum protein4 has about 21% amino acid identity with the 12 antigen SEQ ID NO: 2 and, therefore, is not an I2 antigen as defined herein.

As disclosed above, the invention provides a method of diagnosing clinical subtype of Crohn's disease in a subject having Crohn's disease by determining the presence or absence of IgA anti-I2 antibodies in the sample from the subject, where the presence of the IgA anti-I2 antibodies indicates that the subject has a certain clinical subtype of Crohn's disease. In one embodiment, the clinical subtype of Crohn's disease is a fibrostenotic subtype of Crohn's disease. In another embodiment, the clinical subtype of Crohn's disease is characterized by the need for,small bowel surgery. In a further embodiment, the clinical subtype of Crohn's disease is characterized by the absence of features of ulcerative colitis.

Crohn's disease represents a number of heterogeneous disease subtypes that affect the gastrointestinal tract and may produce similar symptoms. As used herein in reference to Crohn's disease, the term "clinical subtype" means a classification of Crohn's disease defined by a set of clinical criteria that distinguish one classification of Crohn's disease from another. As non-limiting examples, subjects with Crohn's disease can be classified as having fibrostenotic disease, internal-perforating disease, perianal fistulizing disease, ulcerative colitis (UC)-like disease, the need for small bowel surgery or the absence of features of ulcerative colitis. Subjects with Crohn's disease further can be classified as having complicated Crohn's disease, which is a clinical subtype
characterized by one or more of the following complications: fibrostenosis, internal perforating disease and the need for small bowel surgery. Criteria relating to these subtypes have been described, for example, in Gasche et al., Inflammatory Bowel Diseases 6:8-15 (2000); Vasiliauskas et al., Gut 47:487-496 (2000); Vasiliauskas et al., Gastroenterology 110:1810-1819 (1996); and Greenstein et al., Gut 29:588-592 (1988).

The "fibrostenotic subtype" of Crohn's disease is a classification of Crohn's disease characterized by one or more accepted characteristics of fibrostenosing disease. Such characteristics of fibrostenosing disease include, for example, documented persistent intestinal obstruction or an intestinal resection for an intestinal obstruction. The fibrostenotic subtype of Crohn's disease can be accompanied by other symptoms such as perforations, abscesses or fistulae, and further can be characterized by persistent symptoms of intestinal blockage such as nausea, vomiting, abdominal distention and inability to eat solid food. Intestinal X-rays of patients with the fibrostenotic subtype of Crohn's disease can show, for example, distention of the bowel before the point of blockage.

The requirement for small bowel surgery in a subject with the fibrostenotic subtype of Crohn's disease can indicate a more aggressive form of this subtype. As shown in Example I, patients expressing IgA anti-I2 antibodies were significantly more likely to have the fibrostenotic subtype of Crohn's disease and significantly more likely to require small bowel surgery than those not expressing IgA anti-I2 antibodies. In addition, the amplitude or level of IgA anti-I2 antibodies in a subject can be correlated with the likelihood of having a particular clinical subtype of Crohn's disease. As shown in Example I, quartile analyses revealed that higher levels of IgA anti-I2 antibodies were more strongly associated with the fibrostenotic subtype of Crohn's disease and small bowel involvement and were negatively associated with ulcerative colitis-like Crohn's disease than were lower levels. Furthermore, the greater the number of fibrostenotic markers that a subject possesses, the greater chance that the subject will have an aggressive form of the fibrostenotic subtype of Crohn's disease requiring small bowel surgery (see Example I). For example, a subject with two or more markers can have a more severe form of the fibrostenotic subtype than a patient with one marker.

Additional subtypes of Crohn's disease also are known in the art and can be identified using defined clinical criteria. For example, internal perforating disease is a clinical subtype of Crohn's disease defined by current or previous evidence of entero-enteric or entero-vesicular fistulae, intra-abdominal abscesses, or small bowel perforation. Perianal perforating disease is a clinical subtype of Crohn's disease defined by current or previous evidence of either perianal fistulae or abscesses or rectovaginal fistula. The UC-like clinical subtype of Crohn's disease can be defined by current or previous evidence of left-sided colonic involvement, symptoms of bleeding or urgency, and crypt abscesses on colonic biopsies. Disease location can be classified based on one or more endoscopic, radiologic or pathologic studies.

One skilled in the art understands that overlap can exist between clinical subtypes of Crohn's disease and that a subject having Crohn's disease can have more than one clinical subtype of Crohn's disease. For example, a subject having Crohn's disease can have the fibrostenotic subtype of Crohn's disease and can also meet clinical criteria for a clinical subtype characterized by the need for small bowel surgery or the internal perforating disease subtype. Similarly, the markers described herein can be associated with more than one clinical subtype. For example, IgA anti-OmpC antibodies can be associated with the fibrostenotic subtype, need for small bowel surgery, and internal perforating disease subtypes, and can be independently associated with the internal perforating disease subtype. Also, for example, ASCA can be independently associated with the fibrostenotic subtype, a clinical subtype characterized by the need for small bowel surgery, and the internal perforating disease subtype.

The invention further provides a method of diagnosing said clinical subtypes of Crohn's disease in a subject having Crohn's disease by contacting a sample from the subject with an I2 antigen, or immunoreactive fragment thereof, under conditions suitable to form a complex of I2 antigen, or immunoreactive fragment thereof, and antibody against the I2 antigen; contacting the complex with a labeled secondary antibody; and detecting the presence or absence of the complex, where the presence of the complex indicates the presence of the IgA anti-I2 antibodies in the subject, thereby indicating that the.subject has the clinical subtype of Crohn's disease.

The invention additionally provides a method of diagnosing a fibrostenotic subtype of Crohn's disease in a subject having Crohn's disease by contacting a sample from the subject with an I2 antigen, or immunoreactive fragment thereof, under conditions suitable to form a complex of I2 antigen, or immunoreactive fragment thereof, and antibody against the I2 antigen; contacting the complex with a labeled secondary antibody; and detecting the presence or absence of the complex, where the presence of the complex indicates the presence of the IgA anti-I2 antibodies in the subject, thereby indicating that the subject has the fibrostenotic subtype of Crohn's disease.

A sample useful in the methods of the invention can be obtained from any biological fluid having antibodies such as, for example, whole blood, plasma, saliva, or other bodily fluid or tissue, such as serum. It is understood that a sample to be assayed according to the methods of the invention can be a fresh or preserved sample obtained from a subject to be diagnosed.

As used herein, the term "complex" is synonymous with "immune complex" and means an aggregate of two or more molecules that results from specific binding between an antigen, such as a protein or peptide, and an antibody. In a method of the invention, a complex is formed, for example, by specific binding of an antibody and an I2 antigen or immunoreaction fragment thereof.

As used herein, the term "I2 antigen" means a polypeptide which is immunoreactive with IgA anti-I2 antibodies that immunoreact with SEQ ID NO:2. For example, the amino acid sequence SEQ ID NO:2 can be an I2 antigen. An immunoreactive fragment of the I2 antigen also can be used in the methods of the invention. As used herein, the term "immunoreactive fragment" means a portion of a full-length I2 antigen that retains the ability to form a specific complex with IgA anti-I2 antibodies.

An I2 antigen, or immunoreactive fragment thereof, useful in the invention can be produced or synthesized using methods well known in the art. Such methods include recombinant DNA methods and chemical synthesis methods for production of a peptide. Recombinant methods for producing a polypeptide antigen through expression of a nucleic acid sequence encoding the polypeptide in a suitable host cell are well known in the art and are described, for example, in Ausubel et al., *supra*, 1999.

An I2 antigen, or immunoreactive fragment thereof, useful in the invention also can be produced by chemical synthesis, for example, by the solid phase peptide synthesis method of Merrifield et al., J. Am. Chem. Soc. 85:2149 (1964). Standard solution methods well known in the art also can be used to synthesize an I2 antigen, or immunoreactive fragment thereof (see, for example, Bodanszky, Principles of Peptide Synthesis, Springer-Verlag, Berlin (1984) and Bodanszky, Peptide Chemistry, Springer-Verlag, Berlin (1993)). A newly synthesized polypeptide antigen or immunogenic fragment thereof can be purified, for example, by high performance liquid chromatography (HPLC), and can be characterized using, for example, mass spectrometry or amino acid sequence analysis.

It is understood that limited modifications can be made to an I2 antigen without destroying its ability to bind to IgA anti-I2 antibodies. Similarly, limited modifications can be made to an immunoreactive fragment of an I2 antigen without destroying its immunoreactivity. A modification of an antigen disclosed herein that does not destroy its reactivity with IgA antibodies in the sera of patients having Crohn's disease is within the definition of an I2 antigen. Similarly, a modification of an immunoreactive fragment of an I2 antigen disclosed herein that does not destroy its ability to form a complex with IgA antibodies in the sera of a patient having Crohn's disease is within the definition of an immunoreactive fragment. A modification can be, for example, an addition, deletion, or substitution of amino acid residues; substitution of a compound that mimics amino acid structure or function; or addition of chemical moieties such as amino or acetyl groups. The activity of a modified I2 antigen or a modified immunoreactive fragment of an I2 antigen can be assayed, for example, using one of the assays for immunoreactivity disclosed herein.

A useful modification, for example, is one that confers increased stability. Incorporation of one or more D-amino acids is a modification useful in increasing stability of an I2 antigen or immunoreactive fragment thereof. Similarly, deletion or substitution of lysine can increase stability by protecting against degradation. For example, such a substitution can increase stability of an I2 antigen or an immunoreactive fragment thereof, provided that the substitution does not significantly impair immunoreactive activity.

In the methods of the invention, a complex can be detected with a labeled secondary antibody, for example, that has specificity for a class determining portion of an anti-I2 antibody. Such a secondary antibody can be, without limitation, an anti-IgA secondary antibody, an anti-IgG secondary antibody, or a combination of anti-IgA and anti-IgG secondary antibodies.

As used herein, the term "secondary antibody" means an antibody or combination of antibodies, which binds an antibody that specifically binds an I2 antigen, or an immunoreactive fragment thereof. One skilled in the art understands that, preferably, a secondary antibody does not compete with the I2 antigen for binding to the primary antibody. A secondary antibody can bind any epitope of an anti-I2 antibody. A particularly useful secondary antibody is an anti-IgA or anti-IgG antibody having specificity for the class determining portion of the primary antibody.

It is understood that a useful secondary antibody is specific for the species from which the sample was obtained. For example, if human serum is the sample to be assayed, mouse anti-human IgA or IgG can be a useful secondary antibody. A combination of different antibodies, which can be useful in the methods of the invention, also is encompassed within the meaning of the term secondary antibody, provided that at least one antibody of the combination reacts with an antibody that specifically binds an I2 antigen.

The term class determining portion, when used in reference to a secondary antibody, means the heavy chain constant-region sequence of an antibody that determines the isotype, such as IgA, IgD, IgE, IgG or IgM. Thus, a secondary antibody that has specificity for the class determining portion of an IgA molecule, for example, binds IgA in preference to other antibody isotypes.

A secondary antibody useful in the invention can be obtained commercially or by techniques well known in the art. Such an antibody can be a polyclonal or a monoclonal antibody. For example, IgA reactive polyclonal antibodies can be prepared using IgA or Fc fragments of IgA as an immunogen to stimulate the production of antibodies in the antisera of an animal such as a rabbit, goat, sheep or rodent, as described in Harlow and Lane, Antibodies: A Laboratory Manual New York: Cold Spring Harbor Laboratory (1988). Monoclonal secondary antibodies, which are a population of antibody molecules that contain only one species of idiotope capable of binding a particular antigen epitope also can be produced by routine methods (see, for example, Harlow and Lane, *supra,* 1988) or obtained commercially.

The term "labeled secondary antibody" means a secondary antibody, as defined above, that can be detected or measured by analytical methods. Thus, the term labeled secondary antibody includes an antibody labeled directly or indirectly with a detectable marker that can be detected or measured and used in a convenient assay such as an enzyme-linked immunosorbent assay (ELISA), fluorescent assay, radioimmunoassay, radial immunodiffusion assay or Western blotting assay. A secondary antibody can be labeled, for example, with an enzyme, radioisotope, fluorochrome or chemiluminescent marker. In addition, a secondary antibody can be rendered detectable using a biotin-avidin linkage such that a detectable marker is associated with the secondary antibody. Labeling of the secondary antibody, however, should not impair binding of the secondary antibody to the I2 antigen. If desired, a multiple antibody system can be used as discussed above. In such a system, at least one of the antibodies is capable of binding the primary anti-I2 antibody and at least one of the antibodies can be readily detected or measured by analytical methods.

A secondary antibody can be rendered detectable by labeling with an enzyme such as horseradish peroxidase (HRP), alkaline phosphatase (AP), β-galactosidase or urease, for example. A horseradish-peroxidase detection system can be used, for example, with the chromogenic substrate tetramethylbenzidine (TMB), which yields a soluble product in the presence of hydrogen peroxide that is detectable by measuring absorbance at 450 nm. An alkaline phosphatase detection system can be used with the chromogenic substrate p-nitrophenyl phosphate, for example, which yields a soluble product readily detectable by measuring absorbance at 405 nm. Similarly, a β-galactosidase detection system can be used with the chromogenic substrate o-nitrophenyl-β-D-galactopyranoside (ONPG), which yields a soluble product detectable by measuring absorbance at 410nm, or a urease detection system can be used with a substrate such as urea-bromocresol purple (Sigma Immunochemicals, St. Louis, MO). A secondary antibody can be linked to an enzyme by methods well known in the art (Harlow and Lane, *supra,* 1988) or can be obtained from a number of commercial sources. For example, goat F(ab')2 anti-human IgA-alkaline phosphatase is a useful detectable secondary antibody that can be purchased from Jackson ImmunoResearch (West Grove, PA).

A secondary antibody also can be rendered detectable by labeling with a fluorochrome. Such a fluorochrome emits light of ultraviolet or visible wavelength after excitation by light or another energy source. DAPI, fluorescein, Hoechst 33258, R-phycocyanin, B-phycoerythrin, R-phycoerythrin, rhodamine, Texas red or lissamine are, without limitation, fluorochromes that can be linked to a secondary antibody and used to detect the presence or absence of a complex in a method of the invention. Methods of conjugating and using these and other suitable fluorochromes are described, for example, in Van Vunakis and Langone, Methods in Enzymology, Volume 74, Part C (1991). A secondary antibody linked to a fluorochrome also can be obtained from commercial sources. For example, goat F(ab')2 anti-human IgA-FITC is available from Tago Immunologicals (Burlingame, CA).

A secondary antibody also can be labeled with a chemiluminescent marker. Such a chemiluminescent secondary antibody is convenient for sensitive, nonradioactive detection of a complex containing an I2 antigen and can be obtained commercially from various sources such as Amersham Lifesciences, Inc. (Arlington Heights, IL).

A secondary antibody further can be rendered detectable by labeling with a radioisotope. For example, an iodine-125 labeled secondary antibody is a useful detectable secondary antibody (see, for example, Harlow and Lane, *supra*, 1988).

A signal from a detectable secondary antibody can be analyzed, for example, using a spectrophotometer to detect color from a chromogenic substrate; a fluorometer to detect fluorescence in the presence of light of a certain wavelength; or a radiation counter to detect radiation, such as a gamma counter for detection of iodine-125. For detection of an enzyme-linked secondary antibody, for example, a quantitative analysis can be made using a spectrophotometer such as an EMAX Microplate Reader (Molecular Devices; Menlo Park, CA) in accordance with the manufacturer's instructions. If desired, the assays of the invention can be automated or performed robotically, and the signal from multiple samples can be detected simultaneously.

The assays of the present invention can be forward, reverse or simultaneous as described in U.S. Patent No. 4,376,110, issued March 8, 1983, to David et al. In the forward assay, each reagent is sequentially contacted with an I2 antigen of the invention. If desired, separation of bound from unbound reagent can be performed before the addition of the next reagent. In a reverse assay, all reagents are pre-mixed prior to contacting with I2 antigen. A modified reverse assay is described in U.S. Patent No. 4,778,751 issued October18,1988, to El Shami et al. In a simultaneous assay, all reagents are separately but contemporaneously contacted with an I2 antigen of the invention. A reagent is any component useful in performing the assays of the present invention, for example, the sample, I2 antigen, detectable secondary antibody, washing buffer or other solutions.

Separation steps for the various assay formats described herein, including the removal of unbound secondary antibody from the complex, can be performed by methods known in the art (Harlow and Lane, *supra,* 1988). For example, washing with a suitable buffer can be followed by filtration, aspiration or magnetic separation. If the I2 antigen or an immunoreactive fragment thereof is immobilized on a particulate support, such as on microparticles, the particulate material can be centrifuged, if desired, followed by removal of wash liquid. If the I2 antigen or an immunoreactive fragment thereof is immobilized on a membrane, filter or well, a vacuum or liquid absorbing apparatus can be applied to the opposite side of the membrane, filter or well to draw the wash liquid away from the complex.

Antibody based methods can also be useful for determining the presence or absence of IgA anti-I2 antibodies, anti-Saccharomyces cerevisiae antibodies or other antibodies such as IgA anti-OmpC antibodies, and perinuclear anti-neutrophil cytoplasmic antibodies. Such methods rely on anti-idiotypic antibodies specific to the anti-I2 or other antibody of interest. An anti-idiotypic antibody contains an internal image of the antigen used to create the antibody of interest. Therefore, an anti-idiotypic antibody can bind to an anti-I2 antibody or other marker antibody of interest. Methods of making, selecting and using anti-idiotype antibodies are well known in the art. See, for example, Eichmann, et al., CRC Critical Reviews in Immunology 7:193-227 (1987).

An in vitro method of the invention for diagnosing a certain clinical subtype of Crohn's disease in a subject having Crohn's disease by determining the presence or absence of IgA ant-I2 antibodies in the subject can optionally include the additional step of determining the presence or absence in the subject of a NOD2 variant selected from R702W, G908R and 1007fs anti-Saccharomyces cerevisiae antibodies, or IgA anti-OmpC antibodies.

As used herein, the term "marker" means a serological, genetic or other biochemical factor, the presence of which correlates with a clinical subtype of Crohn's disease. Markers for clinical subtypes of Crohn's disease include, without limitation, IgA anti-I2 antibodies, NOD2 variants, anti-Saccharomyces cerevisiae antibodies, IgA anti-OmpC antibodies, and perinuclear anti-neutrophil cytoplasmic antibodies. As used herein, the term "fibrostenotic marker" means a serological, genetic or other biochemical factor, the presence of which correlates with the fibrostenotic subtype of Crohn's disease. Non-limiting examples of fibrostenotic markers useful in the invention include IgA anti-I2 antibodies; NOD2 variants R702W, G908R and 1007fs; anti-Saccharomyces cerevisiae antibodies; anti-OmpC antibodies; antibodies to other bacterial responsive antigens, and markers associated with other types of fibrostenotic disease such as fibrostenosis of the liver. As shown in Example I, the greater the number of fibrostenotic markers that a subject possesses, the greater the chance that the subject will have an aggressive form of the fibrostenotic subtype of Crohn's disease requiring small bowel surgery.

Several methods of the invention involve determining the presence and magnitude of one or more markers such as IgA anti-I2 antibodies, ASCA and IgA anti-OmpC antibodies. One technique for quantifying the magnitude of an antibody response is "quartile analysis," in which each patient response is defined as in the first quartile (0-25%); second quartile (25-50%); third quartile (50-75%) or fourth quartile (75-100%) in relation to a reference database of Crohn's disease patients. Such a fixed database generally should include a large spectrum of Crohn's disease patients and can be, for example, the 303 patient database described herein. From such a database, quartile cut-offs are established, for example, as described herein and shown in Figure 7. One skilled in the art further understands that quartile cut-offs or other cut-offs further can be established using another large Crohn's disease patient database such as, for example, quartile cut-offs determined from sera from 500 known Crohn's disease patients with mild to severe disease from a subspecialty practice such as a gastroenterology practice or an exclusively inflammatory bowel disease (IBD) practice in an academic or private setting.

A NOD2 variant is a fibrostenotic marker useful in the methods of the invention. As used herein, the term "NOD2 variant" means a nucleotide sequence of a NOD2 gene containing one or more changes as compared to the wild-type NOD2 gene or an amino acid sequence of a NOD2 polypeptide containing one or more changes as compared to the wild-type NOD2 polypeptide sequence. NOD2, also known as CARD15, has been localized to the IBD1 locus on chromosome 16 and identified by positional-cloning (Hugot et al., Nature 411:599-603 (2001)) as well as a positional candidate gene strategy (Ogura et al., Nature 411:603-606 (2001), Hampe et al., Lancet 357:1925-1928 (2001)). The IBD1 locus has a high multipoint linkage score (MLS) for inflammatory bowel disease (MLS = 5.7 at marker D16S411 in 16q12). See Cho et al., Inflamm. Bowel Dis. 3:186-190 (1997), Akolkar et al., Am. J. Gastroenterol. 96:1127-1132 (2001), Ohmen et al., Hum. Mol. Genet. 5:1679-1683 (1996), Parkes et al., Lancet 348:1588 (1996), Cavanaugh et al., Ann. Hum. Gent. (1998), Brant et al., Gastroenterology 115:1056-1061 (1998), IF Curran et al., Gastroenterology 115:1066-1071 (1998), Hampe et al., Am. J. Hum. Genet. 64:808-816 (1999), and Annese et al., Eur. J. Hum. Genet. 7:567-573 (1999).

The sequence of the human NOD2 gene can be found in GenBank as accession number NM_022162. In addition, the complete sequence of human chromosome 16 clone RP11-327F22, which includes NOD2, can be found in GenBank as accession number AC007728. Furthermore, the sequence of NOD2 from other species can be found in the GenBank database. A schematic of the NOD2 locus is shown in Figure 2A.

The NOD2 protein contains amino-terminal caspase recruitment domains (CARDs), which can activate NF-kappa B (NF-kB), and several carboxy-terminal leucine-rich repeat domains (Ogura et al, J. Biol. Chem. 276:4812-4818 (2001)). NOD2 has structural homology with the apoptosis regulator Apaf-1/CED-4 and a class of plant disease resistant gene products (Ogura et al., *supra,* 2001). Similar to plant disease resistant gene products, NOD2 has an amino-terminal effector domain, a nucleotide-binding domain and leucine rich repeats (LRRs). Wild-type NOD2 activates nuclear factor NF-kappa B, making it responsive to bacterial lipopolysaccharides (LPS; Ogura et al., *supra*, 2001; Inohara et al., J. Biol. Chem. 276:2551-2554 (20.01). NOD2 can function as an intercellular receptor for LPS, with the leucine rich repeats required for responsiveness. Three single nucleotide polymorphisms in the coding region of NOD2 have been previously described. These three SNPs, designated R702W, G908R and 1007fs, are located in the carboxy-terminal region of the NOD2 gene (Hugot et al., *supra*, 2001).

Two of the NOD2 variants used in the invention are located in a coding region of the NOD2 locus, namely, within a region encoding several leucine-rich repeats in the carboxy-terminal portion of the NOD2 polypeptide: R702W and G908R. A NOD2 variant useful in the invention also can encode a NOD2 polypeptide with reduced ability to activate NF-kappa B as compared to NF-kappa B activation by a wild-type NOD2 polypeptide: the NOD2 variant 1007fs results in a truncated NOD2 polypeptide which has reduced ability to induce NF-kappa B in response to LPS stimulation (Ogura et al., Nature 411:603-606 (2001)).

A NOD2 variant useful in the invention is R702W, G908R, or 1007fs. R702W, G908R, and 1007fs are located within the coding region of NOD2 as shown in Figure 2A. In one embodiment, a method of the invention is practiced with the R702W NOD2 variant. As used herein, the term "R702W" means a single nucleotide polymorphism within exon 4 in the NOD2 gene, which occurs within a triplet encoding amino acid 702 of the NOD2 protein. The wild-type NOD2 allele contains a cytosine (c) residue at position 138,991 of the AC007728 sequence, which occurs within a triplet encoding an arginine at amino acid702. The R702W NOD2 variant contains a thymine (t) residue at position 138,991 of the AC007728 sequence, resulting in an arginine (R) to tryptophan (W) substitution at amino acid 702 of the NOD2 protein. Accordingly, this NOD2 variant is denoted "R702W" or "702W" and can also be denoted "R675W" based on the earlier numbering system of Hugot et al., *supra,* 2001. In addition, the R702W variant is also known as the SNP8 allele or a "2" allele at SNP 8. The NCBI SNP ID number for R702W or SNP 8 is rs2066844. As disclosed herein and described further below, the presence of the R702W NOD2 variant and other NOD2 variants can be conveniently detected, for example, by allelic discrimination assays or sequence analysis. Primers and probes specific for the R702W NOD2 variant can be found in Tables 1 and 2 in ExampleIV and in Figure 2B.

A method of the invention also can be practiced with the G908R NOD2 variant. As used herein, the term "G908R" means a single nucleotide polymorphism within exon 8 in the NOD2 gene, which occurs within a triplet encoding amino acid 908 of the NOD2 protein (see Figure 2C). Amino acid 908 is located within the leucine rich repeat region of the NOD2 gene. The wild-type NOD2 allele contains a guanine (g) residue at position 128,377 of the AC007728 sequence, which occurs within a triplet encoding glycine at amino acid 908. The G908R NOD2 variant contains a cytosine (c) residue at position 128,377 of the AC007728 sequence, resulting in a glycine (G) to arginine (R) substitution at amino acid 908 of the NOD2 protein. Accordingly, this NOD2 variant is denoted "G908R" or "908R" and can also be denoted "G881R" based on the earlier numbering system of Hugot et al., *supra,* 2001. In addition, the G908R variant is also known as the SNP 12 allele or a "2" allele at SNP12. The NCBI SNP ID number for G908R SNP12 is rs2066845. Primers and probes specific for the G908R NOD2 variant can be found in Tables 1 and 2 in Example IV and in Figure 2C.

A method of the invention also can be practiced with the 1007fs NOD2 variant. This variant is an insertion of a single nucleotide that results in a frame shift in the tenth leucine-rich repeat of the NOD2 protein and is followed by a premature stop codon. The resulting truncation of the NOD2 protein appears to prevent activation of NF-kappaB in response to bacterial lipopolysaccharides (Ogura et al., *supra,* 2001). As used herein, the term "1007fs" means a single nucleotide polymorphism within exon 11 in the NOD2 gene, which occurs in a triplet encoding amino acid 1007 of the NOD2 protein. The 1007fs variant contains a cytosine which has been added at position 121,139 of the AC007728 sequence, resulting in a frame shift mutation at amino acid 1007. Accordingly, this NOD2 variant is denoted "1007fs" and can also be denoted "3020insC," or "980fs" based on the earlier numbering system of Hugot et al., *supra,* 2001. In addition, the 1007fs NOD2 variant is also known as the SNP 13 allele or a "2" allele at SNP 13. The NCBI SNP ID number for 1007fs or SNP 13 is rs2066847. Primers and probes specific for the 1007fs NOD2 variant can be found in Tables 1 and 2 in Example IV and in Figure 2D.

A variety of means can be useful for determining the presence or absence of a NOD2 variant in a method of the invention. Since a NOD2 variant can be a nucleotide sequence of a NOD2 gene containing one or more changes as compared to the wild-type NOD2 gene or an amino acid sequence of an NOD2 polypeptide containing one or more changes as compared to the wild-type NOD2 polypeptide sequence, genetic, serological and other biochemical methods can be useful. As an example, enzymatic amplification of nucleic acid from a subject can be conveniently used to obtain nucleic acid for subsequent genetic analysis. The presence or absence of a NOD2 variant also can be determined directly from the individual's nucleic acid without enzymatic amplification. Analysis of nucleic acid from a subject, whether amplified or not, can be performed using any of various techniques, including, without limitation, polymerase chain reaction based analysis, sequence analysis and electrophoretic analysis. Techniques can be used alone or in combination.

The presence or absence of a NOD2 variant or another genetic marker can involve amplification of an individual's nucleic acid by the polymerase chain reaction. The nucleic acid to be amplified can be a single- or double-stranded DNA or RNA molecule, including, for example, genomic DNA, cDNA and mRNA. Use of the polymerase chain reaction for amplification of nucleic acids is well known in the art (see, for example, Mullis et al. (Eds.), The Polymerase Chain Reaction, Birkhäuser, Boston, (1994)). Polymerase chain reaction amplification for determining the presence of a NOD2 variant or other genetic marker can be performed, if desired, using one or more fluorescently labeled primers, or using one or more labeled or unlabeled primers that contain a DNA minor grove binder, as in the Taqman® assay described below.

Any of a variety of different primers can be used to amplify an individual's nucleic acid by the polymerase chain reaction in order to determine the presence or absence of a NOD2 variant or other genetic marker in a method of the invention. For example, the PCR primers listed in Table 1 (SEQ ID NOS:11-16) can be used to amplify specific regions of the NOD2 locus. As non-limiting examples, the region surrounding R702W can be amplified using SEQ ID NO: 11 and12; G908R can be amplified using SEQ ID NOS: 13 and 14, and the region surrounding 1007fs can be amplified using SEQ ID NOS: 15 and 16. As understood by one skilled in the art, additional primers for PCR analysis can be designed based on the sequence flanking the NOD2 or other region of interest. Such primers generally contain about 12 to 30 nucleotides of a sequence upstream or downstream of the region of interest and are generally designed to have sufficient guanine and cytosine content to attain a high melting temperature which allows for a stable annealing step in the amplification reaction. Several computer programs, such as Primer Select, are available to aid in the design of PCR primers.

A Taqman® allelic discrimination assay available from Applied Biosystems can be useful for determining the presence or absence of a NOD2 variant or other genetic marker in a method of the invention. In a Taqman® allelic discrimination assay, a specific, fluorescent, dye-labeled probe for each allele is constructed. Each probe contains a different fluorescent reporter dye such as FAM or VICTM to differentiate the amplification of each allele. In addition, each probe has a quencher dye at one end which reduces fluorescence by fluorescence resonance energy transfer (FRET). During PCR, each probe anneals specifically to complementary sequences in the nucleic acid from the individual. The 5' nuclease activity of Taq polymerase is used to cleave only probe that specifically hybridizes to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. It is understood that improved specificity in allelic discrimination assays can be achieved by conjugating a DNA minor grove binder (MGB) group to a DNA probe as described, for example, in Kutyavin et al., Nucleic Acids Research 28:655-661 (2000). Minor grove binders include, but are not limited to, compounds such as dihydrocyclopyrroloindole tripeptide (DPI3).

Sequence analysis also can be useful for determining the presence or absence of a NOD2 variant or other genetic marker in a method of the invention. A NOD2 variant can be detected by sequence analysis using primers disclosed herein, for example, the PCR primers listed in Table 1 (SEQ ID NOS:11-16). As understood by one skilled in the art, additional primers for sequence analysis can be designed based on the sequence flanking the NOD2 region of interest. As a non-limiting example, a sequence primer can contain about 15 to 30 nucleotides of a sequence about 40 to 400 base pairs upstream or downstream of the region of interest. Such sequencing primers are generally designed to have sufficient guanine and cytosine content to attain a high melting temperature which allows for a stable annealing step in the sequencing reaction.

Sequence analysis refers to any manual or automated process by which the order of nucleotides in the nucleic acid is determined. As an example, sequence analysis can be used to determine the nucleotide sequence of a sample of DNA. The term sequence analysis encompasses, without limitation, chemical and enzymatic methods such as dideoxy enzymatic methods including, for example, Maxam-Gilbert and Sanger sequencing as well as variations thereof. The term sequence analysis further encompasses, but is not limited to, capillary array DNA sequencing, which relies on capillary electrophoresis and laser-induced fluorescence detection and can be performed using instruments such as the MegaBACE 1000 or ABI3700. As additional non-limiting examples, the term sequence analysis encompasses thermal cycle sequencing (Sears et al., Biotechniques 13:626-633 (1992)); solid-phase sequencing (Zimmerman et al., Methods Mol. Cell Biol. 3:39-42 (1992); and sequencing with mass spectrometry such as matrix-assisted laser desorption/ionization time-of-flight mass spectrometry MALDI-TOF MS (Fu et al., Nature Biotech. 16: 381-384 (1998)). The term sequence analysis also includes, yet is not limited to, sequencing by hybridization (SBH), which relies on an array of all possible short oligonucleotides to identify a segment of sequences present in an unknown DNA (Chee et al., Science 274:610-614 (1996); Drmanac et al., Science 260:1649-1652 (1993); and Drmanac et al., Nature Biotech. 16:54-58 (1998)). One skilled in the art understands that these and additional variations are encompassed by the term sequence analysis as defined herein. See, in general, Ausubel et al., *supra,* Chapter 7 and supplement 47.

Genetic methods for determining the presence or absence of a NOD2 variant or other genetic marker utilize a subject's biological matter from which nucleic acid can be prepared. As non-limiting examples, a subject's biological matter can be whole blood, plasma, saliva, cheek swab, or other bodily fluid or tissue that contains nucleic acid. In one embodiment, detecting the presence or absence of a NOD2 variant or other genetic marker is practiced with whole blood, which can be obtained readily by non-invasive means and used to prepare genomic DNA, for example, for enzymatic amplification or automated sequencing. In another embodiment, detecting the presence or absence of a NOD2 variant or other genetic marker is practiced with tissue obtained from an individual such as tissue obtained during surgery or biopsy procedures.

Electrophoretic analysis also can be useful in the methods of the invention. Elecrophoretic analysis, as used herein in reference to one or more nucleic acids such as amplified fragments, means a process whereby charged molecules are moved through a stationary medium under the influence of an electric field. Electrophoretic migration separates nucleic acids primarily on the basis of their charge, which is in proportion to their size, with smaller molecules migrating more quickly. The term electrophoretic analysis includes, without limitation, analysis using slab gel electrophoresis, such as agarose or polyacrylamide gel electrophoresis, or capillary electrophoresis. Capillary electrophoretic analysis generally occurs inside a small-diameter (50-100 m) quartz capillary in the presence of high (kilovolt-level) separating voltages with separation times of a few minutes. Using capillary electrophoretic analysis, nucleic acids are conveniently detected by UV absorption or fluorescent labeling, and single-base resolution can be obtained on fragments up to several hundred base pairs. Such methods of electrophoretic analysis, and variations thereof, are well known in the art, as described, for example, in Ausubel et al., Current Protocols in Molecular Biology Chapter 2 (Supplement 45) John Wiley & Sons, Inc. New-York (1999).

Restriction fragment length polymorphism (RFLP) analysis also can be useful for determining the presence or absence of a NOD2 variant or other genetic marker in a method of the invention (Jarcho et al. in Dracopoli et al., Current Protocols in Human Genetics pages 2.7.1-2.7.5, John Wiley & Sons, New York; Innis et al.,(Ed.), PCR Protocols, San Diego: Academic Press, Inc. (1990)). As used herein, restriction fragment length polymorphism analysis is any method for distinguishing genetic polymorphisms using a restriction enzyme, which is an endonuclease that catalyzes the degradation of nucleic acid and recognizes a specific base sequence, generally a palindrome or inverted repeat. One skilled in the art understands that the use of RFLP analysis depends upon an enzyme that can differentiate two alleles at a polymorphic site.

Allele-specific oligonucleotide hybridization also can be used to detect the presence or absence of a NOD2 variant or other genetic marker. Allele-specific oligonucleotide hybridization is based on the use of a labeled oligonucleotide probe having a sequence perfectly complementary, for example, to the sequence encompassing a NOD2 variant. Under appropriate conditions, the allele-specific probe hybridizes to a nucleic acid containing the NOD2 variant but does not hybridize to the one or more other alleles, which have one or more nucleotide mismatches as compared to the probe. If desired, a second allele-specific oligonucleotide probe that matches an alternate allele also can be used. Similarly, the technique of allele-specific oligonucleotide amplification can be used to selectively amplify, for example, a NOD2 variant by using an allele-specific oligonucleotide primer that is perfectly complementary to the nucleotide sequence of the NOD2 variant but which has one or more mismatches as compared to other alleles (Mullis et al., *supra,* 1994). One skilled in the art understands that the one or more nucleotide mismatches that distinguish between the NOD2 variant and one or more other alleles are often located in the center of an allele-specific oligonucleotide primer to be used in allele-specific oligonucleotide hybridization. In contrast, an allele-specific oligonucleotide primer to be used in PCR amplification generally contains the one or more nucleotide mismatches that distinguish between the subtype-associated and other alleles at the 3' end of the primer.

A heteroduplex mobility assay (HMA) is another well known assay that can be used to detect the presence or absence of a NOD2 variant or other genetic marker in a method of the invention. HMA is useful for detecting the presence of a polymorphic sequence since a DNA duplex carrying a mismatch has reduced mobility in a polyacrylamide gel compared to the mobility of a perfectly base-paired duplex (Delwart et al., Science 262:1257-1261 (1993); White et al., Genomics 12:301-306 (1992)).

The technique of single strand conformational polymorphism (SSCP) also can be used to detect the presence or absence of a NOD2 variant or other genetic marker in a method of the invention (see Hayashi, Methods Applic. 1:34-38 (1991)). This technique is used to detect mutations based on differences in the secondary structure of single-strand DNA that produce an altered electrophoretic mobility upon non-denaturing gel electrophoresis. Polymorphic fragments are detected by comparison of the electrophoretic pattern of the test fragment to corresponding standard fragments containing known alleles.

Denaturing gradient gel electrophoresis (DGGE) also can be used to detect a NOD2 variant or other genetic marker in a method of the invention. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (Sheffield et al., "Identifying DNA Polymorphisms by Denaturing Gradient Gel Electrophoresis" in Innis et al., *supra,* 1990).

Other molecular methods useful for determining the presence or absence of a NOD2 variant or other genetic marker are known in the art and useful in the methods of the invention. Other well-known approaches for determining the presence or absence of a NOD2 variant include, without limitation, automated sequencing and RNAase mismatch techniques (Winter et al., Proc. Natl. Acad. Sci. 82:7575-7579 (1985)). Furthermore, one skilled in the art understands that, where the presence or absence of multiple NOD2 variants is to be determined, individual NOD2 variants can be detected by the same or any combination of molecular methods. See, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple NOD2 variants or other genetic markers can be detected in individual reactions or in a single reaction (a "multiplex" assay). In view of the above, one skilled in the art realizes that the methods of the invention for diagnosing or predicting susceptibility to a clinical subtype of Crohn's disease, such as the fibrostenotic subtype, can be practiced using one or any combination of the well known assays described above or known in the art.

Antibody based methods also can be useful for determining the presence or absence of a NOD2 variant in a method of the invention. As an example, an antibody that is specifically reactive with a NOD2 variant polypeptide or fragment thereof can be used to detect the presence or absence of that NOD2 variant in an individual. Such an antibody can be, for example, specifically reactive with the truncated version of NOD2 generated by the 1007fs NOD2 variant but not reactive with full-length or wild type NOD2.

Antibodies useful in the methods of the invention include, without limitation, monoclonal and polyclonal antibodies, single chain antibodies, chimeric antibodies, bifunctional or bispecific antibodies, humanized antibodies, human antibodies, and complementary determining region (CDR)-grafted antibodies, including compounds which include CDR or antigen-binding sequences, which differentially bind to a polypeptide or fragment encoded by a NOD2 variant but not to other non-variant sequences. Antibody fragments, including Fab, Fab', F(ab')2, and Fv, also can be useful in the methods of the invention as can plastic antibodies or molecularly imprinted polymers (MIPs; Haupt and Mosbauch, Trends in Biotech. 16:468-475 (1998)). Screening assays to determine differential binding specificity of an antibody are well known in the art (see Harlow et al. (Eds), Antibodies: A Laboratory Manual; Cold Spring Harbor Laboratory; Cold Spring Harbor, N.Y. (1988)).

Antibodies useful in a method of the invention can be produced using any method well known in the art, using a polypeptide, or immunogenic fragment thereof, encoded by a NOD2 variant. Immunogenic polypeptides or fragments can be isolated, for example, from natural sources or recombinant host cells, or can be chemically synthesized. Methods for synthesizing such peptides are known in the art as described, for example, in Merrifield, J. Amer. Chem. Soc. 85: 2149-2154 (1963), and Krstenansky et al., FEBS Lett. 211:10 (1987).

Antibodies that differentially bind to NOD2 variants of the invention can be labeled with a detectable label and used to detect the presence, absence or amount of the encoded polypeptide in vivo, in vitro, or in situ. A moiety, such as a fluorescent molecule, can be linked to an antibody for use in a method of the invention using, for example, carbodiimide conjugation (Bauminger and Wilchek, Meth. Enzymol. 70:151-159 (1980)).

In a method of the invention, antibodies that differentially bind to a NOD2 variant can be used in immunoassays to determine the presence or absence of a NOD2 variant in a subject having Crohn's disease. Immunoassays include, without limitation, radioimmunoassays, enzyme-linked immunosorbent assays (ELISAs) and immunoassays with fluorescently labeled antibodies, which are well known in the art. Antibodies can also be used to detect the presence or absence of a NOD2 variant or other fibrostenotic marker in a cell or tissue using immunohistochemistry or other in situ assays. Furthermore, cells containing a polypeptide of interest either on the surface of the cell or internally can be detected by an antibody using assays such as fluorescence activated cell sorting (FACS). One skilled in the art understands that these and other routine assays can be useful for determining the presence or absence of a NOD2 variant according to a method of the invention.

Antibodies can be used to detect the presence or absence of a polypeptide of interest, such as IgA anti-I2 antibodies, an NOD2 variant, anti-Saccharomyces cerevisiae antibodies, IgA anti-OmpC antibodies, and perinuclear anti-neutrophil cytoplasmic antibodies, for example, directly from a blood sample. One skilled in the art understands that when the presence or absence of multiple markers is determined, the same or a different sample can be used.

As disclosed above, the invention provides an in vitro method of diagnosing a certain clinical subtype of Crohn's disease in a subject having Crohn's disease by determining the presence or absence of IgA anti-I2 antibodies in the subject and optionally determining the presence or absence in the subject of anti-Saccharomyces cerevisiae antibodies (ASCA).

Anti-Saccharomyces cerevisiae antibodies (ASCA) are a fibrostenotic marker useful in the invention. As disclosed herein, the presence of ASCA can be used to diagnose or predict susceptibility to a fibrostenotic subtype of Crohn's disease in a subject having Crohn's disease (see Example I). The presence of ASCA can be determined by well known methods such as by reactivity with purified yeast cell wall phosphopeptidomannan (PPM), which can be prepared, for example, from ATCC strain #38926. Methods for determining the presence of ASCA are exemplified herein in Example III. As used herein, "ASCA" means antibody reactivity against S.cerevisiae that is greater than the reactivity observed with control (normal subject) sera analyzed under the same conditions.

Anti-Saccharomyces cerevisiae antibodies (ASCA) are characteristically elevated in patients having Crohn's disease although the nature of the S. cerevisiae antigen supporting the specific antibody response in Crohn's disease is unknown (Sendid et al., Clin. Diag. Lab. Immunol., 3:219-226 (1996)). These antibodies may represent a response against yeasts present in common food or drink or a response against yeasts that colonize the gastrointestinal tract. Studies with periodate oxidation have shown that the epitopes recognized by ASCA in Crohn's disease patient sera contain polysaccharides. Oligomannosidic epitopes are shared by a variety of organisms including different yeast strains and genera, filamentous fungi, viruses, bacteria and human glycoproteins. Thus, the mannose-induced antibody responses in Crohn's disease may represent a response against a pathogenic yeast organism or may represent a response against a cross-reactive oligomannosidic epitope present, for example, on a human glycoprotein autoantigen. Regardless of the nature of the antigen, elevated levels of serum ASCA are a differential marker for Crohn's disease, with only low levels of ASCA reported in UC patients (Sendid et al., *supra,* 1996). Using multiple regression analysis, higher ASCA levels in subjects with Crohn's disease were shown to be independently associated with early age of disease onset as well as both fibrostenosing and internal penetrating disease behaviors (Vasiliauskas et al., Gut 47:487-497 (2000)).

The presence or absence of ASCA can be determined using an antigen specific for ASCA, which is any antigen or mixture of antigens that is bound specifically by ASCA. Although ASCA antibodies were initially characterized by their ability to bind S. cerevisiae, those of skill in the art will understand that an antigen specific for ASCA can be obtained from S. cerevisiae, or can be obtained from a variety of other sources so long as the antigen is capable of binding specifically to ASCA antibodies. Accordingly, exemplary sources of an antigen specific for ASCA contemplated for use in the methods of the invention include whole killed yeast cells, such as from the genera Saccharomyces and Candida, yeast cell wall phosphopeptidomannan (PPM), oligomannosides, neoglycolipids, anti-ASCA idiotypic antibodies, and the like. As described above, different species and strains of yeast, including Saccharomyces, can be used as an antigen specific for ASCA in the methods provided herein. For example, S. cerevisiae strain Su1, Su2, CBS 1315 or BM 156, or Candida albicans strain VW32, can be used as an antigen specific for ASCA in the methods of the invention.

Preparations of yeast cell wall mannans, or phosphopeptidomannans (PPM), are also contemplated herein as antigens specific for ASCA. These water soluble surface antigens can be prepared by appropriate extraction techniques, including autoclaving as described in ExampleIII or can be obtained commercially (see Lindberg et al., Gut 33:909-913 (1992)). The acid stable fraction of yeast cell wall PPM also can be useful in the methods of the invention (Sendid et al., *supra,* 1996). An exemplary PPM for use in diagnosing clinical subtypes of Crohn's disease is derived from S. cerevisiae strain ATCC #38926.

Purified oligosaccharide antigens, such as oligomannosides specific for ASCA, also are contemplated for use in determining the presence or absence of ASCA in the methods of the invention. Purified oligomannoside antigens can be converted, if desired, into neoglycolipids as described in Faille et al., Eur. J. Microbiol. Infect. Dis. 11:438-446 (1992). One skilled in the art understands that the reactivity of such an oligomannoside antigen with ASCA can be optimized by varying the mannosyl chain length (Frosh et al., Proc. Natl. Acad. Sci. USA 82:1194-1198 (1985)); the anomeric configuration (Fukazawa et al., In E. Kurstak (ed.), Immunology of Fungal Disease, Marcel Dekker Inc., New York, pp. 37-62 (1989); Nishikawa et al, Microbiol. Immunol. 34:825-840 (1990); Poulain et al., Eur. J. Clin. Microbiol. 23:46-52 (1993); Shibata et al., Arch. Biochem. Biophys. 243:338-348 (1985); and Trinel et al., Infect. Immun. 60:3845-3851 (1992)); or the position of the linkage (Kikuchi et al., Planta 190:525-535 (1993)).

An oligomannoside antigen specific for ASCA can include the mannotetraose Man(1β3)Man(1β2)Man(1β2)Man, and can be purified from PMM as described in Faille et al., *supra,* 1992. An exemplary neoglycolipid for use in the methods of the invention can be constructed by releasing the oligomannoside from its respective PPM and subsequently coupling the released oligomannoside to 4-hexadecylaniline or the like. These and other antigens specific for ASCA can be used in determining the presence or absence of ASCA in the methods of the invention.

IgA anti-OmpC antibodies are another marker useful for determining a clinical subtype of Crohn's disease in a method of the invention. IgA anti-OmpC antibodies are associated with the fibrostenotic subtype, need for small bowel surgery, and internal perforating disease subtype, and can be independently associated with the internal perforating disease subtype. Provided herein is a method of diagnosing or predicting susceptibility to a clinical subtype of Crohn's disease in a subject having Crohn's disease by determining the presence or absence of IgA anti-OmpC antibodies in the subject, where the presence of IgA anti-OmpC antibodies indicates that the subject has a clinical subtype of Crohn's disease. In one embodiment, the clinical subtype of Crohn's disease is the fibrostenotic subtype. In another embodiment, the clinical subtype of Crohn's disease is the internal perforating disease subtype.

The presence of IgA anti-OmpC antibodies in a subject can indicate that the subject has a fibrostenotic subtype of Crohn's disease. In some cases, the presence of IgA anti-OmpC antibodies can correlate with the presence of ASCA. In some embodiments, the presence of IgA anti-OmpC antibodies and ASCA are determined, while in other embodiments the presence of IgA anti-OmpC antibodies can be used as a surrogate marker for the presence of ASCA.

The outer-membrane protein C (OmpC) is a porin, a class of transmembrane proteins that are found in the outer membranes of bacteria, including gram-negative enteric bacteria such as E. coli. The porins in the outer membrane of an E. coli cell provide channels for passage of disaccharides, phosphate and similar molecules. Porins can be trimers of identical subunits arranged to form a barrel-shaped structure with a pore at the center (Lodish et al., Molecular Cell Biology, Chapter 14 (1995)).

OmpC is one of the major porin proteins found in the outer membranes of bacteria such as E. coli. An OmpC antigen can be prepared, for example, from an encoding nucleic acid sequence such as that available as GenBank accession K00541 or as shown in Figure 3A by methods well known in the art (see, for example, Ausubel et al., Current Protocols in Molecular Biology John Wiley & Sons, Inc. New York (1999)). OmpC is similar in structure and function to outer-membrane protein F ("OmpF"). Both assemble as trimers in the outer membrane to form aqueous channels that allow the passive diffusion of small, hydrophilic molecules across the hydrophobic barrier. However, OmpC pores have a diameter of 1.1 nm, while OmpF pores have a diameter of 1.2 nm. This difference results in a slower rate of diffusion through the OmpC pores than through the OmpF pores.

Porin expression can be influenced by environmental conditions, including osmolarity, temperature, growth phase and toxin concentration. For example, in the intestine, where both nutrient and toxic molecule concentrations are relatively high, OmpC, with a smaller pore diameter, is the predominant porin (Pratt et al., Mol. Micro., 20:911-917 (1996)).

The methods of the invention relate to determining the presence or absence of IgA anti-OmpC antibodies in a subject having Crohn's disease. As used herein, the term "IgA anti-OmpC antibodies" means IgA reactivity against an OmpC antigen that is greater than two standard deviations above the mean IgA anti-OmpC reactivity of control (normal) sera analyzed under the same conditions. Detection of IgA anti-OmpC antibodies using an ELISA is described herein in Example V.

Another marker is perinuclear anti-neutrophil cytoplasmic antibodies (pANCA). Previous studies have shown pANCA reactivity in a small portion of patients with Crohn's disease, although these antibodies are elevated more frequently in patients with ulcerative colitis. The reported prevalence in Crohn's disease varies from 0 to 43%, with most studies reporting that 10 to 30% of Crohn's disease patients express pANCA (see, for example, Saxon et al., J. Allergy Clin. Immunol. 86:202--210 (1990); Cambridge et al., Gut 33:668-674 (1992); Pool et al., Gut 3446-50 (1993); and Brokroelofs et al., Dig. Dis. Sci. 39:545-549 (1994). In subjects with Crohn's disease, serum pANCA expression characterizes a UC-like clinical phenotype of the disease (Vasiliauskas et al., Gastroenterology 110:1810-1819 (1996)).

A further method involves determining the presence or absence of I2 antibodies and optionally determining in a subtype having Crohn's disease, the presence or absence of pANCA in the subject, for example, by reactivity with fixed neutrophil. As used herein, the term "perinuclear anti-neutrophil cytoplasmic antibody" is synonymous with "pANCA" and refers to an antibody that reacts specifically with a neutrophil to give perinuclear to nuclear staining or cytoplasmic staining with perinuclear highlighting. A method for determining the presence of pANCA in a subject is exemplified herein in Example VI.

In one embodiment, the invention provides an in vitro method of diagnosing a fibrostenotic subtype of Crohn's disease in a subject having Crohn's disease by determining the presence or absence of IgA anti-I2 antibodies in the subject, and further determining the presence or absence in the subject of one or more fibrostenotic markers such as a NOD2 variant, anti-Saccharomyces cerevisiae antibodies (ASCA), or anti-OmpC antibodies, where the presence of IgA anti-I2 antibodies or the presence of one of the fibrostenotic markers indicates that the subject has the fibrostenotic subtype of Crohn's disease.

As shown in Example I, the presence of IgA anti-I2 antibodies alone indicated that a subject was more likely to have a fibrostenotic subtype of Crohn's disease than those not expressing IgA anti-I2 antibodies (71.4% vs. 43.3%, p<0.001) and significantly more likely to require small bowel surgery (66.7% vs. 37.1%, p< 0.001). In addition, as shown in Example I, conditional analysis performed on NOD2 variants and ASCA indicated that IgA anti-I2 antibodies were independently associated with the fibrostenotic subtype (p=0.001 and p=0.005 respectively). Similarly, IgA anti-I2 antibodies were independently associated with small bowel surgery when conditioned on NOD2 variation (p= 0.001) or ASCA (p=0.002) (see Example I).

As disclosed herein in Example I, combinations of markers can be diagnostic for a subtype of Crohn's disease. For example, the invention provides a method of diagnosing a fibrostenotic subtype of Crohn's disease in a subject having Crohn's disease by determining the presence or absence of IgA anti-I2 antibodies in the subject, and further determining the presence or absence of a NOD2 variant in the subject, where the combined presence of IgA anti-I2 antibodies and a NOD2 variant in the subject indicates that the subject has the fibrostenotic subtype of Crohn's disease. In one embodiment, the combined presence of the IgA anti-I2 antibodies and the NOD2 variant in the subject is associated with the fibrostenotic subtype of Crohn's disease with an odds ratio of at least 6.

The strength of an association between one or more markers and a clinical subtype of Crohn's disease can be characterized by a particular odds ratio such as an odds ratio of at least 6. Such an odds ratio can be, for example, at least 6.5, 7.0, 8.0, 9.0 or greater. For example, subjects with three markers such as IgA anti-I2 antibodies, NOD2 variation, and ASCA showed the greatest risk of the fibrostenotic subtype of Crohn's disease (82%, odds Ratio = 9.7, p< 0.000001) compared with subjects with two markers (74%, odds Ratio = 6.0), one marker (48%, odds Ratio = 1.9), or none of these markers (33%, odds Ratio = reference group) (see Example I). Methods for determining an odds ratio are well known in the art (see, for example, Schlesselman et al., Case Control Studies: Design, Conduct and Analysis Oxford University Press, New York (1982)).

In one embodiment, a marker or markers is associated with a clinical subtype of Crohn's disease with a p value of equal to or less than 0.05. In other embodiments, a marker is associated with a clinical subtype of Crohn's disease with a p value of equal to or less than 0.001. As used herein, the term "p value" is synonymous with "probability value." As is well known in the art, the expected p value for the association between a random marker and a subtype is 1.00. A p value of less than about 0.05 indicates that the marker and a subtype do not appear together by chance but are influenced by positive factors. Generally, the statistical threshold for significance of linkage has been set at a level where false positives would occur once in twenty (p=0.05). In particular embodiments, a marker is associated with a clinical subtype of Crohn's disease, such as the fibrostenotic subtype with a p value of equal to or less than 0.05, 0.04, 0.03, 0.02, 0.01, 0.009, 0.008, 0.007, 0.006, 0.005, 0.004, 0.003, 0.002 or 0.001, or with a p value of less than 0.000001, 0.00001, 0.00095, 0.0009, 0.00085, 0.0008 or 0.0005. It is recognized that, in some cases, p values may need to be corrected, for example, to account for factors such as sample size (number of families), genetic heterogeneity, clinical heterogeneity, or analytical approach (parametric or nonparametric method).

In addition to IgA anti-I2 antibodies and a NOD2 variant, other combinations of markers can be diagnostic of a particular clinical subtype of Crohn's disease. For example, the invention provides an in vitro method of diagnosing a fibrostenotic subtype of Crohn's disease in a subject having Crohn's disease by determining the presence or absence of IgA anti-I2 antibodies in the subject and further determining the presence or absence of ASCA in the subject; where the combined presence of anti-I2 antibodies and ASCA in the subject indicates that the subject has the fibrostenotic subtype of Crohn's disease. In one embodiment, the combined presence of the IgA anti-I2 antibodies and the ASCA in the subject is associated with the fibrostenotic subtype of Crohn's disease with an odds ratio of at least 6. In another embodiment, the combined presence of IgA anti-I2 antibodies, a NOD2 variant, and ASCA in the subject-indicates that the subject has the fibrostenotic subtype of Crohn's disease. In a related embodiment, the combined presence of the IgA anti-I2 antibodies, a NOD2 variant, and the ASCA in the subject is associated with the fibrostenotic subtype of Crohn's disease with an odds ratio of at least 9.

The methods of the invention optionally include generating a report indicating the presence or absence in a subject of one or more markers associated with a clinical subtype of Crohn's disease as disclosed herein. The methods of the invention also optionally include generating a report indicating the presence or absence in a subject of a clinical subtype of Crohn's disease, for example, the fibrostenotic subtype, or the risk that a subject has of having or developing a particular subtype of Crohn's disease. A report can be in a variety of forms, including, but not limited to, paper reports, oral reports and electronic reports. For example, a report can be printed on paper, or a report can be an electronic report that is not printed but is transmitted over an electronic medium such as electronic mail or a computer diskette.

Also disclosed is a method of predicting a response to therapy in a subject having Crohn's disease by determining the presence or absence in the subject of one or more markers associated with a clinical subtype of Crohn's disease, diagnosing the subject in which the one or more markers are present as having a particular subtype of Crohn's disease, and predicting a response to a therapy based on the diagnosis. Also disclosed is a method of a method of optimizing therapy in a subject having Crohn's disease by determining the presence or absence in the subject of one or more markers associated with a clinical subtype of Crohn's disease, diagnosing the subject in which the one or more markers are present as having a particular clinical subtype of Crohn's disease, and treating the subject having a particular clinical subtype of Crohn's disease based on the diagnosis. As an example, treatment for the fibrostenotic subtype of Crohn's disease currently includes surgical removal of the affected, strictured part of the bowel.

The following examples are intended to illustrate but not limit the present invention.

### EXAMPLE I

### ANTIBODIES AGAINST THE BACTERIAL SEQUENCE I2 ARE A MARKER OF THE FIBROSTENOTIC SUBTYPE OF CROHN'S DISEASE

This example shows that antibodies against the Crohn's disease-associated bacterial sequence I2 are an independent marker of the fibrostenotic subtype of Crohn's disease.

Clinical, serologic and genetic data were examined for 258 Crohn's disease patients under an Institutional Review Board (IRB) approved protocol. Briefly, a diagnosis of Crohn's disease in the patients was defined by the presence of a combination of established features from at least two of the following categories: 1) clinical - perforating or fistulizing disease, obstructive symptoms secondary to small bowel stenosis or stricture; 2) endoscopic - deep linear or serpiginous ulcerations, discrete ulcers in normal-appearing mucosa, cobblestoning, or discontinuous or asymmetric inflammation; 3) radiographic - segmental disease (skip lesions), small bowel or colon strictures, stenosis, or fistula, and; 4) histopathologic - submucosal or transmural inflammation, multiple granulomas, marked focal cryptitis or focal chronic inflammatory infiltration within and between biopsies, or skip lesions including rectal sparing in the absence of local therapy. Patients with primary sclerosing cholangitis and autoimmune hepatitis and those with chronically increased transaminase or alkaline phosphatase levels were excluded to avoid confusion with non-inflammatory bowel disease ANCA.

ELISAs were performed for IgA anti-I2 antibodies and anti-Saccharomyces cerevisiae antibodies (ASCA) as described in Examples II and III. Genotyping was performed for three Crohn's disease associated variants of the NOD2 gene, R702W, G908R, and 1007fs using the Taqman MGB system as described in Example IV.

Analysis of ELISA and genotyping data indicated that IgA antibodies to I2 were present in 56.5% of the Crohn's disease patients in the study. Patients expressing IgA anti-I2 antibodies were significantly more likely to have a fibrostenotic subtype of Crohn's disease than those not expressing IgA anti-I2 antibodies (71.4% vs. 43.3%, p<0.001) and significantly more likely to require small bowel surgery (66.7% vs. 37.1%, p< 0.001). In addition, IgA anti-I2 antibodies expression was negatively associated with ulcerative colitis-like Crohn's disease (20.6% vs. 41.24%, p<0.001). Quartile analyses revealed that higher levels of IgA anti-I2 antibodies were more strongly associated with the fibrostenotic subtype of Crohn's disease (p for the trend < 0.001), small bowel involvement (p= 0.023), and inversely associated with ulcerative colitis-like Crohn's disease (p= 0.005).

Conditional analysis performed on NOD2 variants and ASCA indicated that IgA anti-I2 antibodies were independently associated with the fibrostenotic subtype (p=0.001 and p=0.005, respectively). Similarly, IgA anti-I2 antibodies was independently associated with small bowel surgery when conditioned on NOD2 variation (p= 0.001) or ASCA (p=0.002).

Patients with all three markers, IgA anti-I2 antibodies, NOD2 variation, and ASCA showed the greatest risk of the fibrostenotic subtype of Crohn's disease (82%, Odds Ratio = 9.7, p< 0.000001), compared with patients with two (74%, Odds Ratio = 6.0), one (48%, Odds Ratio = 1.9), or none of these markers (33%, Odds Ratio = reference group).

### EXAMPLE II

### ELISA FOR IGA ANTI-I2 ANTIBODIES

This example shows demonstrates that the presence of IgA anti-I2 antibodies in patient sera can be determined using an ELISA microplate assay.

### A. GST-I2 fusion protein

The full-length 12 encoding nucleic acid sequence (SEQ ID NO: 1) was cloned into the GST expression vector pGEX. After expression in E. coli, the protein was purified on a GST column. A GST control protein was also expressed and purified. The purified protein was shown to be of the expected molecular weight by silver staining, and had anti-GST reactivity upon western analysis. The full-length I2 encoding nucleic acid sequence (SEQ ID NO:1) has also been cloned into a Hex-His6 expression vector, expressed in E. coli, and the resulting protein purified.

### B. ELISA analysis

Human IgA antibodies that bind the I2 polypeptide (SEQ ID NO: 2) were detected by direct ELISA assays essentially as follows. Plates (Greiner, USA Scientific, Ocala, FL) were coated overnight at 4°C with 100 µl/well GST control polypeptide or GST-I2 fusion polypeptide (5 µg/ml in borate buffered saline, pH8.5). After three washes in0.05% Tween 20 in phosphate buffered saline (PBS), the plates were blocked with 150 µl/well of 0.5% bovine serum albumin in PBS, pH7.4 (BSA-PBS) for 30 minutes at room temperature. The blocking solution was then replaced with100 µl/well of Crohn's disease or normal control serum, diluted 1:100. The plates were then incubated for 2 hours at room temperature and washed as before. Alkaline phosphatase conjugated goat anti-human IgA (α-chain specific), or IgG (γ chain specific) (Jackson ImmunoResearch, West Grove, PA) was added to the plates at a dilution of 1:1000 in BSA-PBS. The plates were incubated for 2 hours at room temperature before washing three times with 0.05% Tween 20/PBS followed by another three washes with Tris buffered normal saline, pH 7.5. Substrate solution (1.5 mg/ml disodium p-nitrophenol phosphate (Aresco; Solon, OH) in 2.5 mM MgCl2, 0.01 M Tris, pH 8.6) was added at 100µl/well, and color allowed to develop for one hour. The plates were then analyzed at 405 nm. Nonspecific binding of sera to the control GST protein (typically < 0.1) were subtracted from raw values of I2 binding to obtain I2-specific absorbances.

I2 positive reactivity was defined as reactivity greater than two standard deviations above the mean reactivity obtained with control (normal) sera analyzed at the same time as the test samples.

### EXAMPLE III

### ELISA FOR ANTI-SACCHAROMYCES CEREVISIAE ANTIBODIES (ASCA)

This example demonstrates that the presence of anti-Saccharomyces cerevisiae antibodies in patient sera can be determined using an ELISA microplate assay.

### A. Preparation of yeast cell wall mannan

Yeast cell wall mannan was prepared as follows and as described in Faille et al., Eur. J. Clin. Microbiol. Infect. Dis. 11:438-446 (1992) and in Kocourek and Ballou et al., J. Bacteriol. 100:1175-1181 (1969). A lyophilized pellet of yeast Saccharomyces uvarum was obtained from the American Type Culture Collection (#38926). Yeast were reconstituted in 10 ml 2X YT medium, prepared according to Sambrook et al., Molecular Cloning Cold Spring Harbor Laboratory Press (1989). S. uvarum were grown for two to three days at 30°C. The terminal S. uvarum culture was inoculated on a 2X YT agar plate and subsequently grown for two to three days at 30°C. A single colony was used to inoculate 500 ml 2X YT media, and grown for two to three days at 30°C. Fermentation media (pH 4.5) was prepared by adding 20 gm glucose, 2 gm bacto-yeast extract, 0.25 gm MgS04 and 2.0 ml 28% H3PO4 per liter distilled water. The 500 ml culture was used to inoculate 50 liters of fermentation media, and the culture fermented for three to four days at 37°C.

S. uvarum mannan extract was prepared by adding 50ml 0.02 M citrate buffer (5.88 gm/l sodium citrate; pH7.0+/-0.1) to each 100 grams of cell paste. The cell/citrate mixture was autoclaved at 125°C for ninety minutes and allowed to cool. After centrifuging at 5000 rpm for 10 minutes, the supernatant was removed and retained. The cells were then washed with 75 ml 0.02 M citrate buffer and the cell/citrate mixture again autoclaved at 125°C for ninety minutes. The cell/citrate mixture was centrifuged at 5000 rpm for 10 minutes, and the supernatant retained.

In order to precipitate copper/mannan complexes, an equal volume of Fehling's Solution was added to the combined supernatants while stirring. The complete Fehling's solution was prepared by mixing Fehling's Solution A with Fehling's SolutionB in a 1:1 ratio just prior to use. The copper complexes were allowed to settle, and the liquid decanted gently from the precipitate. The copper/mannan precipitate complexes were then dissolved in 6-8 ml 3N HCl per 100 grams yeast paste.

The resulting solution was poured with vigorous stirring into 100 ml of 8:1 methanol:acetic acid, and the precipitate allowed to settle for several hours. The supernatant was decanted and discarded; then the wash procedure was repeated until the supernatant was colorless, approximately two to three times. The precipitate was collected on a scintered glass funnel, washed with methanol and air dried overnight. On some occasions, the precipitate was collected by centrifugation at 5000 rpm for 10 minutes before washing with methanol and air drying overnight. The dried mannan powder was dissolved in distilled waster, using approximately 5 ml water per gram of dry mannan powder. The final concentration of S. uvarum cell wall mannan was approximately 30µg/ml.

### B. Preparation of S. uvarum mannan ELISA plates

S. uvarum cell mannan ELISA plates were saturated with antigen as follows. Purified S. uvarum mannan prepared as described above was diluted to a concentration of 100µg/ml with phosphate buffered saline/0.2% sodium azide (PBS-N3). Using a multi-channel pipettor, 100 µl of100µg/ml S. uvarum mannan was added per well of a Costar 96-well hi-binding plate (catalogue number 3590; Costar Corp., Cambridge, MA). The antigen was allowed to coat the plate at 4° C for a minimum of 12 hours. Each lot of plates was compared to a previous lot before use. Plates were stored at 2-8° C for up to one month.

### C. Analysis of patient sera

Patient sera were analyzed in duplicate for anti-IgG or anti-IgA reactivity. Microtiter plates saturated with antigen as described above were incubated with phosphate buffered saline/0.05% Tween-20 for 45 minutes at room temperature to inhibit nonspecific antibody binding. Patient sera were subsequently added at a dilution of 1:80 and incubated for 1 hour at room temperature. Wells were washed three times with PBS/0.05% Tween-20. Then a 1:1000 dilution of alkaline phosphatase-conjugated goat anti-human F(ab') fragment-specific IgG (Pierce, Rockford, IL) or alpha chain-specific IgA (Jackson Immunoresearch Labs,Inc., West Grove, PA) was added, and the microtiter plates incubated for 1 hour at room temperature. After washing, a solution of p-nitrophenol phosphate in diethanolamine substrate buffer was added, and color development allowed to proceed for 10 minutes. Absorbance at 405 nm with a reference wavelength of 650 nm was analyzed using an automated EMAX plate reader (Molecular Devices, Menlo Park, CA).

Standard binding of pooled sera from patients with an established diagnosis of Crohn's disease was used as a standard reference for binding and set to be 100 ELISA units. Results with test patient sera were expressed as a percentage of the standard binding of the reference Crohn's disease sera. Sera showing ASCA reactivity (IgG, IgA, or both) exceeding the reference range were termed ASCA positive.

### EXAMPLE IV

### GENOTYPING FOR THREE CROHN'S DISEASE ASSOCIATED VARIANTS OF NOD2

This example shows a genotyping assay that can be used to detect the presence or absence of a NOD2 variant.

Genotyping was performed using a genotyping assay employing 5'-exonuclease technology, the TaqMan MGB^{™} assay (PE Biosystems; Foster City, CA). Primers were designed using the software PrimerExpress 1.5^{™} (PE Biosystems) and sequence information found in dbSNP for NOD2 variants R702W, G908R, and 1007fs. The MGB^{™} design adds a "minor groove binder" to the 3' end of the TaqMan^{™} probes, thereby increasing the binding temperature of the probe and enabling the use of shorter probes than in conventional TaqMan^{™} assays (Kutyavin et al., Nucleic Acids Res. 25:3718-3723 (1997)). This has the effect of increasing the discrimination between the alleles in the assay (Kutyavin et al., Nucleic Acids Res. 28:655-661 (2000)). Assays were performed following the manufacturer's recommendations (PE Biosystems bulletin 4317594) in an ABI 7900 instrument. Genotyping was performed blinded to clinical status of the subjects. Primers and probes used in the genotyping assay are shown in Tables 1 and 2.

**Table 1**

| **Primers Used in Taqman MGB^{™} Assay for NOD2 Variants** | | | |
|---|---|---|---|
| **SNP Primer** | **Forward Primer** | **Reverse Primer** | **SEQ ID NO.** |
| R702W | | | for 11 |
| | | | rev 12 |
| G90R | | | for 13 |
| | | | rev 14 |
| 1007fs | | | for 15 |
| | | | rev 16 |

**Table 2**

| **TAQMAN PROBES** | | |
|---|---|---|
| **Allele detected** | **Probe sequence** | **SEQ ID NO** |
| R702W wild type allele | 6FAM-TGCTCCGGCGCCA-MGBNFQ | 17 |
| R702W variant allele | TET-CTGCTCTGGCGCCA-MGBNFQ | 18 |
| G908R wild type allele | 6FAM-CTCTGTTGCCCCAGAA-MGBNFQ | 19 |
| G908R variant allele | TET-CTCTGTTGCGCCAGA-MGBNFQ | 20 |
| 1007fs wild type allele | TET-CTTTCAAGGGCCTGC-MGBNFQ | 21 |
| 1007fs wild type allele ("2") | 6FAM-CCTTTCAAGGGGCCT-MGBNFQ | 22 |

This example describes an ELISA for direct detection of IgA anti-OmpC antibodies in patient sera.

### A. ELISA

The OmpC direct ELISA is performed as follows. Plates (Greiner, USA Scientific, Ocala, FL) are coated overnight at 4°C with 100 µl/well OmpC prepared as described below at 0.25 µg/ml in borate buffered saline, pH8.5. After three washes in0.05% Tween 20 in phosphate buffered saline (PBS), the plates are blocked with 150 µl/well of 0.5% bovine serum albumin in PBS, pH7.4 (BSA-PBS) for 30 minutes at room temperature. The blocking solution is then replaced with100 µl/well of Crohn's disease or normal control serum, diluted 1:100. The plates are then incubated for 2 hours at room temperature and washed as before. Alkaline phosphatase conjugated goat anti-human IgA (α-chain specific), or IgG (γ chain specific) (Jackson ImmunoResearch, West Grove, PA) is added to the plates at a dilution of 1:1000 in BSA-PBS. The plates are incubated for2 hours at room temperature before washing three times with 0.05% Tween 20/PBS followed by another three washes with Tris buffered normal saline, pH 7.5. Substrate solution (1.5 mg/ml disodium p-nitrophenol phosphate (Aresco; Solon, OH) in 2.5 mM MgCl2, 0.01 M Tris, pH 8.6) is added at 100µl/well, and color allowed to develop for one hour. The plates are then analyzed at 405 nm.

IgA OmpC positive reactivity is defined as reactivity greater than two standard deviations above the mean reactivity obtained with control (normal) sera analyzed at the same time as the test samples.

### B. Purification of OmpC

The protocol below describes purification of OmpC using spheroplast lysis.

OmpF_/_/OmpA_/_ mutant E. coli are inoculated from a glycerol stock into 10-20 ml of Luria Bertani broth supplemented with 100 µg/ml streptomycin (LB-Strep, Teknova, Half Moon Bay, CA), and cultured vigorously at 37°C for about 8 hours to log phase, followed by expansion to 1 liter in LB-Strep over 15 hours at 25°C.

The cells are harvested by centrifugation (JS-4.2,4K/15min/4°C). If necessary, cells are washed twice with 100 ml of ice cold 20 mM Tris-Cl pH 7.5. The cells are subsequently resuspended in ice cold spheroplast forming buffer (20 mM Tris-Cl pH 7.5, 20% sucrose, 0.1 M EDTA pH 8.0, 1 mg/ml lysozyme), after which the resuspended cells are incubated on ice for about 1 hour with occasional mixing by inversion.

If required, the spheroplasts are centrifuged (JA-17, 5.5k/10min/4°C) and resuspended in a smaller volume of spheroplast forming buffer (SFB). The spheroplast pellet is optionally frozen prior to resuspension in order to improve lysis efficiency. Hypotonic buffer is avoided in order to avoid bursting the spheroplasts and releasing chromosomal DNA, which significantly decreases the efficiency of lysis.

The spheroplast preparation is diluted 14-fold into ice cold 10 mM Tris-Cl pH 7.5, 1 mg/ml DNase-I, and vortexed vigorously. The preparation is sonicated on ice4x 30 seconds at 50% power at setting 4, with a pulse "On time" of 1 second, without foaming or overheating the sample.

Cell debris is pelleted by centrifugation (JA-17,5-10K/10min/4°C), and the supernatant removed and clarified by centrifugation a second time (10K/10 min/4°C). The supernatant is removed without collecting any part of the pellet, and placed into ultra centrifuge tubes. The tubes are filled to 1.5 millimeter from top with 20 mM Tris-Cl pH7.5.

The membrane preparation is pelleted by ultra centrifugation at 100,000 g (35K/1 hour/4°C in Beckman SW 60 swing bucket rotor). The pellet is resuspended by homogenizing into 20 mM Tris-Cl pH 7.5 using a 1 ml blue pipette tip and squirting the pellet closely before pipetting up and down for approximately 10 minutes per tube.

In a 15 ml screw cap tube filled with 4 mls, the material is extracted for 1 hour in 20 mM Tris-Cl pH 7.5 with 1% SDS, with rotation at 37°C. The preparation is transferred to ultra centrifugation tubes, and the membrane pelleted at 100,000g (35K/1 hour/4°C in Beckman SW 60). The pellet is resuspended by homogenizing into 20 mM Tris-Cl pH7.5 as before. The membrane preparation is optionally left at 4°C overnight.

OmpC is extracted for 1 hour with rotation at37°C in 20 mM Tris-Cl pH 7.5, 3%SDS, and 0.5 M NaCl (SDS will precipitate if kept below 37°C). The material is transferred to ultra centrifugation tubes, and the membrane pelleted by centrifugation at 100,000g (35K/1 hour/30°C in Beckman SW 60). Lower temperatures are avoided since further cooling will result in extracted protein salting out of solution.

The supernatant containing extracted OmpC is then dialyzed against more than 10,000 volumes to eliminate high salt content. SDS is removed by detergent exchange against0.2% Triton. Triton is removed by further dialysis against 50 mM Tris-Cl.

Purified OmpC, which functions as a porin in its trimeric form, is characterized as follows when analyzed by SDS-PAGE. Electrophoresis at room temperature results in a ladder of about 100 kDa, about 70 kDa, and about 30 kDa bands. Heating for10-15 minutes at 65-70°C partially dissociates the complex and results in only dimers and monomers (about 70 kDa and about 30 kDa bands). Boiling for5 minutes results in monomers of 38 kDa.

### EXAMPLE VI

### ELISA AND INDIRECT IMMUNOFLUORESCENCE FOR DETERMINING PANCA STATUS

This example describes methods for determining the pANCA status of a subject.

### A. Presence of pANCA is determined by fixed neutrophil ELISA

A fixed neutrophil enzyme-linked immunosorbent assay is used to detect pANCA as described in Saxon et al., J. Allergy Clin. Immunol. 86:202-210 (1990), and all samples are analyzed in a blinded fashion. Microtiter plates are coated with 2.5 x 105 neutrophils per well and treated with100% methanol to fix the cells. Cells are incubated with 0.25% bovine serum albumin (BSA) in phosphate-buffered saline to block nonspecific antibody binding. Next, control and coded sera are added at a 1:100 dilution to the bovine serum/phosphate-buffered saline blocking buffer. Alkaline phosphatase conjugated goat F(ab')2 anti-human immunoglobulin G (γ-chain specific) antibody (Jackson Immunoresearch Labs, Inc., West Grove, PA) is added at al:1000 dilution to label neutrophil bound antibody. A p-nitrophenol phosphate substrate solution is added and color development is allowed to proceed until absorbance at 405 nm in the positive control wells is 0.8-1.0 optical density units greater than the absorbance in blank wells.

Levels are determined relative to a standard consisting of pooled sera obtained from well-characterized pANCA positive ulcerative colitis patients. Results are expressed as ELISA units. Sera with circulating antineutrophil cytoplasmic IgG antibody exceeding the reference range value are termed ANCA positive. Numerical values that are below the reference range are termed ANCA negative.

### B. Indirect immunofluorescence assay for determination of ANCA staining pattern

Indirect immunofluorescent staining is performed on samples that are ANCA-positive by ELISA to determine whether the predominant staining pattern is perinuclear (pANCA) or cytoplasmic (cANCA). Glass slides containing approximately 100,000 neutrophils per slide are prepared by cytocentrifugation (Shandon Cytospin, Cheshire, England) and they are fixed in 100% methanol, air-dried, and stored at-20°C. The fixed neutrophils are incubated with human sera are diluted (1:20), and the reaction is visualized with fluorescein-labeled F(ab')2 γ chain-specific antibody as described in Saxon et al., *supra,* 1990. The slides are examined using an epifluorescence-equipped Olympus BH-2 microscope (Olympus, Lake Success, NY).

pANCA positivity is defined as a perinuclear staining pattern combined with ELISA reactivity greater than two standard deviations above the mean reactivity obtained with control (normal) sera analyzed at the same time as the test samples.

### EXAMPLE VII

### Association of Antibody Responses to Microbial Antigens and Complications of Small Bowel Crohn's Disease

This example demonstrates that both the number of antibody responses toward microbial antigens, and the magnitude of the total response, is highly associated with more complicated small bowel Crohn's disease.

### A. Patient Population and Methods

A patient study population of 303 patients was ascertained from patients assessed at Cedars-Sinai Medical Center between 1993 and 2002. All research related activities were approved by the Cedars-Sinai Medical Center Institutional Review Board, and a diagnosis of Crohn's disease was based on standard endoscopic, histologic, and radiographic features as described in Example I. In addition to the Crohn's disease patients reported previously in Vasiliauskas et al., Gastroenterology 123:689-699 (2002), and Abreu et al., Gastroenterology 123:679-688 (2002), the cohort of 303 study patients also included individuals enrolled from the clinic or at the time of surgery.

Crohn's disease phenotype designations were assigned based on standard previously published criteria (Vasiliauskas et al., *supra,* 2002; Vasiliauskas et al., Gastroenterology 110:1810-1819 (1996), and Abreu et al., *supra,* 2002). The phenotypes included fibrostenosing, internal perforating, perianal fistulizing, and ulcerative colitis-like phenotypes. Patients considered to have fibrostenotic disease had evidence of persistent small bowel obstruction or history of resection for small bowel obstruction secondary to Crohn's disease-related bowel stenosis and, furthermore, were required to have non-inflammatory stenosis with evidence of partial or complete small bowel obstruction not due to adhesions on radiographic examination. Patients with a history of or evidence of small bowel perforation (abscesses) or fistula (entero-entero, entero-cutaneous, or entero-vesicular) were assigned the phenotype of internal perforating disease. Perianal perforating disease was defined as a history of perianal abscess/fistula or recto-vaginal fistula. A single patient was in some cases assigned more than one phenotype designation. Disease location was based on endoscopic, histopathologic, and radiographic evidence of chronic inflammation, and defined as presence of inflammation in the small bowel, colon, or both. Patients characterized as having small bowel disease included those with only small bowel disease and those with both small bowel and colonic disease. Significant small bowel surgeries included small bowel resections, ileocolonic resections, and stricturoplasties.

Phenotype and disease location were assigned following discussion of the clinical data by multiple IBD physicians who were blinded to the results of serologic and genetic information. Phenotype designations were generally performed at the time of consent for serologic and genetic analysis, with most patients enrolled during first consultation in the IBD clinic and some additional patients enrolled at the time of surgery. The database was constantly updated, with one hundred and four patients having updated clinical phenotype designations by the time of data analysis. Surgery typically occurred prior to enrollment or at the time of enrollment, and updates were made in the database if surgery occurred following enrollment. Of the patients in the study cohort, twenty-six had serologic assessment at the time of surgery and at least once six months or more following surgery.

Genetic and serological analyses were performed as follows. Three NOD2/CARD15 single nucleotide polymorphisms were analyzed as described in Example IV above. All blood samples for serologic analysis were taken at the time of consent and enrollment. Sera were analyzed for expression of anti-I2, ASCA and anti-OmpC antibodies and pANCA as described above. Analysis of IgG and IgA ASCA and pANCA was performed at Cedars-Sinai Medical Center or Prometheus Laboratories using the same technology while all assays for anti-I2 and anti-OmpC antibodies were performed at Cedars-Sinai *Medical Center.* Antibody levels were determined and results expressed as ELISA units (EU/ml), which are relative to a Cedars-Sinai Laboratory (IgA-I2 and IgA-OmpC) or a Prometheus Laboratory Standard (IgA and IgG ASCA, and ANCA) derived from a pool of patient sera with well-characterized disease found to have reactivity to the particular antigen.

Statistical analyses were performed as follows. To determine associations between antibody responses toward microbial antigens, autoantigens, and NOD2 genotype status and disease phenotype characteristics, univariate analyses utilizing χ² tests were performed using Statistical Analysis Software (Version 8.02; SAS Institute, Inc.; Cary, NC). Odds ratios and 95% confidence intervals were calculated to compare the odds of positive serum reactivity towards the microbial antigens (I2, OmpC, and ASCA) in the group of patients with a certain disease characteristic (for example, the fibrostenotic subtype) with the group of patients lacking this disease characteristic.

To evaluate the association between disease phenotype and the combined level of immune response towards I2, oligomannan and OmpC, sums of quartile scores for anti-I2, ASCA and anti-OmpC were calculated. For each antigen, patients whose antibody levels were in the 1^{st}, 2^{nd}, 3^{rd} and 4^{th} quartile of the distribution were assigned a quartile score of 1, 2, 3 and 4, respectively. By adding individual quartile scores for each microbial antigen, a quartile sum score (ranging from 3-12) represented the cumulative quantitative immune response towards all three antigens for each patient.

The Cochran-Armitage test for trend was utilized to test for a linear relationship between the proportion of patients with a disease phenotype characteristic and the level of antibody response quantified by quartiles. A p-value (p trend) less than or equal to 0.05 indicated that the linear trend was statistically significant. Multivariate analysis with logistic regression modeling was also performed to determine primary associations among qualitative serological and genetic indicators and disease phenotypes. Analysis of variance using the F-test was performed on the 26 patients for whom sequential antibody data were available in order to test for antibody level stability.

### B. Clinical, serologic and genetic characteristics of the study population

The 303 patient cohort described above had similar characteristics as compared to previously reported frequencies of disease phenotypes, individual antibody responses to microbial and autoantigens, and NOD2 gene variations. Crohn's disease patients had previously been reported to have serum reactivity towards I2 (54%; Landers et al., Gastroenterology 123:689-699 (2002)); against oligomannans (ASCA) (40-60%; Vasiliauskas et al., *supra,* 2000; Annese et al., Gastroenterology 96:2407-2412 (2001), and Quinton et al., Gut 42:788-791 (1998)); and towards OmpC (56%; Landers et al., *surpa,* 2002); and to have pANCA reactivity (10-40%; Vasiliauskas, *supra*, 1996). Figure 4 shows scatter graphs of the serologic responses for each antigen in the 303 patient cohort.

Clinical characteristics as well as the serologic profile and NOD2 genotypes of the 303 patient cohort are summarized in Table 3. As shown in the table, there was a high proportion of patients with fibrostenosis (54.8%) and the need for small bowel surgery (52.2%), reflecting the severity of illness of patients referred to the IBD Center. Anti-I2 was seen in 59.4% of patients, and anti-OmpC was seen in 46.2%. Furthermore, approximately thirty-seven percent of patients were heterozygotes, compound heterozygotes, or homozygotes for the R675W, G881R, and 3020insC NOD2 mutations.

**Table 3**

| **Clinical Characteristics of the Crohn's Disease Cohort** | |
|---|---|
| **Clinical Characteristics** | **Cohort (n=303)** |
| Sex (M/F) | 160/143 |
| | |
| Median Age of Onset (yr) | 23.0 |
| | |
| Disease Location (%) | |
| Small bowel only | 19.8 |
| Colon only | 20.5 |
| Small bowel and colon | 59.7 |
| | |
| Disease Behavior (%) | |
| Perianal perforating | 37.3 |
| Internal perforating | 39.6 |
| Fibrostenosing disease | 54.8 |
| UC-like | 25.4 |
| Small bowel surgery | |
| | |
| Serologic Profile (%) | |
| pANCA positive | 17.2 |
| ASCA positive | 52.5 |
| Anti-I2 positive | 59.4 |
| Anti-OmpC positive | 46.2 |
| | |
| NOD2 Genotype for SNP 8, 12, & 13 (%) | 62.7 |
| No mutations | 29.7 |
| Heterozygotes | 7.6 |
| Compound Heterozygotes or Homozygotes | |

### C. Anti-I2, ASCA, anti-OmpC, and pANCA are associated with distinct disease phenotypes

Associations between Crohn's disease patient phenotype and the presence or absence of ASCA and pANCA have indicated that antibody responses can be associated with specific clinical characteristics. Furthermore, antibodies against I2 and OmpC previously have been shown to cluster together in a cohort of CD patients (Landers et al., *supra,* 2002). Table 4 shows the proportion of patients with each phenotype segregated by response to I2, oligomannans (ASCA), OmpC, presence of a NOD2 variant (one or two copies of R675W, G881R, or 3020insC), and pANCA reactivity. As shown in Table 4, anti-I2 reactivity was significantly associated with occurrence of small bowel disease, fibrostenosis, and small bowel surgery, while anti-OmpC was associated with fibrostenosis, internal perforating disease, and small bowel surgery. Reactivity against both of these antigens was negatively associated with ulcerative colitis-like disease. ASCA had the most significant associations with small bowel disease, fibrostenosis, internal perforations and small bowel surgery; ASCA was also negatively associated with UC-like disease, consistent with previous reports (Vasiliauskas et al., *supra,* 2000, and Louis et al., Gut 52:552-557 (2003)). Also consistent with earlier reports, pANCA was associated with ulcerative colitis-like disease, and negatively associated with small bowel disease, fibrostenosis, and small bowel surgery (Vasiliauskas et al., *supra,* 2000).

In sum, these results demonstrate that antibody responses towards I2 and OmpC are associated with complicated small bowel Crohn's disease phenotypes.

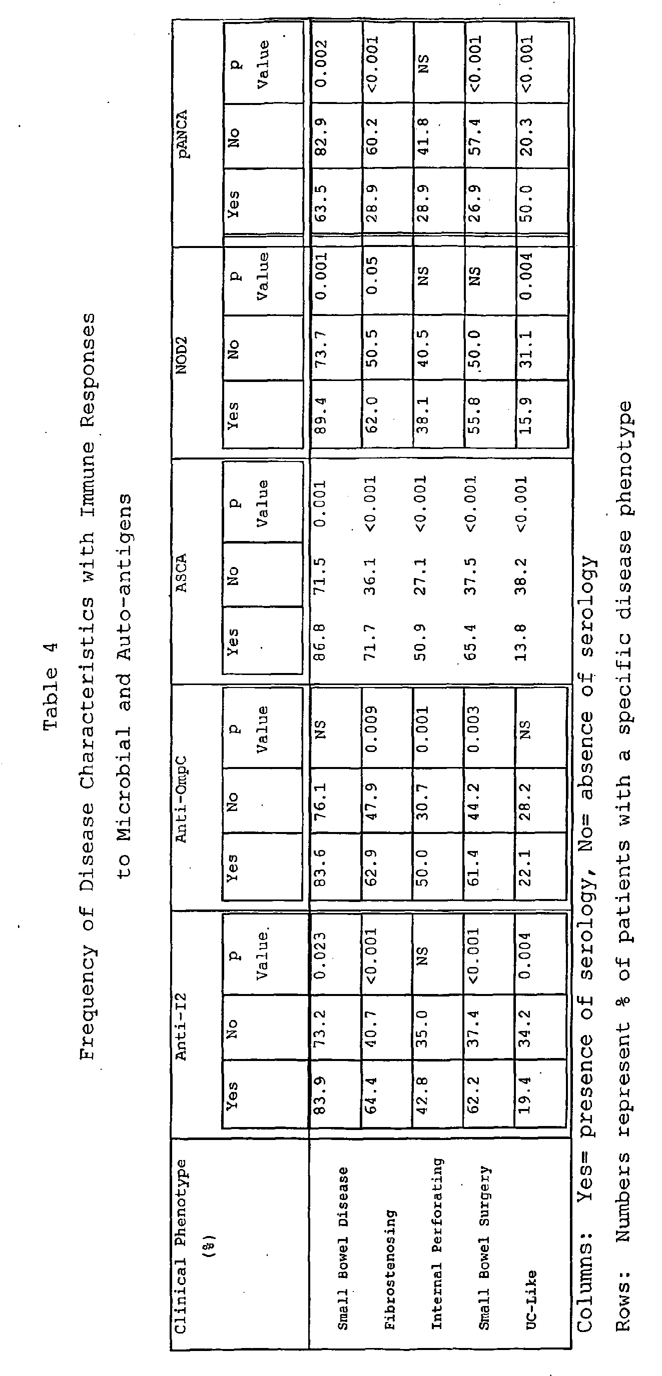

### D. Mutations in NOD2 are associated with fibrostenosing small bowel Crohn's Disease

As summarized in Table 4 above, NOD2 mutations in the cohort of 303 Crohn's disease patients were associated with small bowel disease (p = 0.001) and fibrostenosis (p = 0.05), and were negatively associated with ulcerative colitis-like disease (p = 0.004). NOD2 variants were not associated with small bowel surgery in this cohort (p = 0.332). These results support the association between NOD2 mutations and fibrostenotic small bowel Crohn's disease and are consistent with reports of associations between fibrostenotic disease behavior and the presence of NOD2 mutations in Crohn's (Abreu et al., *supra,* 2002; Helio et al., Gut 52:558-562 (2003); and Radlmayr et al., Gastroenterology 122:2091-2092 (2002)) or an association with small bowel disease only (Ahmad et al., Gastroenterology 122:854-866 (2002), and Elson, New Eng. J. of Med. 346:614-616 (2002)).

Combined with the data presented above, these results demonstrate that there can be a high frequency of Crohn's disease complications regardless of NOD2 genotype and indicate that immune responses towards microbial antigens can be closer to the pathophysiologic pathway of complicated small bowel disease course than genetic predisposition contributed by mutations in NOD2.

### E. Relative contribution of individual and multiple antibody responses against microbial antigens to complicated small bowel Crohn's disease

Multivariate logistic regression analysis was performed to determine which antibody responses were independently associated with disease characteristics. As summarized in Table 5, significant independent serum associations were observed between anti-I2 and fibrostenosis (p = 0.027) and small bowel surgery (p = 0.01); between anti-OmpC and internal perforating behavior (p < 0.006); and between ASCA and small bowel disease (p = 0.023), fibrostenosis (p < 0.001), internal perforating disease (p < 0.001), and small bowel surgery (p < 0.001), and negatively with ulcerative colitis-like disease (p = 0.001). In addition, pANCA was associated with ulcerative colitis-like disease (p < 0.001) and was negatively associated with small bowel disease (p = 0.013), fibrostenosis (p < 0.002), and small bowel surgery (p = 0.001). None of the serologic responses was associated with perianal perforating disease. The genetic marker, NOD2, was independently associated with the occurrence of small bowel disease (p = 0.003) and negatively associated with ulcerative colitis-like disease (p < 0.008). NOD2 was therefore not independently associated with any complicated small bowel disease phenotype, indicating that Crohn's disease phenotypes are more closely associated with immune responses towards microbial antigens than NOD2 genotype.

**Table 5**

| **Association of Clinical Features with Marker Antibodies: Result of Multivariate Logistic Regression** | | | | | |
|---|---|---|---|---|---|
| **Marker** | **Small Bowel Disease** | **Fibrostenosis** | **Internal Perf.** | **Small Bowel Surgery** | **UC-like** |
| Anti-I2 | NS | p=0.027 | NS | p=0.01 | NS |
| Anti-OmpC | NS | NS | p<0.006 | NS | NS |
| ASCA | p=0.023 | p<0.001 | p<0.001 | p<0.001 | p<0.001* |
| pANCA | p=0.013* | p<0.002* | NS | p=0.001* | p<0.001 |
| NOD2 | p=0.003 | NS | NS | NS | p<0.008* |

| | | | | | |
|---|---|---|---|---|---|
| p values represent significant independent associations *negative association | | | | | |

Many patients have immune reactivity towards more than one of the described microbial antigens, as summarized in the Venn diagram shown in Figure 5. Antibody responses in a given patient towards an increasing number of microbial antigens were analyzed for an increased likelihood of complicated small bowel disease phenotypes such as fibrostenosis or internal perforating disease. The relationship between serum reactivity towards one, two, or all three of the antigens (I2, oligomannan and OmpC) and clinical phenotype irrespective of pANCA and NOD2 status is shown in Table 6 below. Patients with all three associated markers were found to be more likely to have fibrostenotic disease, internal perforating disease and small bowel surgery, compared to patients having serum reactivity with none, one or even two of these markers (p for all ≤ 0.001). These results indicate that patients who have antibody responses towards a greater number of the microbial antigens I2, OmpC and oligomannan are at increased risk for fibrostenosis, internal perforating disease, and the need for small bowel surgery as compared with patients with no serologic response towards these microbial antigens or with a serologic response towards a smaller number of antigens.

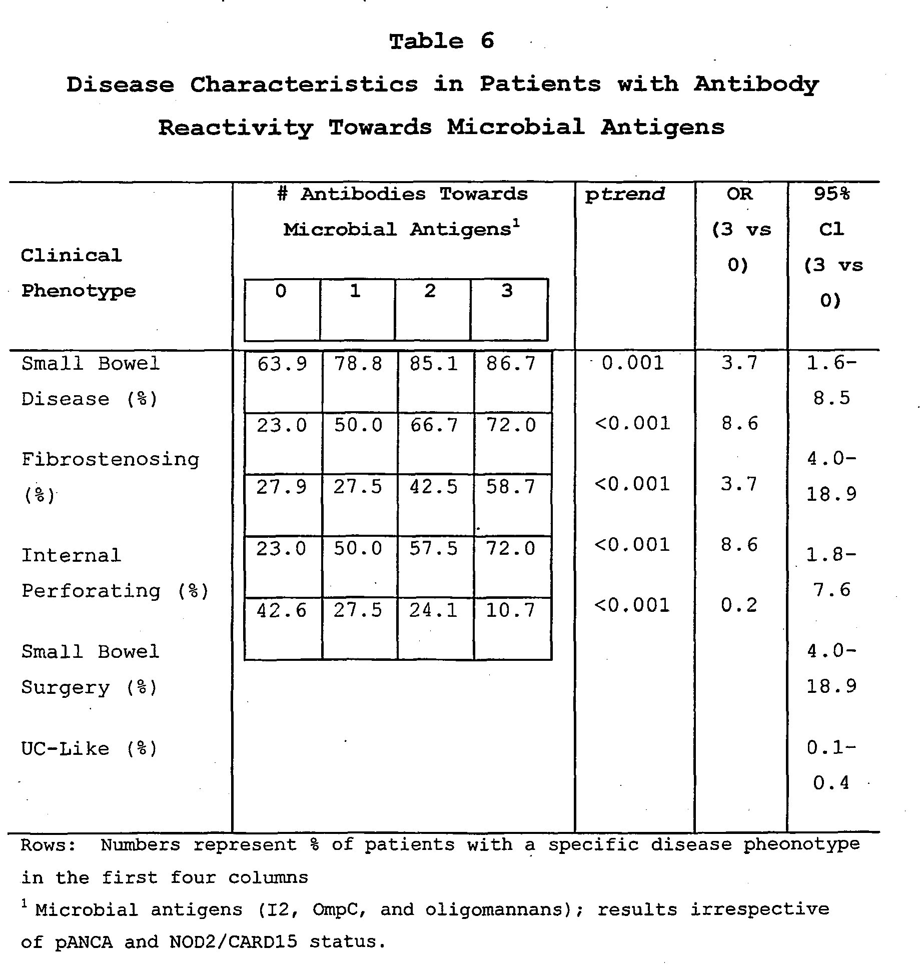

### F. Higher levels of antibody response toward individual and multiple microbial antigens is associated with higher frequency of complicated small bowel disease phenotype

The association between qualitative antibody responses towards microbial antigens and disease phenotypes has been described above. To assess the importance of quantitative antibody response, the association between the level of antibody response divided by quartiles towards I2, oligomannan, and OmpC, and the frequency of various Crohn's disease clinical subtypes was analyzed. Table 7A shows the results of quartile analysis for anti-I2, ASCA and anti-OmpC for each disease characteristic. As shown in Table 7A, there is an increasing percentage of patients with small bowel disease, fibrostenotic disease, internal perforating disease, small bowel surgery, and a decreasing likelihood of UC-like disease, as the magnitude of the antibody response toward a microbial antigen increases. Furthermore, the increased frequency of complications of small bowel disease associated with increasing levels of antibody responses was not solely related to the increase in frequency of small bowel disease. As shown in Table 7B, the frequency of small bowel surgery when only patients with small bowel disease were analyzed also increased. As an example, in the anti-I2 quartile analysis, the frequency of patients with small bowel disease requiring surgery was 42.3% in the lowest quartile while this rate rose to 73.4% in the highest quartile (p < 0.001). In sum, these results demonstrate that the presence and increasing level of an antibody response towards I2, OmpC, or oligomannan are associated with increasing frequency of complicated small bowel CD phenotypes.

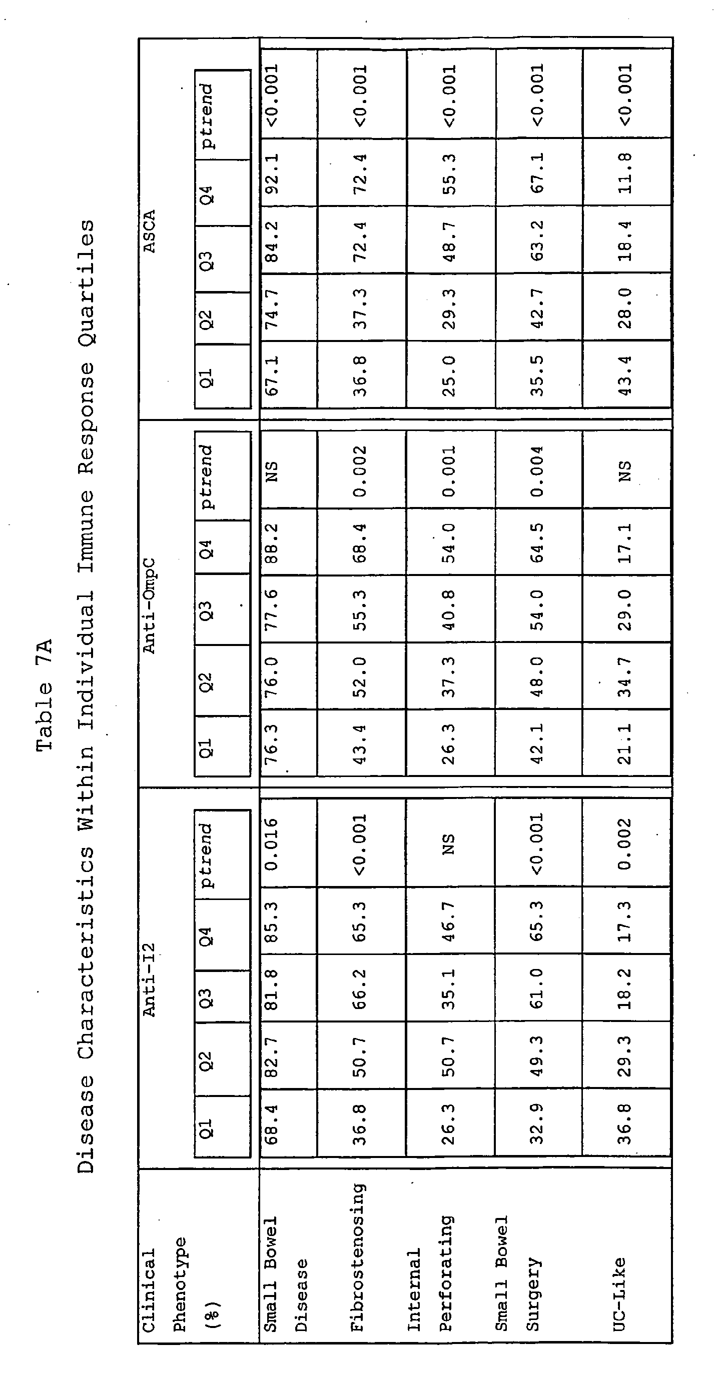

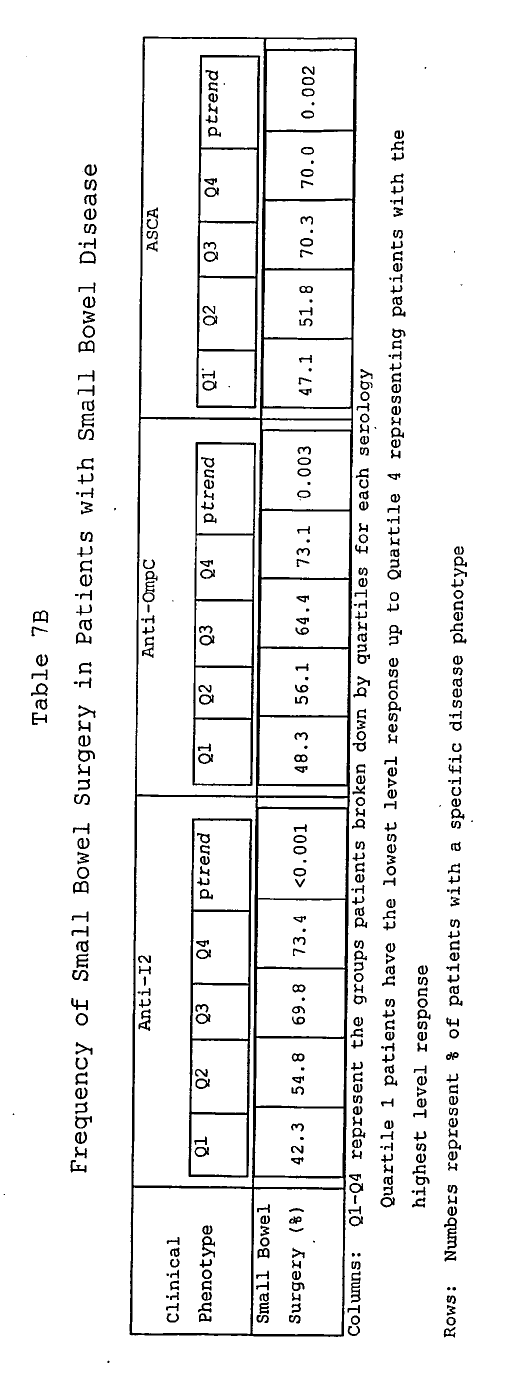

The level of the immune responses was analyzed over time in order to determine the influence of surgery on these antibody responses. In particular, levels of antibody responses towards microbial and autoantigens were analyzed in 26 patients following surgery. As shown in Figure 6, antibody responses towards microbial antigens remain stable for up to 20 months following surgery. In a separate statistically significant analysis, variation among a given patient's antibody levels over time was less than the variation seen among antibody levels from different individuals in the population for all tested serologies: anti-I2 (p < 0.001), anti-OmpC (p = 0.002), IgA ASCA (p < 0.001), IgG ASCA (p <.0.001), and ANCA (p = 0.015). These statistically significant results demonstrate that antibody level variation among patients is greater than within-patient variation, indicating that antibody levels in an individual are relatively stable. These results further indicate that immune reactions, rather than disease duration, are a major factor in development of complicated Crohn's disease phenotype.

The total level of antibody response towards all three microbial antigens was analyzed for any association with disease phenotype using quartile sums, a methodology for summarizing the level of antibody response towards multiple microbial antigens in a given patient population (Landers et al., *supra,* 2002). In particular, quartile sum analysis (sum of quartile scores for anti-I2, ASCA and anti-OmpC) was performed to evaluate a possible association between the level of combined immune response towards I2, oligomannan and OmpC, and disease characteristics for an individual patient. Figure 7 shows individual serologic responses broken down by quartiles and assigned scores of 1 to 4 based on their designated quartile. Individual quartile scores for each microbial antigen were added to obtain a quartile sum score ranging from 3 to 12; this sum score represents the cumulative quantitative immune response towards the three microbial antigens. The right panel of Figure 7 indicates the number of patients within each individual cumulative quartile sum score.

As shown in Figure 8, patients with increasing quartile sum scores tended to have an increasing likelihood of small bowel disease, fibrostenotic disease, and internal perforating disease, an increasing need for small bowel surgery, and a decreasing frequency of UC-like phenotype. Furthermore, when comparing the frequency of disease characteristics in the patients with quartile scores of 10-12 to the patients with the lowest three scores for response to all three antigens (quartile scores of 3-5), patients with quartile sum scores of 10-12 were observed to have the following associations: small bowel disease (OR 4.9, 95%CI=2.1-11.5, p < 0.001); fibrostenosis (OR 4.8, 95%CI=2.5-9.4, p < 0.001); internal perforations (OR 4.4, 95%CI=2.2-8.8, p < 0.001; small bowel surgery (OR 4.5, 95%CI=2.3-8.8, p < 0.001); and a decreased likelihood of UC-like disease (OR 0.2, 95%CI=0.1-0.5, p < 0.001).

Similar to the individual quartile analysis, the increasing frequency of complicated small bowel disease with rise in the quartile sum score was not solely due to an increase in the frequency of small bowel disease. Specifically, the frequency of surgery in patients with small bowel disease was 18.2% (11.8% divided by 64.7%) for a quartile sum score of 3, while this rate rose dramatically to 90% (77.3% divided by 86.3%) in patients with the highest response to all three antigens (quartile sum score of 12; p < 0.001). Furthermore, this association was higher than any of the trends demonstrated for individual antibody responses based on quartile analysis, which were usually around 72% (see Table 7, A and B). These results demonstrate that in this 303 patient cohort, the presence of multiple high-level antibody responses towards microbial antigens (I2, oligomannan and OmpC) is associated with a higher frequency of complicated small bowel disease, an association not solely related to an increase in the frequency of small bowel disease.

In sum, around 80% of Crohn's disease patients express a response towards at least one microbial antigen (I2, oligomannan or OmpC). However, high-level antibody reactivity towards a large number of these microbial antigens is more highly associated with complicated small bowel disease phenotypes, specifically fibrostenosis, internal perforating disease, and the need for small bowel surgery.

### EXAMPLE VIII

### PHENOTYPIC ASSOCIATIONS OF MARKER ANTIBODIES IN A NORTHERN EUROPEAN POPULATION

This example describes phenotypic associations of OmpC, I2, ASCA and ANCA markers in an independent Northern European population, and the relationship to phenotypic associations observed in a population of patients from the United States.

Important genetic and ethnic differences exist between populations in Northern Europe and the United States. In particular, Northern European populations appear to involve a lower contribution of NOD2/CARD15 variants (Bairead et al., Eur. J. Hum. Genet. 11:237-244 (2003); Thjodleifsson et al., Gastroenterology 124:1728-1737 (2003); Helio et al., Gut 52:558-562 (2003); Crichton et al., Gastroenterology 122: A298 (2003)) and a smaller proportion of subjects of Jewish origin. These genetic differences motivated a comparison of phenotypic associations seen with marker antibodies in two patient populations.

### A. Assays and characteristics of the study population

The Northern European population studied consisted of 142 consecutive Caucasian Scottish patients with well-characterized Crohn's disease. All patients attended the Western General Hospital in Edinburgh, Scotland, and gave informed consent to take part in the study as a component of the ongoing genetics program. The Lothian Research Ethics Committee approved the study protocol.

The patient population included 65 male and 77 female patients, with a median age of 39 years (inter quartile range (IQR) 31-54). A diagnosis of Crohn's disease was defined by the Lennard-Jones criteria, and the Vienna classification was used to categorize the clinical characteristics of Crohn's disease according to three overriding phenotypic characteristics: Age at diagnosis (A1<40 years, A2>40 years); location of disease (L1-terminal ileum, L2-colon, L3-ileocolon and L4-upper gastrointestinal); and disease behavior (Lennard-Jones, Scand. J. Gastroenterol. Suppl. 170:2-6 (1989); and Gasche et al., Inflamm. Bowel Dis. 6:8-15 (2000)). There were three mutually exclusive groups of disease behavior: Inflammatory (B1), stricturing (B2) and penetrating disease (B3) types.

Serological and genotyping analyses were performed as follows. All serum assays for expression of pANCA, ASCA, anti-OmpC and anti-I2 were performed in a blinded fashion at Cedars-Sinai Medical Center as described above. Antibody levels were determined, and the results expressed as ELISA units (EU/ml) relative to a control pool of patient sera with well characterized disease found to have reactivity to the particular antigen, as described above.

NOD2/CARD15 genotyping was performed at the Western General Hospital, Edinburgh. The three NOD2/CARD15 variants previously identified as being independently associated with Crohn's disease, R702W, G908R and 1007fs, were typed. The R702W polymorphism was typed using restriction fragment length polymorphism (RFLP) PCR and Taqman analysis while the G908R and R702W polymorphisms were typed by allele-specific PCR. Reported antibody prevalence has varied depending on the cohort studied and methodology used. In the cohort of 142 Scottish patients described above, OmpC was present at a frequency of 36.6% (52/142), I2 was present at a frequency of 52.1% (74/142), ASCA was present at a frequency of 39.4% (56/142), and pANCA was observed at a frequency of 14.1% (20/142). The clinical characteristics of the Scottish study population are shown in Table 8.

**Table 8**

| **The Clinical and Biological Characteristics of the Scottish Study Population** | | |
|---|---|---|
| Clinical Characteristic | N=142 | |
| Sex (M:F) | 64:78 | |
| Age of Diagnosis (median and IQR) (years) | 27 (21-35) | |
| Less than 40 years (A1) (n(%)) | 114 (80.3) | |
| Greater than 40 years (A2) (n(%)) | 28 (19.7) | |
| Disease Duration (median and IQR) (months) | 127.5 (56.5-229.5) | |

| Anatomical location (n(%)) | | |
|---|---|---|
| Small bowel (L1) | 46 (32.4) | |
| Colon (L2) | 57 (40.1) | |
| Ileocolon (L3) | 22 (15.5) | |
| Upper GI (L4) | 17 (12.0) | |

| Disease Behavior (n(%))* | At Diagnosis | At latest follow up |
|---|---|---|
| Inflammatory (B1) | 94 (71.2) | 46 (34.8) |
| Stricturing (B2) | 12 (9.1) | 20 (15.2) |
| Penetrating (B3) | 26 (19.7) | 66 (50.0) |

| Serological profile (n(%)) | | |
|---|---|---|
| pANCA positive | 20 (14.1) | |
| ASCA positive | 56 (39.4) | |
| OmpC positive | 52 (36.6) | |
| I2 positive | 74 (52.1) | |

| NOD2/CARD15 Genotype (n(%)) | | |
|---|---|---|
| Wild type | 108 (76.1) | |
| Simple heterozygote | 26 (18.3) | |
| Homozygote or compound heterozygote | 8 (5.6) | |

| | | |
|---|---|---|
| *Disease progression data was not available in 10 patients. | | |

### B. Relationship to Disease Behavior

Both the presence and magnitude of antibody responses to microbial antigens were assessed for relationship to disease behavior in the Scottish cohort. To quantify the presence of the studied antimicrobial antibodies, the number of positive antibodies was studied by assigning each individual a score of 0 to 3. To quantify the level of response across all antimicrobial antibodies, data were analyzed using quartile sums as described above (see, also, Landers et al., Gastroenterology 123:689-699 (2002)). Response to each antibody was broken down into quartiles and scored as 1 to 4, thus quantifying the level of response for an individual patient and one particular antigen, and scores for different antibodies were summed. The range of quartile sums for the Scottish cohort is displayed in Figure 9A.

All analyses were undertaken using the Minitab 14 statistical software package (Minitab Inc.; State College, PA). For univariate analysis, a χ² test, with Yate's correction if appropriate, was used to compare the frequency of positive and negative results for the different clinical and biological variables studied. Odds ratios (OR) and 95% confidence intervals (CI) were calculated to assess the odds of positive serum results in a group of patients with a certain clinical characteristic. For continuous variables, it was assumed that the data were not normally distributed, and the Mann Whitney-U test was used. For age at testing and disease duration, the cohort was divided into quartiles. To assess variability across the quartiles, the data were assumed to be positively skewed and thus analyzed by the Kruskal-Wallis test. Cluster analysis of quantitative antibodies in Crohn's disease patients was performed by using the k-means method to characterize two to ten patient clusters by minimizing within-cluster variance as described in Landers et al., *supra,* 2002. The pseudo-F statistic was used, and the cluster number corresponding to the largest value was determined to be the optimal number of clusters. Multivariate logistic regression analysis was used on all factors identified in univariate analysis to identify independent associations with seropositivity.

### C. Relationship of Antibody Responses to Age at Time of Serum Draw and Age at Diagnosis

To assess changes in the frequency and level of serum antibodies as a function of age at time of serum draw, the cohort was divided into four quartiles by age. Quartile 1 spanned ages of 16.8-23.2 years; quartile 2 spanned ages of 32.3-40.4 years; quartile 3 spanned ages of 40.5-55 years; and quartile 4 spanned ages of 55.1-88.6 years. Increasing age at time of serum draw was associated with a greater frequency of positive results for 12 (χ²=14.0, p=0.003) and OmpC (χ²=18.5, p<0.0001) but not for ASCA (p=0.660) or ANCA (p=0.939). In addition, there was evidence of increasing antibody levels with increasing age for the 12 (p=0.002) and OmpC (p<0.0001) antigens but again not for ASCA (p=0.107) or ANCA (p=0.941). Young age at onset of symptoms or age at diagnosis of Crohn's disease was not associated with the presence or absence of any of the antibody markers analyzed.

### D. Relationship of Presence and Magnitude of Antibody Response to Disease Duration

The association of positive results with age at serum draw indicated that disease duration may be associated with particular marker antibodies. The cohort was divided into four quartiles by disease duration: 2-72 months for quartile 1; 73-137 months for quartile 2; 138-242 months for quartile 3; and 243-663 months for quartile 4. As shown in Figure 10A, increasing disease duration was associated with an increasing frequency of ASCA (χ²=16.9, p=0.001), I2 (χ²=16.9, p=0.001) and OmpC (χ²=12.9, p=0.005) but not ANCA (p=0.496). When the presence of antibodies to all four microbial components was considered together, a strong association with increasing number of antibodies and disease duration was observed (p<0.001) as shown in Table 9. Increasing antibody levels were also associated with increasing disease duration quartiles for ASCA (p=0.001), I2 (p=0.005) and OmpC (p=0.003). This pattern was not seen for pANCA (Figure 10B). Increasing quartile scores were strongly associated with increasing disease duration (p trend<0.001). The median disease duration of patients with quartile sum scores of 3-5 was 100 months (IQR 28-137) as compared to 249 months (IQR 128-414) for patients with scores of 10-12 (p<0.001). Multiple logistic regression further indicated that the association with disease duration was stronger that that for time since serum draw (p<0.001). These results demonstrate a strong relationship between disease duration and the presence and magnitude of antibody responses to ASCA, I2 and OmpC.

**Table 9**

| **Clinical Features in Relation to Number of Responses to Microbial Antigens** | | | | | | | |
|---|---|---|---|---|---|---|---|
| | **Number of Positive Antibodies** | | | | | | |
| | **0** | **1** | **2** | **3** | **P value** | **OR** | **CI** |
| | **(n=41)** | **(n=47)** | **(n=27)** | **(n=27)** | | **(0 v 3)** | **(0 v 3)** |
| **Disease Duration** | 96 (36-141) | 139 (82-138) | 128 (50-228) | 301 (171-442) | <0.0001 | | |
| **Small bowel** | 18 (43.9) | 24 (51.1) | 15 (55.6) | 22 (81.5) | 0.019 | 5.6 | 1.8-17.8 |
| **Colon** | 21 (51.2) | 20 (42.6) | 11 (40.7) | 5 (18.5) | 0.058 | 0.2 | 0.07-0.7 |
| **Disease progression** | 8/33 (24.2) | 17/33 (51.5) | 11/15 (73.3) | 13/15 (86.7) | <0.0001 | 20.3 | 3.7-109.9 |
| **Perforating disease** | 12 (29.3) | 26 (55.3) | 13 (54.2) | 17 (70.8) | 0.008 | 5.9 | 1.9-17.8 |
| **Stricturing disease** | 4 (9.8) | 6 (12.8) | 6 (25) | 4 (16.7) | 0.385 | 1.8 | 0.4-8.2 |
| **Surgery** | 13 (31.7) | 27 (57.4) | 14 (51.9) | 24 (88.9) | <0.0001 | 17.2 | 4.4-67.7 |

| | | | | | | | |
|---|---|---|---|---|---|---|---|
| The clinical phenotype associated with increasing presence of ASCA, OmpC and I2 is shown. A more severe phenotype is shown by increasing disease progression, perforating disease, intestinal surgery and small bowel disease. Data for disease duration is quoted as median and IQR and analysed by Kruskal-Wallis test. For other variables the p value is generated by χ2 test on a 4x2 contingency table but the OR and CI are generated by a comparison of the group with 3 positive antibodies with those with 0 antibodies. Disease progression figures are an expression of individuals who initially had inflammatory disease (B1) at diagnosis. | | | | | | | |

### E. The Presence and Magnitude of Antibody Response is Related to Disease Location

The distribution of affected bowel differed according to antibody status for ASCA (χ²=11.7, p=0.009) and pANCA (χ²=8.6. p=0.036) but not for I2 (p=0.788) or OmpC (p=0.287). The presence of ASCA was associated with small bowel disease, and was present in 43/85 patients with small intestinal involvement as compared to 13/57 without involvement of the small intestine (χ²=9.89, p=0.0017, OR 3.5 (CI 1.6-7.3)). ANCA was associated with isolated colonic disease, and was observed in 14/57 patients with isolated colonic disease as compared to 6/85 patients without this type of disease (χ²=7.25, p=0.0071, OR 4.3 (CI 1.5-12.0)). As shown in Table 9, small bowel disease was also associated with an increasing number of antibodies to microbial antigens (p=0.019); a trend towards a negative association with colonic disease was also observed (p=0.058). ASCA levels were higher in patients with small bowel involvement (p=0.016), while pANCA levels were higher in patients with colonic disease (p=0.022). No variation in disease location was seen for I2. Small bowel disease was associated with increasing quartile sums, and colonic disease was inversely associated with higher quartile sums (p trend =0.028; Figure 9B). Small bowel disease was present in 15/30 patients with quartile scores of 3-5 as compared to 28/35 patients with scores of 10-12 (χ²=5.2, p=0.02, OR 2.1 (CI 1.3-11.3)).

### F. Antibody Responses were Related to the Need for Surgical Resection

Of 142 patients, 78 had undergone previous surgery, and there was a more frequent detection of ASCA (χ²=19.3, p<0.00001, OR 7.1 (CI 2.5-11.7)) and I2 (χ²=7.03, p=0.008, OR 3.2 (CI 1.3-5.1)) in this group. As shown in Table 10, OmpC reactivity (p=0.0839, OR 1.34) also approached significance, and pANCA showed an inverse association with the need for surgical resection (χ² =7.1, p=0.0078, OR 0.51 (CI 0.11-0.83)). There was a strong association between the number of positive antimicrobial antibodies and the need for surgery: 88.9% of patients who had responses to all three microbial antigens required surgery, as compared to 31.7% of those with no responses to these antigens (p<0.0001; Table 9).

ASCA (p<0.00001), I2 (p=0.0034) and OmpC (p=0.0252) levels were also greater in patients who had previously undergone surgery (Table 10). This was reflected by a strong association between quartile sum score and need for surgery (p= trend 0.001; Figure 9B). In particular, 7/30 patients with quartile sum scores of 3-5 required surgery, compared with 28/35 patients with quartile sum scores of 10-12 needing surgery (χ²=23.2, p<0.00001, OR 12.5 (CI 3.8-41.2)). Although surgery may be secondary to disease progression, multivariate analysis indicated that surgery and disease progression were independently related to antibody status.

**Table 10**

| **Antibody Levels in Patients With and Without Intestinal Resection** | | | |
|---|---|---|---|
| | **Surgery** | **No Surgery** | **P value** |
| | **(n=78)** | **(n=64)** | |
| ASCA | 26.25 (9.78-62.41) | 4.35 (1.78-12.68) | <0.00001 |
| I2 | 31.06 (15.93-65.96) | 13.13 (5.53-42.64) | 0.0034 |
| OmpC | 20.70 (10.44-33.30) | 12.96 (8.24-24.95) | 0.0252 |
| pANCA | 13.78 (9.89-19.62) | 16.00 (10.65-26.42) | 0.0972 |

| | | | |
|---|---|---|---|
| Data shown demonstrates differences in antibody levels in patients with and without previous intestinal resection. Antibodies to microbial components were all different between the groups whereas pANCA trended towards an inverse relationship. Data are given in ELISA units and expressed as median and interquartile ranges. Analysis is with the Mann-Whitney test. | | | |

### G. Reactivity to Microbial Antigens is Associated with the Progression of Disease Type

Inflammatory, stricturing and penetrating disease types are encompassed within the Vienna classification of CD (Gashe et al., *supra,* 2000), a system that allows the assessment of changes in disease behavior over time. Using alternative classifications, fibrostenosing disease has previously been associated with responses to microbial antigens (Vasiliauskas et al., Gut 47:487-496 (2001)). Of the individuals in the 142 patient cohort, 94 patients had inflammatory disease, 12 had stricturing disease and 26 had penetrating disease at the time of diagnosis. At latest follow up of the patients with inflammatory disease, 45 patients remained inflammatory, 16 progressed to stricturing disease, and 33 progressed to penetrating disease. Data were unavailable in 10 patients.

Progression of disease type was associated with the presence of ASCA (p<0.00001) and I2 (p=0.0390). OmpC did not achieve statistical significance (p=0.0810), and pANCA was inversely associated with disease progression (p=0.04; Table 11). When all antibodies were considered, there was a strong association with the number of positive antibodies and the progression of disease type. Disease progressed in 24.2% of patients with no antibody response compared to disease progression observed in 86.7% of patients with a response to all three antibodies (p<0.0001). An association with progression to perforating disease (p=0.008) but not stricturing disease (p=0.385) was also seen, as summarized in Table 9 above.

Antibody levels to ASCA (p<0.00001), I2 (p=0.0403) and OmpC (p=0.0007) but not pANCA (p=0.2177) were elevated in patients showing disease progression. Increasing quartile scores were strongly associated with disease progression (p trend=0.001; see Figure 9B). Disease progression was observed in 3/24 patients with quartile sum scores of 3-5, as compared to disease progression in 16/20 patients with quartile sum scores of 10-12 (χ² =23.1, p<0.00001, OR 28 (CI 5.5-51.8)). There was no association of the presence or magnitude of antibody response with family history of IBD, the presence of extraintestinal manifestations or the need for infliximab or azathioprine. In addition, there was no association of the studied parameters with NOD2/CARD15 status.

**Table 11**

| **Antibody Frequency and Level Relative to Disease Progression** | | | | | | |
|---|---|---|---|---|---|---|
| | **Antibody Frequency (%)** | | | **Antibody Level (median)** | | |
| | **NP** | **P** | **P value** | **NP** | **P** | **P value** |
| | **(n=43)** | **(n=49)** | | **(n=43)** | **(n=49)** | |
| ASCA | 9.3 | 51.0 | <0.00001 | 3.19 (1.32-9.90) | 20.28 (9.91-57.34) | <0.00001 |
| I2 | 41.9 | 61.2 | 0.0390 | 18.31 (5.57-38.03) | 29.86 (15.46-74.94) | 0.0403 |
| PANCA | 23.3 | 6.1 | 0.0400 | 16.11 (10.59-26.23) | 13.87 (10.28-19.76) | 0.2177 |
| OmpC | 13.9 | 26.5 | 0.0810 | 10.36 (7.19-17.71) | 21.31 (12.26-37.44) | 0.0007 |

| | | | | | | |
|---|---|---|---|---|---|---|
| Differing antibody frequency and level are displayed in relation to disease type that progressed and that that did not. It is clear that the antibodies to microbial components are associated with progression of disease type while pANCA is not. Antibody frequencies are displayed as percentages and analysed by χ2. Antibody levels are displayed as median and interquartile ranges and analysed by Mann-Whitney test. NP-No progression. P-Disease progression. | | | | | | |

These results demonstrate a strong relationship between disease progression from purely inflammatory disease to stricturing or penetrating disease and presence of ASCA and I2 antibodies. These results further demonstrate a strong relationship between disease progression and the magnitude of ASCA, I2 and OmpC responses.

### H. Cluster Analysis

Cluster analysis was performed in order to study phenotypic patterns by an alternative method. In particular, cluster analysis is a separate mathematical approach useful for evaluating patient response without bias to antibody distribution. This method represents a purity analysis by selecting high response groups. The cluster analysis evaluated quantitative levels of the following five antibodies (ASCA IgA, ASCA IgG, ANCA, I2 and OmpC) in the Scottish cohort. On the basis of the largest pseudo-F statistic, four clusters were shown to be optimal in this cohort. These were clusters characterized by low reactivity to all antibodies (Cluster 1), high IgG and IgA ASCA (Cluster 2), High OmpC and I2 (Cluster 3) and high pANCA (Cluster 4).

An analysis of the phenotypic associations of the cluster groups revealed major differences seen between those with and without responses to microbial antigens. Age at testing (p<0.0001), disease duration (p<0.0001), disease location (p=0.001), frequency of previous surgery (p=0.004), disease behavior (p=0.004), and family history of IBD (p=0.005) all varied across the clusters. The small size of cluster 4 makes firm conclusions difficult in this group.

Specifically, the presence of antibodies to microbial components in cluster 2 (high ASCA) and cluster 3 (high I2 and OmpC)) was associated with a higher age at testing (p=0.0006), longer disease duration (p=0.0021), small bowel disease (p=0.043) and disease progression (p=0.007). A family history of inflammatory bowel disease was seen more frequently in patients with low or absent antibody response (cluster 1; p=0.009). As summarized in Table 12, there was no association of cluster type with sex, extraintestinal manifestations, the need for Infliximab or azathioprine or NOD2/CARD15 status.

**Table 12**

| **Association of Cluster Analysis Stratification and Clinical Variables** | | | | | |
|---|---|---|---|---|---|
| | **Cluster 1** | **Cluster 2** | **Cluster 3** | **Cluster 4** | **P value** |
| | **(n=96)** | **(n=17)** | **(n=23)** | **(n=6)** | |
| **Age at test (Years)** | 37.4 29.7-60.0 | 43.5 36.1-55.1 | 57.1 37.5-70.8 | 58.7 41.0-81.0 | <0.0001 |
| **Disease duration (Months)** | 120.5 57.3-195.5 | 158.0 98.0-374.5 | 240.0 121.0-411.0 | 130.0 67.5-309.8 | <0.0001 |
| **Disease location (n (%) L2)** | 42 (43.7) | 3 (17.6) | 6 (26.1) | 6 (100) | 0.001 |
| **Previous surgery** | 49 (51.0) | 13 (76.5) | 16 (69.6) | 0 (0) | 0.004 |
| **B1 at diagnosis** | 67 (69.8) | 7 (41.2) | 14 (60.9) | 6 (100) | 0.034 |
| **Disease progress** | 31 (46.3) | 7 (100) | 11 (78.6) | - | 0.004 |
| **Family history** | 29 (30.2) | 0 (0) | 2 (8.7) | 0 (0) | 0.005 |
| **NOD2 positive** | 23 (24.0) | 6 (35.3) | 4 (17.4) | 1 (16.6) | 0.568 |

| | | | | | |
|---|---|---|---|---|---|
| Data are displayed as median and interquartile range (and analysed by Kruskal-Wallis test) or frequency of positive results (and analysed by χ²). Disease location is expressed as the number of patients who had isolated colonic disease (L2). B1 at diagnosis represents the proportion of patients who had inflammatory disease type at diagnosis and disease progress represents the proportion of these patients who progressed to a more severe disease type. | | | | | |

### I. Multivariate Analysis

Independent variables associated with individual antibodies are shown in Table 13 below. An analysis of the overall responses found that disease duration (p<0.001) and disease progression (p<0.001) were independently associated with both presence (0,1,2,3) and magnitude (quartile sum) of antibody response.

**Table 13**

| **Multivariate Analysis of Antibodies to Microbial Antigens** | | | | |
|---|---|---|---|---|
| | **Small bowel disease** | **Disease duration** | **Disease progression** | **Surgery** |
| **ASCA** | NS | NS | <0.0001 | NS |
| **I2** | NS | 0.002 | NS | 0.033 |
| **OmpC** | NS | 0.002 | 0.005 | NS |

| | | | | |
|---|---|---|---|---|
| The p values represent significant independent associations, NS no significant association. | | | | |

### J. Comparison with North American Patients

When data from the Scottish population were compared with data from a study population of U.S. patients (Landers et al., *supra*, 2002), results for ASCA IgG, ASCA IgA and OmpC prevalence were higher in the cohort of U.S. patients. Despite distinct differences in individual antibody levels and in clinical and genetic characteristics of the cohorts, considerable overlap of phenotypic associations was observed, as summarized in Table 14. An association of both presence and magnitude of antibody response was observed for small bowel disease, penetrating disease type, UC-like disease and the need for surgery. However, an association with fibrostenosing disease was not observed in the Scottish cohort. Disease duration and progression of disease type, both strong associations in the Scottish cohort, were not assessed in the cohort of U.S. patients.

In sum, the results obtained with the Scottish cohort corroborate the associations observed between presence and magnitude of antibody response and the occurrence of small bowel disease, penetrating disease, UC-like disease, and the need for surgery.

**Table 14**

| **Comparison of Response and Phenotype in Scottish and U.S. populations** | | | |
|---|---|---|---|
| | **United Kingdom** | **United States** | **P value** |
| | **(n=142)** | **(n=300)** | |
| **Individual Antibody Levels** | | | |
| ASCA IgA | 11.6 (-1.04-213.39) | 15.38 (0-164) | 0.0004 |
| ASCA IgG | 10.43 (0.19-77.21) | 27.2 (-3.78-323.38) | 0.0001 |
| ANCA | 14.39 (3.26-240.24) | 13.45 (2-255) | 0.0857 |
| OmpC | 16.93 (-0.18-129.26) | 19.75 (-0.3-192.93) | 0.0294 |
| I2 | 23.93 (0-269.43) | 22.1 (-1.56-260.15) | 0.5755 |

| **Antibody Presence (Analysis of 0,1,2,3 antibodies)** | | | |
|---|---|---|---|
| Small bowel | Yes (0.019) | Yes (0.001) | |
| Fibrostenosing | No (0.385) | Yes (<0.001) | |
| Penetrating | Yes (0.008) | Yes (<0.001) | |
| Surgery | Yes (<0.001 | Yes (<0.001) | |
| UC like | No (0.058) | Yes (<0.001) | |

| **Magnitude of Response (Analysis of Quartile sums)** | | | |
|---|---|---|---|
| Small bowel | Yes (0.028) | Yes (<0.001) | |
| Fibrostenosing | No (0.245) | Yes (<0.001) | |
| Penetrating | Yes (<0.001) | Yes (<0.001) | |
| Surgery | Yes (<0.001) | Yes (<0.001) | |
| UC like | Yes (0.028) | Yes (<0.001) | |

| | | | |
|---|---|---|---|
| Antibody levels are expressed as median ± IQR. Other data are expressed as presence of an association with p value from study. It should be noted that disease duration and disease progression were not assessed in the US cohort and no comparison can be made. | | | |

Although the invention has been described with reference to the disclosed embodiments, those skilled in the art will readily appreciate that the specific experiments detailed are only illustrative of the invention.

## Claims

1. An *in vitro* method of diagnosing a clinical subtype of Crohn's disease in a subject having Crohn's disease, said method comprising determining the presence or absence of IgA anti-I2 antibodies in a sample from the subject, wherein the presence of said IgA anti-I2 antibodies indicates that the subject has said subtype of Crohn's disease wherein said clinical subtype of Crohn's disease is a fibrostenotic subtype of Crohn's disease, a subtype of Crohn's disease **characterized by** the need for small bowel surgery, or a subtype of Crohn's disease **characterized by** the absence of features of ulcerative colitis.

2. The method of claim 1, further comprising determining the presence or absence in the sample of one or more fibrostenotic markers selected from a NOD2 variant or anti-Saccharomyces cerevisiae antibodies (ASCA), wherein said NOD2 variant is selected from R702W, G908R, and 1007fs, and wherein the presence of said IgA anti-I2 antibodies and the presence of one of said one or more fibrostenotic markers indicates a greater chance that the subject will have an aggressive form of the fibrostenotic subtype of Crohn's disease requiring small bowel surgery.

3. The method of claim 2, wherein said one or more fibrostenotic markers is a NOD2 variant.

4. The method of claim 2, wherein said one or more fibrostenotic markers is ASCA.

5. The method of claim 2, wherein said one or more fibrostenotic markers are a NOD2 variant and ASCA.

6. The method of claim 1, wherein determining the presence or absence of IgA anti-I2 antibodies in the subject comprises the steps of:
(a) contacting a sample from the subject with an I2 antigen, or immunoreactive fragment thereof, under conditions suitable to form a complex of I2 antigen, or immunoreactive fragment thereof, and antibody against said I2 antigen;
(b) contacting said complex with a labeled secondary antibody; and
(c) detecting the presence or absence of said complex,
wherein the presence of said complex indicates the presence of said IgA ant-I2 antibodies in the subject.

7. The method of claim 1, further comprising determining the presence or absence of a NOD2 variant in the sample, wherein the presence of IgA anti-I2 antibodies and the presence of a NOD2 variant in the subject indicates that the subject has said fibrostenotic subtype of Crohn's disease, wherein said NOD2 variant is selected from R702W, G908R, and 1007fs.

8. The method of claim 7, wherein the combined presence of said IgA anti-I2 antibodies and said NOD2 variant in the subject is associated with said fibrostenotic subtype of Crohn's disease with an odds ratio of at least 6.

9. The method of claim 1, further comprising determining the presence or absence of ASCA in the sample, wherein the presence of said IgA anti-I2 antibodies and the presence of said ASCA in the subject indicates that the subject has said fibrostenotic subtype of Crohn's disease.

10. The method of claim 9, wherein the combined presence of said IgA anti-I2 antibodies and said ASCA in the subject is associated with said fibrostenotic subtype of Crohn's disease with an odds ratio of at least 6.

11. The method of claim 7, further comprising determining the presence or absence of said ASCA in the sample, wherein the combined presence of IgA anti-I2 antibodies, said NOD2 variant, and said ASCA in the subject indicates that the subject has said fibrostenotic subtype of Crohn's disease.

12. The method of claim 11, wherein the combined presence of said IgA anti-I2 antibodies, said NOD2 variant, and said ASCA in the subject is associated with said fibrostenotic subtype of Crohn's disease with an odds ratio of at least 9.

13. An *in vitro* method of determining a risk of having or developing a clinical subtype of Crohn's disease **characterized by** fibrostenosis, internal perforating disease or the need for small bowel surgery in a subject having Crohn's disease, said method comprising determining the presence or absence of three markers in a sample from the subject, said three markers being IgA anti-I2 antibodies, anti-Saccharomyces cerevisiae antibodies (ASCA), and IgA anti-OmpC antibodies,
wherein the presence of said three markers indicates a first risk of having or developing said clinical subtype of Crohn's disease,
the presence of exactly two of said three markers indicates a second risk of having or developing said clinical subtype of Crohn's disease,
the presence of exactly one of said three markers indicates a third risk of having or developing said clinical subtype of Crohn's disease, and
the absence of said three markers indicates a fourth risk of having or developing said clinical subtype of Crohn's disease, and
wherein said first risk is greater than said second risk, said second risk is greater than said third risk, and said third risk is greater than said fourth risk.

14. An *in vitro* method of determining a risk of having or developing a clinical subtype of Crohn's disease in a subject having Crohn's disease, said clinical subtype **characterized by** fibrostenosis or the need for small bowel surgery, said method comprising determining the presence and magnitude of IgA anti-I2 antibody response in a sample from the subject, wherein a greater magnitude of IgA anti-12 antibody response indicates a greater risk of having or developing said clinical subtype **characterized by** fibrostenosis or the need for small bowel surgery.

15. An *in vitro* method of determining a risk of having or developing a clinical subtype of Crohn's disease **characterized by** fibrostenosis, internal perforating disease or the need for small bowel surgery in a subject having Crohn's disease, comprising determining the presence and magnitude of three markers in a sample from the subject, said three markers being IgA anti-I2 antibodies, anti-Saccharomyces cerevisiae antibodies (ASCA), and IgA anti-OmpC antibodies, wherein a greater magnitude of said three markers combined indicates a greater risk of having or developing said clinical subtype **characterized by** fibrostenosis, internal perforating disease or the need for small bowel surgery.

16. An *in vitro* method of determining a risk of having or developing a clinical subtype of Crohn's disease **characterized by** fibrostenosis, comprising determining the presence or absence of three markers in a sample from the subject, said three markers being IgA anti-I2 antibodies, anti-*Saccharomyces cerevisiae* antibodies (ASCA), and one of the NOD2 variants selected from R702W, G908R, and 1007fs, wherein the presence of said three markers indicates a first risk of having or developing said clinical subtype of Crohn's disease, the presence of exactly two of said three markers indicates a second risk of having or developing said clinical subtype of Crohn's disease, the presence of exactly one of said three markers indicates a third risk of having or developing said clinical subtype of Crohn's disease, and the absence of said three markers indicates a fourth risk of having or developing said clinical subtype of Crohn's disease, and wherein said first risk is greater than said second risk, said second risk is greater than said third risk, and said third risk is greater than said fourth risk.

## Patentansprüche

1. Ein *in vitro* Verfahren zur Diagnose eines klinischen Subtyps von Morbus Crohn bei einem Subjekt, welches unter Morbus Crohn leidet, wobei das Verfahren Schritte umfaßt, bei denen man die Anwesenheit oder Abwesenheit von IgA anti-I2 Antikörpern in einer Probe von dem Subjekt bestimmt, wobei die Anwesenheit der IgA anti-I2 Antikörper anzeigt, daß das Subjekt den Subtyp von Morbus Crohn aufweist, wobei der klinische Subtyp von Morbus Crohn ein fibrostenotischer Subtyp von Morbus Crohn, ein Subtyp von Morbus Crohn, welcher durch die Notwendigkeit einer Dünndarm-Operation charakterisiert ist, oder ein Subtyp von Morbus Crohn ist, welcher durch die Abwesenheit von Merkmalen der Colitis Ulcerosa charakterisiert ist.

2. Das Verfahren nach Anspruch 1, welches ferner Schritte umfasst, bei denen man die Anwesenheit oder Abwesenheit von einem oder mehreren fibrostenostischen Markern in der Probe bestimmt, ausgewählt aus einer NOD2 Variante oder anti-Saccharomyces cerevisiae Antikörpern (ASCA), wobei die NOD2 Variante aus R702W, G908R und 1007fs ausgewählt ist, und wobei die Anwesenheit der IgA anti-I2 Antikörper und die Anwesenheit von einem von dem einen oder mehreren fibrostenotischen Markern eine größere Wahrscheinlichkeit anzeigt, daß das Subjekt eine aggressive Form des fibrostenotischen Subtyps von Morbus Crohn haben wird, die eine Operation des Dünndarms erforderlich macht.

3. Das Verfahren nach Anspruch 2, **dadurch gekennzeichnet, daß** der eine oder mehrere fibrostenotische Marker eine NOD2 Variante ist.

4. Das Verfahren nach Anspruch 2, **dadurch gekennzeichnet, daß** der eine oder mehrere fibrostenotische Marker ASCA ist.

5. Das Verfahren nach Anspruch 2, **dadurch gekennzeichnet, daß** der eine oder mehrere fibrostenotische Marker eine Variante von NOD2 und ASCA sind.

6. Das Verfahren nach Anspruch 1, **dadurch gekennzeichnet, daß** die Bestimmung der Anwesenheit oder Abwesenheit von IgA anti-I2 Antikörpern bei dem Subjekt die Schritte umfaßt, bei denen man:
(a) eine Probe des Subjektes mit einem I2 Antigen oder einem immunreaktiven Fragment davon unter Bedingungen in Kontakt bringt, die zur Bildung eines Komplexes aus I2 Antigen oder einem immunreaktiven Fragment davon und Antikörper gegen das I2 Antigen geeignet sind,
(b) den Komplexes mit einem markierten sekundären Antikörper in Kontakt bringt, und
(c) die Anwesenheit oder Abwesenheit des Komplexes nachweist, wobei die Anwesenheit des Komplexes die Anwesenheit der IgA anti-I2 Antikörper bei dem Subjekt anzeigt.

7. Das Verfahren nach Anspruch 1, welches zudem eine Bestimmung der Anwesenheit oder Abwesenheit einer NOD2 Variante in der Probe umfaßt, wobei die Anwesenheit von IgA anti-I2 Antikörpern und die Anwesenheit einer NOD2 Variante bei dem Subjekt anzeigt, daß das Subjekt den fibrostenotischen Subtyp von Morbus Crohn aufweist, wobei die NOD2 Variante aus R702W, G908R und 1007fs ausgewählt ist.

8. Das Verfahren nach Anspruch 7, wobei die gemeinsame Anwesenheit der IgA anti-I2 Antikörper und der NOD2 Variante bei dem Subjekt mit dem fibrostenotischen Subtyp von Morbus Crohn mit einer Wahrscheinlichkeit von mindestens 6 assoziiert ist.

9. Das Verfahren nach Anspruch 1, welches ferner die Bestimmung der Anwesenheit oder Abwesenheit von ASCA in der Probe umfaßt, wobei die Anwesenheit der IgA anti-I2 Antikörper und die Anwesenheit des ASCA bei dem Subjekt anzeigt, daß das Subjekt den fibrostenotischen Subtyp von Morbus Crohn hat.

10. Das Verfahren nach Anspruch 9, **dadurch gekennzeichnet, daß** die gemeinsame Anwesenheit der IgA anti-I2 Antikörper und des ASCA bei dem Subjekt mit dem fibrostenotischen Subtyp von Morbus Crohn mit einer Wahrscheinlichkeit von mindestens 6 assoziiert ist.

11. Das Verfahren nach Anspruch 7, ferner umfassend die Bestimmung der Anwesenheit oder Abwesenheit des ASCA in der Probe, wobei die gemeinsame Anwesenheit von IgA anti-I2 Antikörpern, der NOD2 Variante und des ASCA bei dem Subjekt anzeigen, daß das Subjekt den fibrostenotischen Subtyp von Morbus Crohn hat.

12. Das Verfahren nach Anspruch 11, **dadurch gekennzeichnet, daß** die gemeinsame Anwesenheit der IgA anti-I2 Antikörper, der NOD2 Variante und des ASCA bei dem Subjekt mit dem fibrostenotischen Subtyp von Morbus Crohn mit einer Wahrscheinlichkeit von mindestens 9 assoziiert ist.

13. Ein *in vitro* Verfahren zur Bestimmung des Risikos eines Subjekts, das an Morbus Crohn leidet, einen klinischen Subtyp von Morbus Crohn, welcher durch Fibrostenose, interne perforierende Erkrankung (internal perforating disease), oder die Notwendigkeit einer Dünndarm-Operation charakterisiert ist, zu haben oder zu entwickeln, wobei das Verfahren Schritte umfasst, bei denen man die Anwesenheit oder Abwesenheit von drei Markern in einer Probe von dem Subjekt bestimmt, wobei die drei Marker IgA anti-I2 Antikörper, anti-Saccharomyces cerevisiae Antikörper (ASCA) und IgA anti-OmpC Antikörper sind, wobei die Anwesenheit der drei Marker ein erstes Risiko anzeigt, den klinischen Subtyp von Morbus Crohn zu haben oder zu entwickeln,
wobei die Anwesenheit von genau zwei der drei Marker ein zweites Risiko anzeigt, den klinischen Subtyp von Morbus Crohn zu haben oder zu entwickeln,
die Anwesenheit von genau einem der drei Marker ein drittes Risiko anzeigt, den klinischen Subtyp von Morbus Crohn zu haben oder zu entwickeln, und
die Abwesenheit der drei Marker ein viertes Risiko anzeigt, den klinischen Subtyp von Morbus Crohn zu haben oder zu entwickeln, und
wobei das erste Risiko größer ist als das zweite Risiko, das zweite Risiko größer ist als das dritte Risiko, und das dritte Risiko größer als das vierte Risiko ist.

14. Ein *in vitro* Verfahren zur Bestimmung des Risikos eines Sujekts, welches an Morbus Crohn leidet, einen klinischen Subtyp von Morbus Crohn zu haben oder zu entwickeln, wobei der klinische Subtyp **gekennzeichnet ist durch** Fibrostenose oder die Notwendigkeit einer Dünndarm-Operation, wobei das Verfahren die Bestimmung der Anwesenheit und des Ausmaßes der IgA anti-I2 Antikörper-Antwort in einer Probe von dem Subjekt umfaßt, wobei ein größeres Ausmaß der IgA anti-I2 Antikörper-Antwort ein größeres Risiko anzeigt, den **durch** Fibrostenose oder die Notwendigkeit einer Dünndarm-Operation charakterisierten klinischen Subtyp zu haben oder zu entwickeln.

15. Ein *in vitro* Verfahren zur Bestimmung des Risikos eines Subjekts, welches an Morbus Crohn leidet, einen klinischen Subtyp von Morbus Crohn zu haben oder zu entwickeln, welcher charakterisiert ist durch Fibrostenose, interne perforierende Erkrankung (internal perforating disease) oder die Notwendigkeit einer Dünndarm-Operation, wobei das Verfahren die Bestimmung der Anwesenheit und des Ausmaßes von drei Markern in einer Probe von dem Subjekt umfaßt, wobei die drei Marker IgA anti-I2 Antikörper, Anti-Saccharomyces cerevisiae Antikörper (ASCA) und IgA anti-OmpC Antikörper sind, wobei ein größeres Ausmaß der drei Marker in Kombination ein größeres Risiko anzeigt, den durch Fibrostenose, interne perforierende Erkrankung oder die Notwendigkeit einer Dünndarm-Operation charakterisierten klinischen Subtyp zu haben oder zu entwickeln.

16. Ein *in vitro* Verfahren zur Bestimmung eines Risikos, einen durch Fibrostenose **gekennzeichneten** klinischen Subtyp von Morbus Crohn zu haben oder zu entwickeln, umfassend die Bestimmung der Anwesenheit oder Abwesenheit von drei Markern in einer Probe von dem Subjekt, wobei die drei Marker IgA anti-I2 Antikörper, anti-*Saccharomyces cerevisiae* Antikörper (ASCA) und eine der NOD2 Varianten, ausgewählt aus R702W, G908R und 1007fs, sind, wobei die Anwesenheit der drei Marker ein erstes Risiko anzeigt, den klinischen Subtyp von Morbus Crohn zu haben oder zu entwickeln, die Anwesenheit von genau zwei der drei Marker ein zweites Risiko anzeigt, den klinischen Subtyp von Morbus Crohn zu haben oder zu entwickeln, die Anwesenheit von genau einem der drei Marker ein drittes Risiko anzeigt, den klinischen Subtyp von Morbus Crohn zu haben oder zu entwickeln, und die Abwesenheit der drei Marker ein viertes Risiko anzeigt, den klinischen Subtyp von Morbus Crohn zu haben oder zu entwickeln, und wobei das erste Risiko größer ist als das zweite Risiko, das zweite Risiko größer als das dritte Risiko ist und das dritte Risiko größer als das vierte Risiko ist.

## Revendications

1. Procédé *in vitro* de diagnostic d'un sous-type clinique de la maladie de Crohn chez un sujet atteint de la maladie de Crohn, lequel procédé comporte le fait de déterminer la présence ou l'absence d'anticorps IgA anti-I2 dans un échantillon provenant du sujet, étant entendu que la présence de ces anticorps IgA anti-I2 indique que le sujet présente ledit sous-type de la maladie de Crohn, lequel sous-type clinique de la maladie de Crohn est un sous-type fibrosténotique de la maladie de Crohn, un sous-type de la maladie de Crohn **caractérisé par** la nécessité d'une petite intervention chirurgicale au niveau de l'intestin, ou un sous-type de la maladie de Crohn **caractérisé par** l'absence de signes de colite ulcéreuse.

2. Procédé conforme à la revendication 1, qui comporte en outre le fait de déterminer la présence ou l'absence, dans l'échantillon, d'un ou de plusieurs marqueurs de fibrosténose choisis parmi un variant du gène Nod2 et des anticorps anti-*Saccharomyces cerevisiae* (AASC), lequel variant de Nod2 est choisi parmi R702W, G908R et 1007fs, étant entendu que la présence desdits anticorps IgA anti-I2 et la présence de l'un desdits marqueurs de fibrosténose indiquent que le sujet a un plus grand risque de présenter une forme agressive du sous-type fibrosténotique de la maladie de Crohn, nécessitant une petite intervention chirurgicale au niveau de l'intestin.

3. Procédé conforme à la revendication 2, dans lequel ledit ou lesdits marqueur(s) de fibrosténose est ou sont un variant du gène Nod2.

4. Procédé conforme à la revendication 2, dans lequel ledit ou lesdits marqueur(s) de fibrosténose est ou sont des anticorps AASC.

5. Procédé conforme à la revendication 2, dans lequel ledit ou lesdits marqueur(s) de fibrosténose est ou sont un variant du gène Nod2 et des anticorps AASC.

6. Procédé conforme à la revendication 1, dans lequel le fait de déterminer la présence ou l'absence d'anticorps IgA anti-I2 chez le sujet comporte les étapes suivantes :
a) mettre un échantillon provenant du sujet en contact avec un antigène 12 ou un fragment immuno-réactif d'un tel antigène, dans des conditions appropriées pour qu'il se forme un complexe de cet antigène 12 ou du fragment immuno-réactif d'un tel antigène et d'un anticorps dirigé contre cet antigène 12 ;
b) mettre ce complexe en contact avec un anticorps secondaire marqué ;
c) et déterminer si ledit complexe est présent ou absent, étant entendu que la présence de ce complexe indique la présence desdits anticorps IgA anti-I2 chez le sujet.

7. Procédé conforme à la revendication 1, qui comporte en outre le fait de déterminer la présence ou l'absence d'un variant du gène Nod2 dans l'échantillon, étant entendu que la présence desdits anticorps IgA anti-12 et la présence d'un variant du gène Nod2 chez le sujet indiquent que le sujet présente ledit sous-type fibrosténotique de la maladie de Crohn, ledit variant de Nod2 étant choisi parmi R702W, G908R et 1007fs.

8. Procédé conforme à la revendication 7, dans lequel la présence conjointe desdits anticorps IgA anti-I2 et dudit variant du gène Nod2 chez le sujet est associée audit sous-type fibrosténotique de la maladie de Crohn avec un rapport de cotes d'au moins 6.

9. Procédé conforme à la revendication 1, qui comporte en outre le fait de déterminer la présence ou l'absence d'anticorps AASC dans l'échantillon, étant entendu que la présence desdits anticorps IgA anti-I2 et la présence d'anticorps AASC chez le sujet indiquent que le sujet présente ledit sous-type fibrosténotique de la maladie de Crohn.

10. Procédé conforme à la revendication 9, dans lequel la présence conjointe desdits anticorps IgA anti-I2 et desdits anticorps AASC chez le sujet est associée audit sous-type fibrosténotique de la maladie de Crohn avec un rapport de cotes d'au moins 6.

11. Procédé conforme à la revendication 7, qui comporte en outre le fait de déterminer la présence ou l'absence d'anticorps AASC dans l'échantillon, étant entendu que la présence conjointe desdits anticorps IgA anti-I2, dudit variant du gène Nod2 et desdits anticorps AASC chez le sujet indique que le sujet présente ledit sous-type fibrosténotique de la maladie de Crohn.

12. Procédé conforme à la revendication 11, dans lequel la présence conjointe desdits anticorps IgA anti-I2, dudit variant du gène Nod2 et desdits anticorps AASC chez le sujet est associée audit sous-type fibrosténotique de la maladie de Crohn avec un rapport de cotes d'au moins 9.

13. Procédé *in vitro* de détermination du risque qu'a un sujet atteint de la maladie de Crohn de présenter ou développer un sous-type clinique de la maladie de Crohn **caractérisé par** une fibrosténose, un désordre intestinal perforant ou la nécessité d'une petite intervention chirurgicale au niveau de l'intestin, lequel procédé comporte le fait de déterminer la présence ou l'absence de trois marqueurs dans un échantillon provenant du sujet, ces trois marqueurs étant des anticorps IgA anti-I2, des anticorps anti-*Saccharomyces cerevisiae* (AASC) et des anticorps IgA anti-OmpC, étant entendu que :
la présence de ces trois marqueurs est l'indice d'un premier risque de pré-senter ou développer ledit sous-type clinique de la maladie de Crohn,
la présence de juste deux de ces trois marqueurs est l'indice d'un deuxième risque de présenter ou développer ledit sous-type clinique de la maladie de Crohn,
la présence de juste un de ces trois marqueurs est l'indice d'un troisième risque de présenter ou développer ledit sous-type clinique de la maladie de Crohn,
et l'absence de ces trois marqueurs est l'indice d'un quatrième risque de présenter ou développer ledit sous-type clinique de la maladie de Crohn,
et que ledit premier risque est plus grand que ledit deuxième risque, ledit deuxième risque est plus grand que ledit troisième risque, et ledit troisième risque est plus grand que ledit quatrième risque.

14. Procédé *in vitro* de détermination du risque qu'a un sujet atteint de la maladie de Crohn de présenter ou développer un sous-type clinique de la maladie de Crohn, sous-type clinique **caractérisé par** une fibrosténose ou la nécessité d'une petite intervention chirurgicale au niveau de l'intestin, lequel procédé comporte le fait de déterminer la présence d'anticorps IgA anti-I2 et le niveau de la réponse en ces anticorps dans un échantillon provenant du sujet, étant entendu qu'un niveau plus élevé de la réponse en anticorps IgA anti-I2 est l'indice d'un plus grand risque de présenter ou développer ledit sous-type clinique **caractérisé par** une fibrosténose ou la nécessité d'une petite intervention chirurgicale au niveau de l'intestin.

15. Procédé *in vitro* de détermination du risque qu'a un sujet atteint de la maladie de Crohn de présenter ou développer un sous-type clinique de la maladie de Crohn **caractérisé par** une fibrosténose, un désordre intestinal perforant ou la nécessité d'une petite intervention chirurgicale au niveau de l'intestin, lequel procédé comporte le fait de déterminer la présence et le niveau de trois marqueurs dans un échantillon provenant du sujet, ces trois marqueurs étant des anticorps IgA anti-12, des anticorps anti-*Saccharomyces cerevisiae* (AASC) et des anticorps IgA anti-OmpC, étant entendu qu'un niveau plus élevé de ces trois marqueurs combinés est l'indice d'un plus grand risque de présenter ou développer ledit sous-type clinique **caractérisé par** une fibrosténose, un désordre intestinal perforant ou la nécessité d'une petite intervention chirurgicale au niveau de l'intestin.

16. Procédé *in vitro* de détermination du risque de présenter ou développer un sous-type clinique de la maladie de Crohn **caractérisé par** une fibrosténose, lequel procédé comporte le fait de déterminer la présence ou l'absence de trois marqueurs dans un échantillon provenant du sujet, ces trois marqueurs étant des anticorps IgA anti-I2, des anticorps anti-*Saccharomyces cerevisiae* (AASC) et l'un des variants du gène Nod2, choisi parmi R702W, G908R et 1007fs, étant entendu que :
la présence de ces trois marqueurs est l'indice d'un premier risque de présenter ou développer ledit sous-type clinique de la maladie de Crohn,
la présence de juste deux de ces trois marqueurs est l'indice d'un deuxième risque de présenter ou développer ledit sous-type clinique de la maladie de Crohn,
la présence de juste un de ces trois marqueurs est l'indice d'un troisième risque de présenter ou développer ledit sous-type clinique de la maladie de Crohn,
et l'absence de ces trois marqueurs est l'indice d'un quatrième risque de présenter ou développer ledit sous-type clinique de la maladie de Crohn,
et que ledit premier risque est plus grand que ledit deuxième risque, ledit deuxième risque est plus grand que ledit troisième risque, et ledit troisième risque est plus grand que ledit quatrième risque.


### SEQUENCE LISTING

<110> Cedars-Sinai Medical Center
   Targan, Stephan R.
   Vasiliauskas, Eric A.
   Mow, William S.
   Yang, Huiying
   Fleshner, Phillip R.
   Rotter, Jerome I.
<120> Methods of Assessing Crohn's Disease
   Patient Phenotype by I2, OmpC and ASCA Serologic Response
<130> 66783-147
<150> US 10/723,164
   <151> 2003-11-26
<150> US 10/413,501
   <151> 2003-04-11
<160> 22
<170> FastSEQ for Windows Version 4.0
<210> 1
   <211> 302
   <212> DNA
   <213> P. aeruginosa
<220>
   <221> CDS
   <222> (2)...(301)
<400> 1
   a gat ctg gcc agc gcc gtg ggc atc cag tcc ggc agc atc ttt cat cac 49
     Asp Leu Ala Ser Ala Val Gly Ile Gln Ser Gly Ser Ile Phe His His
      1               5                  10                  15
   ttc aag agc aag gat gag ata ttg cgt gcc gtg atg gag gaa acc atc   97
   Phe Lys Ser Lys Asp Glu Ile Leu Arg Ala Val Met Glu Glu Thr Ile
               20                  25                  30
   cat tac aac acc gcg atg atg cgc gct tca ctg gag gag gcg agc acg   145
   His Tyr Asn Thr Ala Met Met Arg Ala Ser Leu Glu Glu Ala Ser Thr
           35                  40                  45
   gtg cgc gaa cgc gtg ctg gcg ctg atc cgc tgc gag ttg cag tcg atc   193
   Val Arg Glu Arg Val Leu Ala Leu Ile Arg Cys Glu Leu Gln Ser Ile
       50                  55                  60
   atg ggc ggc agt ggc gag gcc atg gcg gtg ctg gtc tac gaa tgg cgc   241
   Met Gly Gly Ser Gly Glu Ala Met Ala Val Leu Val Tyr Glu Trp Arg
   65                  70                  75                  80
   tcg ctg tcg gcc gaa ggc cag gcg cac gtg ctg gcc ctg cgt gac gtg   289
   Ser Leu Ser Ala Glu Gly Gln Ala His Val Leu Ala Leu Arg Asp Val
                   85                  90                  95
   tat gag cag atc t                                                 302
   Tyr Glu Gln Ile
               100
<210> 2
   <211> 100
   <212> PRT
   <213> P. aeruginosa
<400> 2
   Asp Leu Ala Ser Ala Val Gly Ile Gln Ser Gly Ser Ile Phe His His
   1               5                   10                  15
   Phe Lys Ser Lys Asp Glu Ile Leu Arg Ala Val Met Glu Glu Thr Ile
               20                  25                  30
   His Tyr Asn Thr Ala Met Met Arg Ala Ser Leu Glu Glu Ala Ser Thr
           35                  40                  45
   Val Arg Glu Arg Val Leu Ala Leu Ile Arg Cys Glu Leu Gln Ser Ile
       50                  55                  60
   Met Gly Gly Ser Gly Glu Ala Met Ala Val Leu Val Tyr Glu Trp Arg
   65                  70                  75                  80
   Ser Leu Ser Ala Glu Gly Gln Ala His Val Leu Ala Leu Arg Asp Val
                   85                  90                  95
   Tyr Glu Gln Ile
               100
<210> 3
   <211> 494
   <212> DNA
   <213> Homo sapiens
<400> 3
   accttcagat cacagcagcc ttcctggcag ggctgttgtc ccgggagcac tggggcctgc 60
   tggctgagtg ccagacatct gagaaggccc tgctccggcg ccaggcctgt gcccgctggt 120
   gtctggcccg cagcctccgc aagcacttcc actccatccc gccagctgca ccgggtgagg 180
   ccaagagcgt gcatgccatg cccgggttca tctggctcat ccggagcctg tacgagatgc 240
   aggaggagcg gctggctcgg aaggctgcac gtggcctgaa tgttgggcac ctcaagttga 300
   cattttgcag tgtgggcccc actgagtgtg ctgccctggc ctttgtgctg cagcacctcc 360
   ggcggcccgt ggccctgcag ctggactaca actctgtggg tgacattggc ctggagcagc 420
   tgctgccttg ccttggtgtc tgcaaggctc tgtagtgagt gttactgggc attgctgttc 480
   aggtatgggg gagc                                                   494
<210> 4
   <211> 494
   <212> DNA
   <213> Homo sapiens
<400> 4
   gctcccccat acctgaacag caatgcccag taacactcac tacagagcct tgcagacacc 60
   aaggcaaggc agcagctgct ccaggccaat gtcacccaca gagttgtagt ccagctgcag 120
   ggccacgggc cgccggaggt gctgcagcac aaaggccagg gcagcacact cagtggggcc 180
   cacactgcaa aatgtcaact tgaggtgccc aacattcagg ccacgtgcag ccttccgagc 240
   cagccgctcc tcctgcatct cgtacaggct ccggatgagc cagatgaacc cgggcatggc 300
   atgcacgctc ttggcctcac ccggtgcagc tggcgggatg gagtggaagt gcttgcggag 360
   gctgcgggcc agacaccagc gggcacaggc ctggcgccgg agcagggcct tctcagatgt 420
   ctggcactca gccagcaggc cccagtgctc ccgggacaac agccctgcca ggaaggctgc 480
   tgtgatctga aggt                                                   494
<210> 5
   <211> 540
   <212> DNA
   <213> Homo sapiens
<400> 5
   atcaaaaccc tgagaggaca agggacattt ccaagtcacc cagaaagact cgagtgtcct 60
   ctcttgaaat ccaatggtct tttttcctta ctccattgcc taacattgtg gggtagaaat 120
   aaagttcaaa gaccttcaga actggcccca gctcctccct cttcacctga tctccccaag 180
   aaaactgcag gatagactct gaagcttacc tgagccacct caagctctgg tgatcaccca 240
   aggcttcagc cagggcctgg gccccctcgt cacccactct gttgccccag aatctgaaaa 300
   ggccaaaaga gtcaacagac agtgtcagtg agtacctgat atgtgttcta gacatgaact 360
   aacagtcctc ctccctctgc agtcccagcc agaggggcag gaccactcaa tcccagagtg 420
   gcctcactgg ggctcctggt cccagcaaag tggacctgcc tccatctttt gggtgggatg 480
   gccaaactta acccaagagt tttcagtggc tttacattac agacttagag aatagtagag 540
<210> 6
   <211> 540
   <212> DNA
   <213> Homo sapiens
<400> 6
   ctctactatt ctctaagtct gtaatgtaaa gccactgaaa actcttgggt taagtttggc 60
   catcccaccc aaaagatgga ggcaggtcca ctttgctggg accaggagcc ccagtgaggc 120
   cactctggga ttgagtggtc ctgcccctct ggctgggact gcagagggag gaggactgtt 180
   agttcatgtc tagaacacat atcaggtact cactgacact gtctgttgac tcttttggcc 240
   ttttcagatt ctggggcaac agagtgggtg acgagggggc ccaggccctg gctgaagcct 300
   tgggtgatca ccagagcttg aggtggctca ggtaagcttc agagtctatc ctgcagtttt 360
   cttggggaga tcaggtgaag agggaggagc tggggccagt tctgaaggtc tttgaacttt 420
   atttctaccc cacaatgtta ggcaatggag taaggaaaaa agaccattgg atttcaagag 480
   aggacactcg agtctttctg ggtgacttgg aaatgtccct tgtcctctca gggttttgat 540
<210> 7
   <211> 541
   <212> DNA
   <213> Homo sapiens
<400> 7
   tttaaaaatg aaatcattgc tccctactta aagaggtaaa gacttctttc ttagacagag 60
   aatcagatcc ttcacatgca gaatcattct cactgaatgt cagaatcaga agggatcctc 120
   aaaattctgc cattcctctc tcccgtcacc ccattttaca gatagaaaaa ctgaggttcg 180
   gagagctaaa acaggcctgc ccaggggcct taccagactt ccaggatggt gtcattcctt 240
   tcaaggggcc tgcaggaggg cttctgcccc taggtaggtg atgcagttat tggacaacct 300
   ggaaaagaag atacaatggt gagcttcaag gattcttggt tttcctcttg aaactgtcca 360
   gttaaagaga ctgcaggagt tagccagtct actgaagccc acctgtccct tagacacatc 420
   ctgctcatgt ctgagattcc caatgagctc atcaacaaag gctcagtacc atcagtgaaa 480
   tgtaaccgtc tctcttccat tcactagatg agtttatcaa attaagtagc cactccctta 540
   g                                                                 541
<210> 8
   <211> 541
   <212> DNA
   <213> Homo sapiens
<400> 8
   ctaagggagt ggctacttaa tttgataaac tcatctagtg aatggaagag agacggttac 60
   atttcactga tggtactgag cctttgttga tgagctcatt gggaatctca gacatgagca 120
   ggatgtgtct aagggacagg tgggcttcag tagactggct aactcctgca gtctctttaa 180
   ctggacagtt tcaagaggaa aaccaagaat ccttgaagct caccattgta tcttcttttc 240
   caggttgtcc aataactgca tcacctacct aggggcagaa gccctcctgc aggccccttg 300
   aaaggaatga caccatcctg gaagtctggt aaggcccctg ggcaggcctg ttttagctct 360
   ccgaacctca gtttttctat ctgtaaaatg gggtgacggg agagaggaat ggcagaattt 420
   tgaggatccc ttctgattct gacattcagt gagaatgatt ctgcatgtga aggatctgat 480
   tctctgtcta agaaagaagt ctttacctct ttaagtaggg agcaatgatt tcatttttaa 540
   a                                                                 541
<210> 9
   <211> 1101
   <212> DNA
   <213> E. coli
<220>
   <221> CDS
   <222> (1)...(1101)
<400> 9
   atg aaa gtt aaa gta ctg tcc ctc ctg gtc cca gct ctg ctg gta gca    48
   Met Lys Val Lys Val Leu Ser Leu Leu Val Pro Ala Leu Leu Val Ala
    1               5                  10                  15
   ggc gca gca aac gct gct gaa gtt tac aac aaa gac ggc aac aaa tta    96
   Gly Ala Ala Asn Ala Ala Glu Val Tyr Asn Lys Asp Gly Asn Lys Leu
               20                  25                  30
   gat ctg tac ggt aaa gta gac ggc ctg cac tat ttc tct gac aac aaa    144
   Asp Leu Tyr Gly Lys Val Asp Gly Leu His Tyr Phe Ser Asp Asn Lys
           35                  40                  45
   gat gta gat ggc gac cag acc tac atg cgt ctt ggc ttc aaa ggt gaa    192
   Asp Val Asp Gly Asp Gln Thr Tyr Met Arg Leu Gly Phe Lys Gly Glu
       50                  55                  60
   act cag gtt act gac cag ctg acc ggt tac ggc cag tgg gaa tat cag    240
   Thr Gln Val Thr Asp Gln Leu Thr Gly Tyr Gly Gln Trp Glu Tyr Gln
    65                  70                  75                  80
   atc cag ggc aac agc gct gaa aac gaa aac aac tcc tgg acc cgt gtg    288
   Ile Gln Gly Asn Ser Ala Glu Asn Glu Asn Asn Ser Trp Thr Arg Val
                   85                  90                  95
   gca ttc gca ggt ctg aaa ttc cag gat gtg ggt tct ttc gac tac ggt    336
   Ala Phe Ala Gly Leu Lys Phe Gln Asp Val Gly Ser Phe Asp Tyr Gly
               100                 105                 110
   cgt aac tac ggc gtt gtt tat gac gta act tcc tgg acc gac gta ctg    384
   Arg Asn Tyr Gly Val Val Tyr Asp Val Thr Ser Trp Thr Asp Val Leu
           115                 120                 125
   cca gaa ttc ggt ggt gac acc tac ggt tct gac aac ttc atg cag cag    432
   Pro Glu Phe Gly Gly Asp Thr Tyr Gly Ser Asp Asn Phe Met Gln Gln
       130                 135                 140
   cgt ggt aac ggc ttc gcg acc tac cgt aac act gac ttc ttc ggt ctg    480
   Arg Gly Asn Gly Phe Ala Thr Tyr Arg Asn Thr Asp Phe Phe Gly Leu
   145                 150                 155                 160
   gtt gac ggc ctg aac ttt gct gtt cag tac cag ggt aaa aac ggc aac    528
   Val Asp Gly Leu Asn Phe Ala Val Gln Tyr Gln Gly Lys Asn Gly Asn
                   165                 170                 175
   cca tct ggt gaa ggc ttt act agt ggc gta act aac aac ggt cgt gac    576
   Pro Ser Gly Glu Gly Phe Thr Ser Gly Val Thr Asn Asn Gly Arg Asp
               180                 185                 190
   gca ctg cgt caa aac ggc gac ggc gtc ggc ggt tct atc act tat gat    624
   Ala Leu Arg Gln Asn Gly Asp Gly Val Gly Gly Ser Ile Thr Tyr Asp
           195                 200                 205
   tac gaa ggt ttc ggt atc ggt ggt gcg atc tcc agc tcc aaa cgt act    672
   Tyr Glu Gly Phe Gly Ile Gly Gly Ala Ile Ser Ser Ser Lys Arg Thr
       210                 215                 220
   gat gct cag aac acc gct gct tac atc ggt aac ggc gac cgt gct gaa    720
   Asp Ala Gln Asn Thr Ala Ala Tyr Ile Gly Asn Gly Asp Arg Ala Glu
   225                 230                 235                 240
   acc tac act ggt ggt ctg aaa tac gac gct aac aac atc tac ctg gct    768
   Thr Tyr Thr Gly Gly Leu Lys Tyr Asp Ala Asn Asn Ile Tyr Leu Ala
                   245                 250                 255
   gct cag tac acc cag acc tac aac gca act cgc gta ggt tcc ctg ggt    816
   Ala Gln Tyr Thr Gln Thr Tyr Asn Ala Thr Arg Val Gly Ser Leu Gly
               260                 265                 270
   tgg gcg aac aaa gca cag aac ttc gaa gct gtt gct cag tac cag ttc    864
   Trp Ala Asn Lys Ala Gln Asn Phe Glu Ala Val Ala Gln Tyr Gln Phe
           275                 280                 285
   gac ttc ggt ctg cgt ccg tcc ctg gct tac ctg cag tct aaa ggt aaa    912
   Asp Phe Gly Leu Arg Pro Ser Leu Ala Tyr Leu Gln Ser Lys Gly Lys
       290                 295                 300
   aac ctg ggt cgt ggc tac gac gac gaa gat atc ctg aaa tat gtt gat    960
   Asn Leu Gly Arg Gly Tyr Asp Asp Glu Asp Ile Leu Lys Tyr Val Asp
   305                 310                 315                 320
   gtt ggt gct acc tac tac ttc aac aaa aac atg tcc acc tac gtt gac    1008
   Val Gly Ala Thr Tyr Tyr Phe Asn Lys Asn Met Ser Thr Tyr Val Asp
                   325                 330                 335
   tac aaa atc aac ctg ctg gac gac aac cag ttc act cgt gac gct ggc    1056
   Tyr Lys Ile Asn Leu Leu Asp Asp Asn Gln Phe Thr Arg Asp Ala Gly
               340                 345                 350
   atc aac act gat aac atc gta gct ctg ggt ctg gtt tac cag ttc        1101
   Ile Asn Thr Asp Asn Ile Val Ala Leu Gly Leu Val Tyr Gln Phe
           355                 360                 365
<210> 10
   <211> 367
   <212> PRT
   <213> E. coli
<400> 10
   Met Lys Val Lys Val Leu Ser Leu Leu Val Pro Ala Leu Leu Val Ala
    1               5                  10                  15
   Gly Ala Ala Asn Ala Ala Glu Val Tyr Asn Lys Asp Gly Asn Lys Leu
               20                  25                  30
   Asp Leu Tyr Gly Lys Val Asp Gly Leu His Tyr Phe Ser Asp Asn Lys
           35                  40                  45
   Asp Val Asp Gly Asp Gln Thr Tyr Met Arg Leu Gly Phe Lys Gly Glu
       50                  55                  60
   Thr Gln Val Thr Asp Gln Leu Thr Gly Tyr Gly Gln Trp Glu Tyr Gln
   65                  70                  75                  80
   Ile Gln Gly Asn Ser Ala Glu Asn Glu Asn Asn Ser Trp Thr Arg Val
                   85                  90                  95
   Ala Phe Ala Gly Leu Lys Phe Gln Asp Val Gly Ser Phe Asp Tyr Gly
               100                 105                 110
   Arg Asn Tyr Gly Val Val Tyr Asp Val Thr Ser Trp Thr Asp Val Leu
           115                 120                 125
   Pro Glu Phe Gly Gly Asp Thr Tyr Gly Ser Asp Asn Phe Met Gln Gln
       130                 135                 140
   Arg Gly Asn Gly Phe Ala Thr Tyr Arg Asn Thr Asp Phe Phe Gly Leu
   145                 150                 155                 160
   Val Asp Gly Leu Asn Phe Ala Val Gln Tyr Gln Gly Lys Asn Gly Asn
                   165                 170                 175
   Pro Ser Gly Glu Gly Phe Thr Ser Gly Val Thr Asn Asn Gly Arg Asp
               180                 185                 190
   Ala Leu Arg Gln Asn Gly Asp Gly Val Gly Gly Ser Ile Thr Tyr Asp
           195                 200                 205
   Tyr Glu Gly Phe Gly Ile Gly Gly Ala Ile Ser Ser Ser Lys Arg Thr
       210                 215                 220
   Asp Ala Gln Asn Thr Ala Ala Tyr Ile Gly Asn Gly Asp Arg Ala Glu
   225                 230                 235                 240
   Thr Tyr Thr Gly Gly Leu Lys Tyr Asp Ala Asn Asn Ile Tyr Leu Ala
                   245                 250                 255
   Ala Gln Tyr Thr Gln Thr Tyr Asn Ala Thr Arg Val Gly Ser Leu Gly
               260                 265                 270
   Trp Ala Asn Lys Ala Gln Asn Phe Glu Ala Val Ala Gln Tyr Gln Phe
           275                 280                 285
   Asp Phe Gly Leu Arg Pro Ser Leu Ala Tyr Leu Gln Ser Lys Gly Lys
       290                 295                 300
   Asn Leu Gly Arg Gly Tyr Asp Asp Glu Asp Ile Leu Lys Tyr Val Asp
   305                 310                 315                 320
   Val Gly Ala Thr Tyr Tyr Phe Asn Lys Asn Met Ser Thr Tyr Val Asp
                   325                 330                 335
   Tyr Lys Ile Asn Leu Leu Asp Asp Asn Gln Phe Thr Arg Asp Ala Gly
               340                 345                 350
   Ile Asn Thr Asp Asn Ile Val Ala Leu Gly Leu Val Tyr Gln Phe
           355                 360                 365
<210> 11
   <211> 21
   <212> DNA
   <213> Artificial Sequence
<220>
   <223> primer
<400> 11
   ctggctgagt gccagacatc t 21
<210> 12
   <211> 17
   <212> DNA
   <213> Artificial Sequence
<220>
   <223> primer
<400> 12
   ggcgggatgg agtggaa 17
<210> 13
   <211> 21
   <212> DNA
   <213> Artificial Sequence
<220>
   <223> primer
<400> 13
   ccacctcaag ctctggtgat c 21
<210> 14
   <211> 23
   <212> DNA
   <213> Artificial Sequence
<220>
   <223> primer
<400> 14
   gttgactctt ttggcctttt cag 23
<210> 15
   <211> 23
   <212> DNA
   <213> Artificial Sequence
<220>
   <223> primer
<400> 15
   ccttaccaga cttccaggat ggt 23
<210> 16
   <211> 25
   <212> DNA
   <213> Artificial Sequence
<220>
   <223> primer
<400> 16
   tgtccaataa ctgcatcacc tacct 25
<210> 17
   <211> 13
   <212> DNA
   <213> Artificial Sequence
<220>
   <223> primer
<400> 17
   tgctccggcg cca 13
<210> 18
   <211> 14
   <212> DNA
   <213> Artificial Sequence
<220>
   <223> primer
<400> 18
   ctgctctggc gcca 14
<210> 19
   <211> 16
   <212> DNA
   <213> Artificial Sequence
<220>
   <223> primer
<400> 19
   ctctgttgcc ccagaa 16
<210> 20
   <211> 15
   <212> DNA
   <213> Artificial Sequence
<220>
   <223> primer
<400> 20
   ctctgttgcg ccaga 15
<210> 21
   <211> 15
   <212> DNA
   <213> Artificial Sequence
<220>
   <223> primer
<400> 21
   ctttcaaggg cctgc 15
<210> 22
   <211> 15
   <212> DNA
   <213> Artificial Sequence
<220>
   <223> primer
<400> 22
   cctttcaagg ggcct 15